# FASCICULE DE BREVET EUROPEEN

(11) **EP 0 952 974 B1**
(45) Date de publication et mention de la délivrance du brevet: **21.11.2001**
(21) Numéro de dépôt: 98942767.9
(22) Date de dépôt: 21.08.1998
(51) Int. Cl.: C07C 65/17, C07C 65/26, C07C 69/94, A61K 31/19, A61K 31/235

(54) **DERIVES BIPHENYLIQUES SUBSTITUES PAR UN RADICAL AROMATIQUE OU HETEROAROMATIQUE ET COMPOSITIONS PHARMACEUTIQUES ET COSMETIQUES LES CONTENANT**
VON EINEM AROMATISCHEN ODER HETEROAROMATISCHEN RADIKAL SUBSTITUIERTE BIPHENYLDERIVATE UND DIESE ENTHALTENDE PHARMACEUTISCHE UND KOSMETISCHE COMPOSITIONEN
BIPHENYL DERIVATIVES SUBSTITUTED BY AN AROMATIC OR HETEROAROMATIC RADICAL AND PHARMACEUTICAL AND COSMETIC COMPOSITIONS CONTAINING SAME

(30) Priorité: 21.08.1997 FR 9710552
(43) Date de publication de la demande: 03.11.1999
(73) Titulaire: Galderma Research & Development, 06560 Valbonne (FR)
(72) Inventeur: BERNARDON, Jean-Michel, F-06650 Le Rouret (FR); NEDONCELLE, Philippe, F-06130 Grasse (FR)
(74) Mandataire: Stalla-Bourdillon, Bernard
(86) Numéro de dépôt international: FR9801834
(87) Numéro de publication internationale: WO9910308

(56) Documents cités:
- EP-A- 0 382 077
- GB-A- 2 150 563

## Description

L'invention concerne, à titre de produits industriels nouveaux et utiles, des dérivés biphényliques substitués par un radical aromatique ou hétéroaromatique. Elle concerne également l'utilisation de ces nouveaux composés dans des compositions pharmaceutiques destinées à un usage en médecine humaine ou vétérinaire, ou bien encore dans des compositions cosmétiques.

Les composés selon l'invention ont une activité marquée dans les domaines de la différenciation et de la prolifération cellulaire et trouvent des applications plus particulièrement dans le traitement topique et systémique des affections dermatologiques liées à un désordre de la kératinisation, des affections dermatologiques (ou autres) à composante inflammatoire et/ou immunoallergique, et des proliférations dermiques ou épidermiques qu'elles soient bénignes ou malignes. Ces composés peuvent en outre être utilisés dans le traitement des maladies de dégénérescence du tissu conjonctif, pour lutter contre le vieillissement de la peau, qu'il soit photoinduit ou chronologique, et traiter les troubles de la cicatrisation. Ils trouvent par ailleurs une application dans le domaine ophtalmologique, notamment dans le traitement des cornéopathies.

On peut également utiliser les composés selon l'invention dans des compositions cosmétiques pour l'hygiène corporelle et capillaire.

Il a déjà été décrit dans l'EP-382 077 des dérivés triaromatiques dont la structure est essentiellement constituée par deux noyaux aromatiques substitués et reliés entre eux par un radical divalent hétéroaryle à 5 ou 6 chaînons comportant en tant qu'hétéroatome un atome d'oxygène, un atome de soufre et/ou au moins un atome d'azote.

Les composés selon la présente invention qui sont également des dérivés triaromatiques se distinguent, par rapport à ceux de l'EP-382 077, essentiellement en ce que lorsqu'ils possèdent un radical hétéroaryle, notamment un radical pyridyle, furyle ou thiényle substitué(s), celui-ci est situé en bout de chaîne conférant ainsi à ces composés une structure chimique totalement différente de celle des composés de l'EP-382 077.

Bien que les composés selon l'invention ne soient pas limités à ceux comportant un radical hétéroaryle, on a néanmoins constaté de façon inattendue et surprenante que les composés comportant un tel radical présentaient d'excellentes propriétés pharmaceutiques et cosmétiques tout à fait similaires à celles des composés selon l'invention comportant en bout de chaîne un radical phényle substitué(s).

On a par ailleurs pu mettre en évidence que les composés selon l'invention tout en présentant une excellente activité étaient pratiquement dénués d'effets secondaires.

La présente invention a donc pour objet de nouveaux composés qui peuvent être représentés par la formule générale suivante : 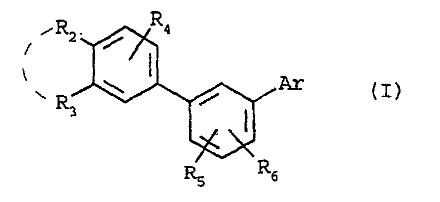 dans laquelle :
Ar représente un radical aromatique ou hétéroaromatique choisi parmi : 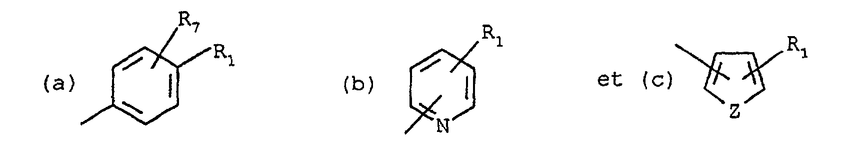
Z étant O ou S,
R₁ représente -CH₃, -CH₂-OH, -OR₈ ou -COR₉,
R₂ et R₃, identiques ou différents, représentent H, alkyle, linéaire ou ramifié, en C₁-C₁₅, cycloalkyle, -ZR₁₀ ou un radical polyéther, l'un au moins de R₂ ou R₃ représentant un alkyle, linéaire ou ramifié, en C₁-C₁₅, ou
R₂ et R₃, pris ensemble forment un cycle à 5 ou 6 chaînons, éventuellement substitué par au moins un méthyle et/ou éventuellement interrompu par un atome d'oxygène ou de soufre ou par un radical SO ou SO₂,
R₄ représente H, un atome d'halogène, alkyle, linéaire ou ramifié, en C₁-C₂₀, -OR₁₀, -OCOR₁₁, ou un radical polyéther,
R₅ représente H, un atome d'halogène, alkyle, linéaire ou ramifié, en C₁-C₂₀, -OCOR₁₁, -OR₁₂, mono- ou polyhydroxyalkyle, -NO₂, 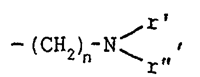 - (CH₂)ₙ-NHCOCH₃, -CH=CH-COR₁₃, - (CH₂)ₙCOR₁₃,
n étant 0 à 6, -O-(CH₂)ₘCOR₁₃, -O-(CH₂)ₘOH, m étant 1 à 12, aryle éventuellement substitué, aralkyle éventuellement substitué, hétéroaryle éventuellement substitué, un radical polyéther ou un radical -CH₂-polyéther.
R₆ représente H, alkyle inférieur ou -OR₁₀,
R₇ représente H, un atome d'halogène, alkyle, linéaire ou ramifié en C₁-C₂₀, -OR₁₀ ou -OCOR₁₁ ou un radical polyéther,
R₈ représente H, alkyle inférieur ou -COR₁₁,
R₉ représente H, alkyle inférieur, -OR₁₄ ou 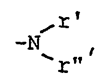
R₁₀ représente H ou alkyle inférieur,
R₁₁ représente alkyle inférieur,
R₁₂ représente H, alkyle, linéaire ou ramifié, en C₁-C₂₀, mono- ou polyhydroxyalkyle, aryle ou aralkyle éventuellement substitué,
R₁₃ représente H, alkyle inférieur, -OR₁₀, aryle ou 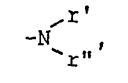
R₁₄ représente H, alkyle, linéaire ou ramifié, en C₁-C₂₀, alkényle, mono- ou polyhydroxyalkyle, aryle ou aralkyle éventuellement substitué, ou un reste de sucre,
r' et r", identiques ou différents, représentent H, OH, alkyle inférieur, mono- ou polyhydroxyalkyle, aryle éventuellement substitué, un reste d'aminoacide, un reste de peptide ou r' et r", pris ensemble, forment un hétérocycle,
et les sels des composés de formule (I) lorsque R₁ représente une fonction acide carboxylique ainsi que les isomères optiques et géométriques desdits composés de formule (I).

Lorsque les composés selon l'invention se présentent sous forme d'un sel, il s'agit de préférence d'un sel d'un métal alcalin ou alcalino-terreux, ou encore de zinc ou d'une amine organique.

Selon la présente invention, on entend par alkyle inférieur un radical en C₁-C₆, de préférence les radicaux méthyle, éthyle, isopropyle, butyle, tertiobutyle, et hexyle.

Par alkyle, linéaire ou ramifié, en C₁-C₁₅, on entend notamment les radicaux méthyle, éthyle, propyle, 2-éthyl-hexyle, octyle et dodécyle. Lorsque le radical alkyle est en C₁-C₂₀ on entend en outre les radicaux hexadécyle et octadécyle.

Par cycloalkyle, on entend un radical mono- ou polycyclique ayant de 5 à 10 atomes de carbone éventuellement substitué notamment un radical cyclopentyle, cyclohexyle, 1-méthylcyclohexyle ou 1-adamantyle.

Par monohydroxyalkyle, on entend un radical ayant de préférence 1 à 6 atomes de carbone, notamment un radical hydroxy-méthyle, 2-hydroxy-éthyle, 2-hydroxypropyle ou 3-hydroxypropyle.

Par polyhydroxyalkyle, on entend un radical ayant de préférence 3 à 6 atomes de carbone et de 2 à 5 groupes hydroxyles tels que les radicaux 2,3-dihydroxypropyle, 2,3,4-trihydroxybutyle, 2,3,4,5-tétrahydroxypentyle ou le reste du pentaérythritol.

Par radical polyéther, on entend un radical ayant de 2 à 6 atomes de carbone interrompu par au moins deux atomes d'oxygène tel que les radicaux méthoxyméthoxy, méthoxyéthoxy et méthoxyéthoxyméthoxy.

Par radical -CH₂-polyéther, on entend un radical choisi de préférence parmi les radicaux méthoxyméthoxyméthyle, éthoxyméthoxyméthyle ou méthoxyéthoxyméthoxyméthyle.

Par aryle, on entend de préférence un radical phényle éventuellement substitué par au moins un halogène, un alkyle inférieur, un hydroxyle, un alkoxy en C₁-C₃, une fonction nitro, un radical polyéther ou une fonction amino éventuellement protégée par un groupe acétyle ou éventuellement substitué par au moins un alkyle inférieur ou un alkoxy en C₁-C₆.

Par aralkyle, on entend de préférence le radical benzyle ou phénéthyle éventuellement substitué par au moins un halogène, un alkyle inférieur, un hydroxyle, un alkoxy en C₁-C₃, une fonction nitro, un radical polyéther ou une fonction amino éventuellement protégée par un groupe acétyle ou éventuellement substitué par au moins un alkyle inférieur ou un alkoxy en C₁-C₆.

Par radical hétéroaryle on entend de préférence un radical pyridyle, furyle ou thiényle, éventuellement substitué par au moins un halogène, un alkyle inférieur, un hydroxyle, un alkoxy en C₁-C₃, une fonction nitro, un radical polyéther ou une fonction amino éventuellement protégée par un groupe acétyle ou éventuellement substitué par au moins un alkyle inférieur ou un alkoxy en C₁-C₆.

Par alkényle, on entend un radical ayant de préférence 2 à 5 atomes de carbone et présentant une ou plusieurs insaturations éthyléniques, tel que plus particulièrement le radical allyle.

Par reste de sucre, on entend un reste dérivant notamment du glucose, du galactose ou du mannose, ou bien encore de l'acide glucuronique.

Par reste d'aminoacide, on entend notamment un reste dérivant de la lysine, de la glycine ou de l'acide aspartique, et par reste de peptide on entend plus particulièrement un reste de dipeptides ou de tripeptides résultant de la combinaison d'acides aminés.

Par hétérocycle, on entend de préférence un radical pipéridino, morpholino, pyrrolidino ou pipérazino, éventuellement substitué en position 4 par un alkyle inférieur en C₁-C₆ ou un mono- ou polyhydroxyalkyle tels que définis ci-dessus.

Lorsque R₄, R₅ et/ou R₇ représentent un atome d'halogène, celui-ci est de préférence un atome de fluor, de chlore ou de brome.

Selon une forme de réalisation préférée, les composés selon l'invention répondent aux formules générales (II) et (III) suivantes : 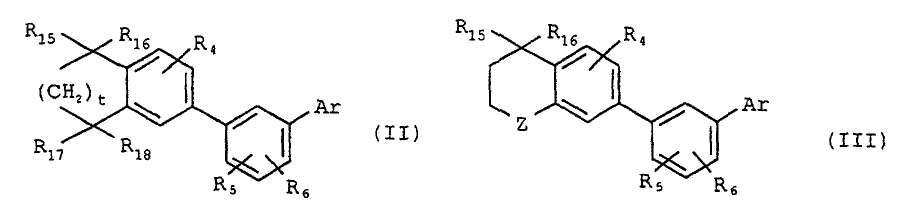 dans lesquelles :
Ar représente un radical de formule (a) ou (b) suivante : 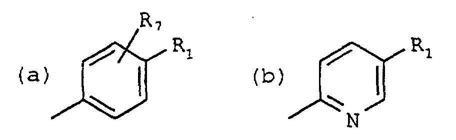
R₁, R₄, R₅, R₆, R₇ et Z ayant les mêmes significations que celles données ci-dessus pour la formule (I),
R₁₅, R₁₆, R₁₇ et R₁₈, identiques ou différents, représentent H ou -CH₃, et
t est 1 ou 2.

Parmi les composés des formules (I) à (III) ci-dessus selon la présente invention, on peut notamment citer les suivants :
- acide 4-[4-hydroxy-3-(5,6,7,8-tétrahydro-5,5,8,8-tétra-méthyl-2-naphtyl)phényl]benzoïque, et son ester méthylique,
- acide 4-[4-(5-hydroxypentyloxy)-3-(5,6,7,8-tétrahydro-5,5,8,8-tétraméthyl-2-naphtyl)phényl]benzoïque, et son ester méthylique,
- acide 4-[4-(6-hydroxyhexyloxy)-3-(5,6,7,8-tétrahydro-5,5,8,8-tétraméthyl-2-naphtyl)phényl]benzoïque, et son ester méthylique,
- acide 4-[4-(7-hydroxyheptyloxy)-3-(5,6,7,8-tétrahydro-5,5,8,8-tétraméthyl-2-naphtyl)phényl]benzoïque,
- acide 4-[4-(8-hydroxyoctyloxy)-3-(5,6,7,8-tétrahydro-5,5,8,8-tétraméthyl-2-naphtyl)phényl]benzoïque,
- acide 4-[4-(9-hydroxynonyloxy)-3-(5,6,7,8-tétrahydro-5,5,8,8-tétraméthyl-2-naphtyl)phényl]benzoïque,
- acide 4-[4-méthoxy-3-(5,6,7,8-tétrahydro-5,5,8,8-tétraméthyl-2-naphtyl) phényl]benzoïque,
- acide 4-[4-méthoxyéthoxyméthoxy-3-(5,6,7,8-tétrahydro-5,5,8,8-tétraméthyl-2-naphtyl)phényl]benzoïque,
- acide 4-[4-benzyloxy-3-(5,6,7,8-tétrahydro-5,5,8,8-tétraméthyl-2-naphtyl)phényl]benzoïque,
- acide 4'-(2,3-dihydroxy-propoxy)-3'-(5,5,8,8-tétraméthyl-5,6,7,8-tétrahydro-naphthalèn-2-yl)-biphényl-4-carboxylique (racémique),
- acide 4'-(2,2-diméthyl-[1,3]dioxolan-4-ylméthoxy)-3'-(5,5,8,8-tétraméthyl-5,6,7,8-tétrahydro-naphthalèn-2-yl)-biphényl-4-carboxylique (racémique),
- acide 4'-(2-morpholin-4-yl-éthoxy)-3'-(5,5,8,8-tétraméthyl-5,6,7,8-tétrahydro-naphthalèn-2-yl)-biphényl-4-carboxylique,
- 2'-(5,5,8,8-tétraméthyl-5,6,7,8-tétrahydronaphthalèn-2-yl)-[1,1';4',1"]terphényl-4"-carboxylate de méthyle,
- acide 2'-(5,5,8,8-tétraméthyl-5,6,7,8-tétrahydronaphthalèn-2-yl)-[1,1';4',1"]terphényl-4"-carboxylique,
- acide 4-méthoxyméthoxy-2'-(5,5,8,8-tétraméthyl-5,6,7,8-tétrahydro-naphthalèn-2-yl)-[1,1';4',1"]terphényl-4"-carboxylique,
- acide 4-hydroxy-2'-(5,5,8,8-tétraméthyl-5,6,7,8-tétrahydro-naphthalèn-2-yl)-[1,1';4',1"]terphényl-4"-carboxylique,
- acide 4-méthoxy-2'-(5,5,8,8-tétraméthyl-5,6,7,8-tétrahydro-naphthalèn-2-yl)-[1,1';4',1'']terphényl-4''-carboxylique,
- acide 3-méthoxyméthoxy-2'-(5,5,8,8-tétraméthyl-5,6,7,8-tétrahydro-naphthalèn-2-yl)-[1,1';4',1'']terphényl-4''-carboxylique,
- acide 3-hydroxy-2'-(5,5,8,8-tétraméthyl-5,6,7,8-tétrahydro-naphthalèn-2-yl)-[1,1';4',1'']terphényl-4''-carboxylique,
- acide 3-méthoxy-2'-(5,5,8,8-tétraméthyl-5,6,7,8-tétrahydro-naphthalèn-2-yl)-[1,1';4',1"]terphényl-4"-carboxylique,
- acide 2-méthoxyméthoxy-2'-(5,5,8,8-tétraméthyl-5,6,7,8-tétrahydro-naphthalèn-2-yl)-[1,1';4',1"]terphényl-4"-carboxylique,
- acide 2-hydroxy-2'-(5,5,8,8-tétraméthyl-5,6,7,8-tétrahydro-naphthalèn-2-yl)-[1,1';4',1"]terphényl-4"-carboxylique,
- acide 2-méthoxy-2'-(5,5,8,8-tétraméthyl-5,6,7,8-tétrahydro-naphthalèn-2-yl)-[1,1';4,1'']terphényl-4"-carboxylique,
- acide 2'-méthoxyméthoxy-5'-(5,5,8, 8-tétraméthyl-5,6,7,8-tétrahydro-naphthalèn-2-yl)-biphényl-4-carboxylique,
- acide 2'-méthoxy-5'-(5,5,8,8-tétraméthyl-5,6,7,8-tétrahydro-naphthalèn-2-yl)-biphényl-4-carboxylique,
- acide 2'-propyloxy-5'-(5,5,8,8-tétraméthyl-5,6,7,8-tétrahydro-naphthalèn-2-yl)-biphényl-4-carboxylique,
- acide 2'-hydroxy-5'-(5,5,8,8-tétraméthyl-5,6,7,8-tétrahydro-naphthalèn-2-yl)-biphényl-4-carboxylique,
- acide 4'-(5,5,8,8-tétraméthyl-5,6,7,8-tétrahydro-naphthalèn-2-yl)-[1,1';2',1'']terphényl-4''-carboxylique,
- acide 2'-méthoxyméthoxy-3'-(5,5,8,8-tétraméthyl-5,6,7,8-tétrahydro-naphthalèn-2-yl)-biphényl-4-carboxylique,
- acide 2'-hydroxy-3'-(5,5,8,8-tétraméthyl-5,6,7,8-tétrahydro-naphthalèn-2-yl)-biphényl-4-carboxylique,
- acide 2'-méthoxy-3'-(5,5,8,8-tétraméthyl-5,6,7,8-tétrahydro-naphthalèn-2-yl)-biphényl-4-carboxylique,
- acide 3'-méthoxyméthoxyméthyl-5'-(5,5,8,8-tétra méthyl-5,6,7,8-tétrahydro-naphthalèn-2-yl)-biphényl-4-carboxylique,
- acide 3'-hydroxyméthyl-5'-(5,5,8,8-tétraméthyl-5,6,7,8-tétrahydro-naphthalèn-2-yl)-biphényl-4-carboxylique,
- acide 2'-(4,4-diméthyl-thiochroman-7-yl)-[1,1';4',1'']terphényl-4''-carboxylique,
- acide 2'-(4,4-diméthyl-thiochroman-6-yl)-[1,1';4',1'']terphényl-4''-carboxylique,
- acide 2'-(3,5,5,8,8-pentaméthyl-5,6,7,8-tétrahydro-naphthalèn-2-yl)-[1,1';4',1"]terphényl-4"-carboxylique,
- acide 2'-(3-méthoxyméthoxy-5,5,8,8-tetraméthyl-5,6,7,8-tétrahydro-naphthalèn-2-yl)-[1,1';4',1"]terphényl-4"-carboxylique,
- acide 2'-(3-hydroxy-5,5,8,8-tétraméthyl-5,6,7,8-tétrahydro-naphthalèn-2-yl)-[1,1';4',1"]terphényl-4"-carboxylique,
- acide 2'-(3-méthoxy-5,5,8,8-tétraméthyl-5,6,7,8-tétrahydro-naphthalèn-2-yl]-[1,1';4',1"]terphényl-4"-carboxylique,
- acide 2'-(3-propyloxy-5,5,8,8-tétraméthyl-5,6,7,8-tétrahydro-naphthalèn-2-yl)-[1,1';4',1'']terphényl-4''-carboxylique,
- acide 3"-méthyl-2'-(5,5,8,8-tétraméthyl-5,6,7,8tétrahydro-naphthalèn-2-yl)-[1,1';4',1"]terphényl-4"-carboxylique,
- acide 2"-hydroxy-2'-(5,5,8,8-tétraméthyl-5,6,7,8-tétrahydro-naphthalèn-2-yl)-[1,1';4'1"]terphényl-4"-carboxylique,
- acide 2"-méthoxyméthoxy-2'-(5,5,8,8-tétraméthyl-5,6,7,8-tétrahydro-naphthalèn-2-yl)-[1,1';4',1"]terphényl-4"-carboxylique,
- acide 2"-méthoxy-2'-(5,5,8,8-tétraméthyl-5,6,7,8-tétrahydro-naphthalèn-2-yl)-[1,1';4',1"]terphényl-4"-carboxylique,
- acide 2"-propyloxy-2'-(5,5,8,8-tétraméthyl-5,6,7,8-tétrahydro-naphthalèn-2-yl)-[1,1';4',1"]terphényl-4"-carboxylique,
- acide 3''-hydroxy-2'-(5,5,8,8-tétraméthyl-5,6,7,8-tétrahydro-naphthalèn-2-yl)-[1,1';4',1"]terphényl-4"-carboxylique,
- acide 6-[2-(5,5,8,8-tétraméthyl-5,6,7,8-tétrahydro-naphthalèn-2-yl)-biphényl-4-yl]-nicotinique,
- acide 5-[2-(5,5,8,8-tétraméthyl-5,6,7,8-tétrahydro-naphthalèn-2-yl)-biphényl-4-yl]-pyridine-2-carboxylique,
- acide 2'-(5,5,8,8-tétraméthyl-5,6,7,8-tétrahydro-naphthalèn-2-yl)-[1,1';4',1'']terphényl-4"-hydroxamique,
- 2'-(5,5,8,8-tétraméthyl-5,6,7,8-tétrahydro-naphthalèn-2-yl)-[1,1';4',1"]terphényl-4"-ol,
- [2'-(5,5,8,8-tétraméthyl-5,6,7,8-tétrahydro-naphthalèn-2-yl)-[1,1';4',1"]terphényl-4"-yl]-méthanol,
- 2'-(5,5,8,8-tétraméthyl-5,6,7,8-tétrahydro-naphthalèn-2-yl)-[1,1';4',1"]terphényl-4"-carbaldéhyde,
- acide 4'-méthoxycarbonylméthoxy-3'-(5,5,8,8-tétraméthyl-5,6,7,8-tétrahydro-naphthalèn-2-yl)-biphényl-4-carboxylique,
- acide 4'-carboxyméthoxy-3'-(5,5,8,8-tétraméthyl-5,6,7,8-tétrahydro-naphthalèn-2-yl)-biphényl-4-carboxylique,
- acide 4'-(5-éthoxycarbonyl-pentyloxy)-3'-(5,5,8,8-tétraméthyl-5,6,7,8-tétrahydro-naphthalèn-2-yl)-biphényl-4-carboxylique,
- acide 4'-(5-carboxy-pentyloxy)-3'-(5,5,8,8-tétraméthyl-5,6,7,8-tétrahydro-naphthalèn-2-yl)-biphényl-4-carboxylique,
- 2'-(5,5,8,8-tétraméthyl-5,6,7,8-tétrahydro-naphthalèn-2-yl)-[1,1'4',1"]terphényl-4"-carboxamide,
- N-éthyl-2'-(5,5,8,8-tétraméthyl-5,6,7,8-tétrahydro-naphthalèn-2-yl)-[1,1';4',1"]terphényl-4"-carboxamide,
- N,N-diéthyl-2'-(5,5,8,8-tétraméthyl-5,6,7,8-tétrahydro-naphthalèn-2-yl)-[1,1';4',1'']terphényl-4"-carboxamide,
- morpholin-4-yl-[2'-(5,5,8,8-tétraméthyl-5,6,7,8-tétrahydro-naphthalèn-2-yl)-[1,1';4',1"]terphényl-4"-yl]-méthanone,
- (4-hydroxy-phényl)-2'-(5,5,8,8-tétraméthyl-5,6,7,8-tétrahydro-naphthalèn-2-yl)-[1,1';4',1"]terphényl-4"-carboxamide,
- acide 3-(5,5,8,8-tétraméthyl-5,6,7,8-tétrahydro-naphthalèn-2-yl)-biphényl-4-carboxyméthyle-4'-carboxylique,
- acide 3-(5,5,8,8-tétraméthyl-5,6,7,8-tétrahydro-naphthalèn-2-yl)-biphényl-4,4'-dicarboxylique,
- acide 3'-méthoxyméthoxy-5'-(5,5,8,8-tétraméthyl-5,6,7,8-tétrahydro-naphthalèn-2-yl)-biphényl-4-carboxylique,
- acide 3'-méthoxy-5'-(5,5,8,8-tétraméthyl-5,6,7,8-tétrahydro-naphthalèn-2-yl)-biphényl-4-carboxylique,
- acide 3'-propyloxy-5'-(5,5,8,8-tétraméthyl-5,6,7,8-tétrahydro-naphthalèn-2-yl)-biphényl-4-carboxylique,
- acide 3'-hydroxy-5'-(5,5,8,8-tétraméthyl-5,6,7,8-tétrahydro-naphthalèn-2-yl)-biphényl-4-carboxylique,
- acide 4'-(5,5,8,8-tétraméthyl-5,6,7,8-tétrahydro-naphthalèn-2-yl)-[1,1';3',1"]terphényl-4"-carboxylique,
- acide 4'-(5-carboxamido-pentyloxy)-3'-(5,5,8,8-tétraméthyl-5,6,7,8-tétrahydro-naphthalèn-2-yl)-biphényl-4-carboxylique,
- acide 3'-méthoxycarbonyl-5'-(5,5,8,8-tétraméthyl-5,6,7,8-tétrahydro-naphthalèn-2-yl)-biphényl-4-carboxylique,
- acide 3'-carboxyl-5'-(5,5,8,8-tétraméthyl-5,6,7,8-tétrahydro-naphthalèn-2-yl)-biphényl-4-carboxylique,
- acide 2'-(4-hydroxy-5,5,8,8-tétraméthyl-5,6,7,8-tétrahydro-naphthalèn-2-yl)-[1,1';4',1'']terphényl-4''-carboxylique,
- acide 2'-(4-méthoxy-5,5,8,8-tétraméthyl-5,6,7,8-tétrahydro-naphthalèn-2-yl)-[1,1';4',1'']terphényl-4''-carboxylique,
- acide 2'-(4-propyloxy-5,5,8,8-tétraméthyl-5,6,7,8-tétrahydro-naphthalèn-2-yl)-[1,1';4',1'']terphényl-4"-carboxylique,
- acide 2'-(4-méthoxyméthoxy-5,5,8,8-tétraméthyl-5,6,7,8-tétrahydro-naphthalèn-2-yl)-[1,1';4',1"]terphényl-4"-carboxylique,
- acide 2-[2-(5,5,8,8-tétraméthyl-5,6,7,8-tétrahydro-naphthalèn-2-yl)-biphényl-4-yl]-thiophene-4-carboxylique.

La présente invention a également pour objet les procédés de préparation des composés de formule (I) ci-dessus selon les schémas réactionnels donnés aux Tableaux A et B.

En se référant au Tableau A, les composés de formule (Ia) peuvent être obtenus par réaction de couplage de type Suzuki entre un dérivé boronique de formule (6) et un dérivé bromé bi-aromatique de formule (7). Le dérivé boronique de formule (6) est obtenu à partir du dérivé halogéné de formule (5), de préférence le dérivé bromé ou iodé. Le dérivé bromé bi-aromatique de formule (7) peut être obtenu par deux voies différentes faisant intervenir une réaction de couplage de type Suzuki. La première consistant à faire réagir un composé aromatique halogéné de formule (1) avec un dérivé bromé boronique de formule (2) et la seconde en faisant réagir un dérivé boronique aromatique de formule (3) avec un dérivé aromatique idobromé de formule (4).

Les conditions de réaction de ces différentes étapes sont essentiellement décrites dans :
- N. MIYAURA Synthetic Communications **1981,** *11(7)*, 513-9,
- A. SUZUKI Synlett **1990,** 221,
- A. R. MARTIN Acta Chemica Scandinavia **1993,** *47,* 221-30,
- G. MARCK Tetrahedron Letters **1994,** vol.35, No. 20, 3277-80,
- T. WALLOW J. Org. Chem. **1994,** *59*, 5034-7,
- H. ZHANG Tetrahedron Letters **1996,** vol.37, No. 7, 1043-4.

Les dérivés boroniques des formules (2), (3) et (6) peuvent être préparés selon les deux méthodes suivantes :
(a) soit par réaction avec le butyllithium puis avec un borate d'alkyle, de préférence le borate de triisopropyle ou de triméthyle, puis hydrolyse avec de l'acide chlorhydrique,
(b) soit par réaction avec l'ester pinacolique de l'acide diboronique selon la méthode décrite par T. ISHIYAMA J. Org. Chem. **1995,** *60,* 7508-10.

A partir du composé de formule (Ia) on peut accéder aux composés des formules (Ib) et (Ic).

Les composés de formule (Ib) peuvent être obtenus à partir des composés de formule (Ia) (R₅=OH) par réaction d'un dérivé halogéné (9) en présence d'un solvant tel que l'acétone, la méthylcétone, le DMF et d'une base telle que le carbonate de potassium ou l'hydrure de sodium.

Les composés de formule (Ic) peuvent être obtenus à partir des composés de formule (Ia) (R₅=OH) par réaction acylation classique à partir d'un acide (10).

Les composés de formule (Id) peuvent être obtenus à partir de composés de formule (Ia) (R₅=OH) qui sont dans une première étape transformés en dérivés triflates de formule (8) puis dans une deuxième étape, mis à réagir soit dans les conditions de réaction de type Suzuki avec un dérivé boronique aromatique (12) soit dans les conditions de réaction de type Stille avec un dérivé stannique aromatique (11) selon la méthode décrite par A.M. ECHAVARREN J. Am. Chem. Soc. **1987,** *109,* 5478-86.

En se référant maintenant au Tableau B, les composés des formules (Ie), (If) et (Ig) peuvent être directement obtenus à partir des dérivés triflates de formule (8) par carbonylation en présence d'un catalyseur au palladium et respectivement à l'aide d'un dérivé alcoolique, d'une amine ou d'un trialkylsilane selon les méthodes décrites par J.K. STILLE, Angew Chem. Int., Ed. Engl. **1996,** 508-524 et H. KOTSUKI, Synthesis **1996,** 470-2.

Les composés de formule (Ih) peuvent être également obtenus à partir des dérivés triflates de formule (8) par réaction avec un ester acrylique (13) en présence d'un catalyseur au palladium selon la méthode décrite dans J. Med. Chem. 1990, vol.33, No.7, 1919-24. A partir du composé insaturé de formule (Ih), on peut directement accéder par hydrogénation catalytique au composé de formule (Ii).

Les composés de formule (Ij) peuvent être obtenus toujours à partir des dérivés triflates de formule (8) par réaction avec des dérivés stanniques tels que le vinyltributylétain ou l'allyltributylétain (14) en présence d'un catalyseur au palladium. Le composé intermédiaire obtenu de formule (15) est alors soumis à une réaction d'oxydation avec le tétraoxyde d'osmium dans les conditions décrites dans J. Org. Chem. **1990**, vol.55, No.3, 906-9 et J. Med. Chem. **1991**, vol.34, No.5, 1614-23.

Lorsque dans les composés de formule (I) selon l'invention, R₁ représente le radical -COOH, ceux-ci sont préparés selon deux voies de synthèse différentes :
(a) La première consiste à protéger la fonction acide carboxylique par un groupe protecteur de type alkyle, allyle, benzyle ou tert-butyle.
   Lorsque le groupe protecteur est un alkyle, la déprotection est obtenue au moyen de soude ou d'hydroxyde de lithium dans un solvant alcoolique tel que le méthanol ou le THF.
   Lorsque le groupe protecteur est un radical allyle, la déprotection est réalisée au moyen d'un catalyseur tel certains complexes de métaux de transition en présence d'une amine secondaire telle que la morpholine.
   Lorsque le groupe protecteur est un radical benzyle, la déprotection est réalisée en présence d'hydrogène au moyen d'un catalyseur tel que le palladium sur charbon.
   Enfin, lorsque le groupe protecteur est un radical tert-butyle, la déprotection est réalisée au moyen d'iodure de triméthylsilane.
(b) La deuxième consiste à partir du composé phénolique correspondant qui est transformé en triflate, puis soumis à une carbonylation en présence d'un catalyseur au palladium.

Lorsque dans les composés de formule (I) selon l'invention R₁ représente une fonction alcool, ceux-ci peuvent être obtenus :
(a) soit à partir des dérivés aldéhydiques correspondants par action d'un hydrure alcalin tel que le borohydrure de sodium dans un solvant alcoolique tel que le méthanol,
(b) soit à partir des dérivés acides de formule (Ie)(R₁₀=H) par réduction avec l'hydrure double de lithium et d'aluminium.

Lorsque dans les composés de formule (I) selon l'invention, R₁ représente une fonction aldéhyde, ceux-ci peuvent être obtenus par oxydation des alcools correspondants en présence d'oxyde de manganèse, de dichromate de pyridinium ou du réactif de Swern.

Enfin, lorsque dans les composés de formule (I) selon l'invention, R₁ représente une fonction amide, ceux-ci peuvent être obtenus par réaction des chlorures d'acides, obtenus à partir des acides carboxyliques correspondants, avec des amines aliphatiques, aromatiques, hétérocycliques en présence de dicyclohexylcarbodiimide ou de carbonyldiimidazole.

La présente invention a également pour objet à titre de médicament les composés de formule (I) telle que définie ci-dessus.

Ces composés présentent une activité agoniste ou antagoniste vis-à-vis de l'expression d'un ou plusieurs marqueurs biologiques dans le test de différenciation des cellules (F9) de tératocarcinome embryonnaire de la souris (Skin Pharmacol. 3, p.256-267, **1990**) et/ou sur la différenciation des kératinocytes humains in vitro (Skin Pharmacol. 3, p.70-85, **1990**). Ces tests sus-mentionnés montrent les activités des composés dans les domaines de la différenciation et de la prolifération. Les activités peuvent aussi être mesurées dans des tests de transactivation cellulaire à l'aide de récepteurs recombinants RARs selon la méthode B.A. Bernard et al., Biochemical and Bio-physical Research Communication, **1992,** vol. 186, 977-983.

Les composés selon l'invention conviennent particulièrement bien dans les domaines des traitements suivants :
1) pour traiter les affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle,
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée telle que l'eczéma, ou l'atopie respiratoire ou encore l'hypertrophie gingivale ; les composés peuvent également être utilisés dans certaines affections inflammatoires ne présentant pas de trouble de la kératinisation,
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires,
5) pour traiter d'autres désordres dermatologiques tels que les dermatoses bulleuses et les maladies du collagène,
6) pour traiter certains troubles ophtalmologiques, notamment les cornéopathies,
7) pour réparer ou lutter contre le vieillissement de la peau, qu'il soit photoinduit ou chronologique ou pour réduire les pigmentations et les kératoses actiniques, ou toutes pathologies associées au vieillissement chronologique ou actinique,
8) pour prévenir ou guérir les stigmates de l'atrophie épidermique et/ou dermique induite par les corticostéroïdes locaux ou systémiques, ou toute autre forme d'atrophie cutanée,
9) pour prévenir ou traiter les troubles de la cicatrisation ou pour prévenir ou pour réparer les vergetures,
10) pour lutter contre les troubles de la fonction sébacée tels que l'hyperséborrhée de l'acné ou la séborrhée simple,
11) pour traiter ou prévenir des états cancéreux ou précancéreux,
12) pour traiter les affections inflammatoires telles que l'arthrite,
13) pour traiter toute affection d'origine virale au niveau cutané ou général,
14) pour prévenir ou traiter l'alopécie,
15) pour traiter les affections dermatologiques ou générales à composante immunologique, et
16) pour traiter les affections du système cardiovasculaire telles que l'artériosclérose.

Dans les domaines thérapeutiques mentionnés ci-dessus, les composés selon l'invention peuvent être avantageusement employés en combinaison avec d'autres composés à activité de type rétinoïde, avec les vitamines D ou leurs dérivés, avec des corticostéroïdes, avec des anti-radicaux libres, des α-hydroxy ou α-cétoacides ou leurs dérivés ou bien encore avec des bloqueurs de canaux ioniques. Par vitamines D ou leurs dérivés, on entend par exemple les dérivés de la vitamine D₂ ou D₃ et en particulier la 1,25-dihydroxyvitamine D₃. Par anti-radicaux libres, on entend par exemple l'α-tocophérol, la Super Oxyde Dismutase ou SOD, l'Ubiquinol ou certains chélatants de métaux. Par α-hydroxy ou α-céto acides ou leurs dérivés, on entend par exemple l'acide lactique, l'acide malique, l'acide citrique, l'acide glycolique, l'acide mandélique, l'acide tartrique, l'acide glycérique ou l'acide ascorbique ou leurs sels, amides ou esters. Enfin, par bloqueurs de canaux ioniques, on entend par exemple le Minoxidil (2,4-diamino-6-pipéridino-pyrimidine-3-oxyde) et ses dérivés.

La présente invention a également pour objet des compositions pharmaceutiques contenant au moins un composé de formule (I) telle que définie ci-dessus, l'un de ses isomères optiques ou géométriques ou un de ses sels.

Les compositions pharmaceutiques sont destinées notamment au traitement des affections susmentionnées, et sont caractérisées par le fait qu'elles contiennent un support pharmaceutiquement acceptable et compatible avec le mode d'administration retenu, au moins un composé de formule (I), l'un de ses isomères optiques ou géométriques ou un de ses sels.

L'administration des composés selon l'invention peut être effectuée par voie entérale, parentérale, topique ou oculaire.

Par voie entérale, les compositions peuvent se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, les compositions peuvent se présenter sous forme de solutions ou de suspensions pour perfusion ou pour injection.

Les composés selon l'invention sont généralement administrés à une dose journalière d'environ 0,01 mg/kg à 100 mg/kg en poids corporel, et ceci à raison de 1 à 3 prises.

Par voie topique, les compositions pharmaceutiques à base des composés selon l'invention sont plus particulièrement destinées au traitement de la peau et des muqueuses et peuvent alors se présenter sous forme d'onguents, de crèmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée du principe actif. Ces compositions par voie topique peuvent par ailleurs se présenter soit sous forme anhydre, soit sous une forme aqueuse, selon l'indication clinique.

Par voie oculaire, ce sont principalement des collyres.

Ces compositions à usage topique ou oculaire contiennent au moins un composé de formule (I) telle que définie ci-dessus, ou l'un de ses isomères optiques ou géométriques ou encore l'un de ses sels, à une concentration de préférence comprise entre 0,001 % et 5 % en poids par rapport au poids total de la composition.

Les composés de formule (I) selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photoinduit ou chronologique.

Dans le domaine cosmétique, les composés selon l'invention peuvent par ailleurs être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques tous ces derniers composés étant tels que définis ci-dessus.

La présente invention a donc également pour objet une composition cosmétique qui est caractérisée par le fait qu'elle contient dans un support cosmétiquement acceptable, au moins un composé de formule (I) telle que définie ci-dessus ou l'un de ses isomères optiques ou géométriques ou l'un de ses sels, ladite composition cosmétique pouvant notamment se présenter sous forme d'une crème, d'un lait, d'une lotion, d'un gel, de microsphères ou de nanosphères ou de vésicules lipidiques ou polymériques, d'un savon ou d'un shampooing.

La concentration en composé de formule (I) dans les compositions cosmétiques selon l'invention est avantageusement comprise entre 0,001 % et 3 % en poids par rapport au poids total de la composition.

Les compositions pharmaceutiques et cosmétiques selon l'invention peuvent en outre contenir des additifs inertes ou même pharmacodynamiquement ou cosmétiquement actifs ou des combinaisons de ces additifs et notamment : des agents mouillants, des agents dépigmentants tels que l'hydroquinone, l'acide azélaïque, l'acide caféique ou l'acide kojique ; des émollients ; des agents hydratants comme le glycérol, le PEG 400, la thiamorpholinone et ses dérivés ou bien encore l'urée ; des agents antiséborrhéiques ou antiacnéiques, tels que la S-carboxyméthylcystéine, la S-benzyl-cystéamine, leurs sels ou leurs dérivés, ou le peroxyde de benzoyle ; des antibiotiques comme l'érythromycine et ses esters, la néomycine, la clindamysine et ses esters, les tétracyclines ; des agents antifongiques tels que le kétokonazole ou les polyméthylène-4,5 isothiazolidones-3 ; des agents favorisant la repousse des cheveux, comme le Minoxidil (2,4-diamino-6-pipéridino-pyrimidine- 3-oxyde) et ses dérivés, le Diazoxide (7-chloro 3-méthyl 1,2,4-benzothiadiazine 1,1-dioxyde) et le Phénytoïn (5,4-diphényl-imidazolidine 2,4-dione) ; des agents anti-inflammatoires non stéroïdiens ; des caroténoïdes et, notamment le β-carotène ; des agents antipsoriatiques tels que l'anthraline et ses dérivés ; et enfin les acides eicosa-5,8,11,14-tétraynoïque et eicosa-5,8,11- trynoïque, leurs esters et amides.

Les compositions selon l'invention peuvent également contenir des agents d'amélioration de la saveur, des agents conservateurs tels que les esters de l'acide parahydroxybenzoïque, des agents stabilisants, des agents régulateurs d'humidité, des agents régulateurs de pH, des agents modificateurs de pression osmotique, des agents émulsionnants, des filtres UV-A et UV-B, des antioxydants tels que l'α-tocophérol, le butylhydroxyanisole ou le butylhydroxytoluène.

On va maintenant donner à titre d'illustration et sans aucun caractère limitatif, plusieurs exemples de préparation des composés actifs de formule (I) selon l'invention, ainsi que diverses formulations pharmaceutiques et cosmétiques à base de ces composés.

### EXEMPLES

### EXEMPLE 1

### Acide 4-[4-hydroxy-3-(5,6,7,8-tétrahydro-5,5,8,8-tétraméthyl-2-naphtyl) phényl]benzoïque.

(a) acide 5,6,7,8-tétrahydro-5,5,8,8-tétraméthyl-2-naphtylboronique.
   Dans un tricol et sous courant d'azote, on introduit 21,38 g (80,0 mmoles) de 5,6,7,8-tétrahydro-5,5,8,8-tétraméthyl-2-bromonaphtalène et 50 ml de THF. A -78°C, on ajoute goutte à goutte 38,4 ml (96,0 mmoles) de n-butyllithium (2,5M dans l'hexane) et agite une heure. A cette même température on ajoute goutte à goutte 27,7 ml (120,0 mmoles) de triisopropylborate et agite pendant 2 heures. A -50°C on ajoute 350 ml d'acide chlorhydrique (1N) et laisse remonter à la température ambiante. On extrait le milieu réactionnel avec du dichlorométhane, décante la phase organique, sèche sur sulfate de magnésium, évapore. On recueille 18,60 g (100%) de l'acide boronique attendu, sous la forme d'une huile qui cristallise lentement. Point de fusion 190-192°C.
   ¹H NMR (CDCl₃) δ 1,34 (s, 6H), 1,39 (s, 6H), 1,75 (s, 4H), 4,88 (s, 2H), 7,47 (d, 1H, *J* = 7,9 Hz), 7,97 (d, 1H, *J* = 7,9 Hz), 8,21 (s, 1H).
(b) 4-(4-hydroxyphényl)benzoate de méthyle (ou d'éthyle).
   Dans un ballon, on introduit 10,10 g (47,3 mmoles) de l'acide 4-(4-hydroxyphényl)benzoïque, 150 ml de méthanol (ou d'éthanol) et ajoute goutte à goutte 2,5 ml d'acide sulfurique concentré. On chauffe à reflux pendant douze heures, évapore à sec le milieu réactionnel. On reprend le résidu obtenu par un mélange d'eau et d'éther éthylique, décante la phase organique, lave à l'eau, sèche sur sulfate de magnésium, évapore. On recueille 10,60 g (98%) de l'ester attendu, sous la forme d'une huile incolore.
   ¹H NMR (ester méthylique) (CDCl₃) δ 3,87 (s, 3H), 6,90 (d, 2H, *J* = 8,5 Hz), 7,59 (d, 2H, *J* = 8,6 Hz), 7,74 (d, 2H, *J* = 8,4 Hz), 7,99 (d, 2H, *J* = 8,4 Hz), 9,77 (s, 1H).
(c) 4-(3-bromo-4-hydroxyphényl)benzoate de méthyle (ou d'éthyle).
   Dans un ballon, on introduit 9,35 g (41,0 mmoles) de 4-(4-hydroxyphényl) benzoate de méthyle (ou d'éthyle), 125 ml de dioxanne et 40 ml de THF. On ajoute 12,19 g (49,1 mmoles) de complexe Br₂/dioxanne et agite 24 heures à la température ambiante. On évapore à sec le milieu réactionnel, reprend par l'eau et l'acétate d'éthyle, décante la phase organique, sèche sur sulfate de magnésium, évapore. Le résidu obtenu est purifié par chromatographie sur colonne de silice, élué avec un mélange d'acétate d'éthyle et d'heptane (20/80). Après évaporation des solvants, on recueille 10,80 g (86%) du produit attendu, sous la forme de cristaux blancs de point de fusion 145-146°C (ester méthylique).
   ¹H NMR (ester méthylique) (CDCl₃) δ 3,94 (s, 3H), 5,74 (s, 1H), 7,11 (d, 1H, *J* = 8,5 Hz), 7,49 (dd, 1H, *J* = 8,5 / 2,1 Hz), 7,58 (d, 2H, *J* = 8,5 Hz), 7,74 (d, 1H, *J* = 2,1 Hz), 8,08 (d, 2H, *J* = 8,5 Hz).
(d) 4-[4-hydroxy-3-(5,6,7,8-tétrahydro-5,5,8,8-tétraméthyl-2-naphtyl)phényl] benzoate de méthyle (ou d'éthyle).
   Dans un tricol, on introduit 11,41 g (49,1 mmoles) d'acide 5,6,7,8-tétrahydro-5,5,8,8-tétraméthyl-2-naphtylboronique, 0,06 g (32,7 mmoles) de 4-(3-bromo-4-hydroxyphényl)benzoate de méthyle (ou d'éthyle), 640 ml de toluène et 39,3 ml (78,6 mmoles) d'une solution de carbonate de potassium (2M). On dégaze le milieu réactionnel par barbotage d'azote, ajoute 69 mg (0,06 mmole) de tétrakistriphénylphosphine palladium(0) et chauffe à 90°C pendant vingt heures. On évapore à sec le milieu réactionnel, reprend par l'eau et l'éther éthylique, acidifie. On décante la phase organique, sèche sur sulfate de magnésium, évapore. Le résidu obtenu est purifié par chromatographie sur colonne de silice élué avec du dichlorométhane. On recueille 11,57 g (85%) du produit attendu, sous la forme d'un solide jaune très clair de point de fusion 178-181°C (ester méthylique).
   ¹H NMR (ester méthylique) (CDCl₃) δ 1,32 (s, 6H), 1,33 (s, 6H), 1,73 (s, 4H), 3,93 (s, 3H), 5,52 (s, 1H), 7,09 (d, 1H, *J* = 9,1 Hz), 7,26 (dd, 1H, *J* = 7,1 / 1,9 Hz), 7,42 à 7,44 (m, 2H), 7,47 à 7,55 (m, 2H), 7,64 (d, 2H, *J* = 8,4 Hz), 8,08 (d, 2H, *J* = 8,4 Hz).
(e) acide 4-[4-hydroxy-3-(5,6,7,8-tétrahydro-5,5,8,8-tétraméthyl-2-naphtyl)phényl] benzoïque.
   Dans un ballon, on introduit 1,24 g (3,0 mmoles) de l'ester méthylique (ou éthylique) obtenu à l'exemple 1(d) et 7,5 ml de soude méthanolique (4N). On chauffe à reflux pendant quatre heures, verse le milieu réactionnel dans l'eau, acidifie, extrait avec de l'éther éthylique, décante la phase organique, sèche sur sulfate de magnésium, évapore. Le résidu obtenu est chromatographié sur une courte colonne de silice, élué avec de l'éther éthylique. On recueille 1,00 g (83%) d'acide 4-[4-hydroxy-3-(5,6,7,8-tétrahydro-5,5,8,8-tétraméthyl-2-naphtyl)phényl] benzoïque sous la forme de cristaux blancs de point de fusion 240-241°C.
   ¹H NMR (CDCl₃) δ 1,33 (s, 6H), 1,34 (s, 6H), 1,74 (s, 4H), 7,10 (d, 1H, *J* = 8,7 Hz), 7,26 (dd, 1H, *J* = 7,2 / 1,8 Hz), 7,42 à 7,48 (m, 2H), 7,54 à 7,58 (m, 2H), 7,69 (d, 2H, *J* = 8,4 Hz), 8,16 (d, 2H, *J* = 8,4 Hz).

### EXEMPLE 2

### Acide 4-[4-(5-hydroxypentyloxy)-3-(5,6,7,8-tétrahydro-5,5,8,8-tétraméthyl-2-naphtyl)phényl]benzoïque.

(a) 4-[4-(5-acétoxypentyloxy)-3-(5,6,7,8-tétrahydro-5,5,8,8-tétraméthyl-2-naphtyl) phényl]benzoate de méthyle.
   Dans un ballon, on introduit 1,66 g (4,0 mmoles) de l'ester méthylique obtenu à l'exemple 1(d), 150 ml d'acétone et 2,21 g (16,0 mmoles) de carbonate de potassium. On ajoute 2,67 ml (16,0 mmoles) d'acétate de 5-bromopentyle et chauffe à reflux pendant huit heures. On évapore à sec le milieu réactionnel, reprend le résidu par l'acétate d'éthyle et l'eau, décante la phase organique, sèche sur sulfate de magnésium, évapore. On recueille 2,20 g (100%) du produit attendu, sous forme d'une huile incolore.
   ¹H NMR (CDCl₃) δ 1,32 (s, 12H), 1,66 (s, 4H), 1,45 à 2,05 (m, 6H), 3,41 (t, 2H, *J* = 6,7 Hz), 3,93 (s, 3H), 4,04 (t, 2H, *J* = 6,7 Hz), 7,04 (d, 1H, *J* = 8,5 Hz), 7,29 à 7,38 (m, 2H), 7,52 à 7,58 (m, 2H), 7,62 (d, 1H, *J* = 2,4 Hz), 7,66 (d, 2H, *J* = 8,4 Hz), 7,87 (d, 2H, *J* = 8,3 Hz).
(b) acide 4-[4-(5-hydroxypentyloxy)-3-(5,6,7,8-tétrahydro-5,5,8,8-tétraméthyl-2-naphtyl)phényl]benzoïque.
   De manière analogue à l'exemple 1(e), à partir de 2,20 g (4,0 mmoles) de l'ester méthylique obtenu à l'exemple 2(a), on obtient après reprise dans un mélange éther éthylique / hexane (10/90) et filtration, 1,45 g (74%) d'acide 4-[4-(5-hydroxypentyloxy)-3-(5,6,7,8-tétrahydro-5,5,8,8-tétraméthyl-2-naphtyl)phényl]benzoïque, sous la forme d'un solide blanc de point de fusion 195-196°C.
   ¹H NMR (CDCl₃) δ 1,32 (s, 12H), 1,49 à 1,61 (m, 3H), 1,72 (s, 4H), 1,77 à 1,85 (m, 3H), 3,59 (t, 2H, *J* = 6,1 Hz), 4,03 (t, 2H, *J* = 6,4 Hz), 7,04 (d, 1H, *J* = 8,6 Hz), 7,30 à 7,38 (m, 2H), 7,54 (dd, 1H, *J* = 8,5 / 2,2 Hz), 7,58 à 7,62 (m, 2H), 7,65 (d, 2H, *J* = 8,4 Hz), 8,10 (d, 2H, *J* = 8,3 Hz).

### EXEMPLE 3

### Acide 4-[4-(6-hydroxyhexyloxy)-3- (5, 6, 7,8-tétrahydro-5,5,8,8-tétraméthyl-2-naphtyl)phényl]benzoïque.

(a) 4-[4-(6-hydroxyhexyloxy)-3-(5,6,7,8-tétrahydro-5,5,8,8-tétraméthyl-2-naphtyl) phényl]benzoate de méthyle.
   De manière analogue à l'exemple 2(a), par réaction de 1,66 g de l'ester méthylique obtenu à l'exemple 1(d), avec 2,1 ml (16,0 mmoles) de 6-bromohexanol, on obtient 2,10 g (100%) du produit attendu, sous forme d'une huile incolore.
   ¹H NMR (CDCl₃) δ 1,33 (s, 12H), 1,41 à 1,93 (m, 8H), 1,72 (s, 4H), 3,65 (t, 2H, *J* = 6,3 Hz), 3,93 (s, 3H), 4,02 (t, 2H, *J* = 6,4 Hz), 7,04 (d, 1H, *J* = 8,5 Hz), 7,30 à 7,38 (m, 2H), 7,54 (dd, 1H, *J* = 8,5 / 2,3 Hz), 7,59 à 7,62 (m, 2H), 7,66 (d, 2H, *J* = 8,4 Hz), 8,08 (d, 2H, *J* = 8,3 Hz).
(b) acide 4-[4-(6-hydroxyhexyloxy)-3-(5,6,7,8-tétrahydro-5,5,8,8-tétraméthyl-2-naphtyl)phényl]benzoïque.
   De manière analogue à l'exemple 1(e), à partir de 2,10 g (4,0 mmoles) de l'ester méthylique obtenu à l'exemple 3(a), on obtient 1,70 g (86%) d'acide 4-[4-(6-hydroxyhexyloxy)-3-(5,6,7,8-tétrahydro-5,5,8,8-tétraméthyl-2-naphtyl)phényl]benzoïque, sous la forme d'un solide blanc de point de fusion 200-201°C.
   ¹H NMR (CDCl₃) δ 1,33 (s, 12H), 1,38 à 1,83 (m, 8H), 1,72 (s, 4H), 3,59 (t, 2H, *J* = 6,4 Hz), 4,03 (t, 2H, *J* = 6,5 Hz), 7,04 (d, 1H, *J* = 8,6 Hz), 7,30 à 7,38 (m, 2H), 7,54 (dd, 1H, *J* = 8,5/ 2,3 Hz), 7,60 à 7,62 (m, 2H), 7,65 (d, 2H, *J* = 8,4 Hz), 8,10 (d, 2H, *J* = 8,4 Hz).

### EXEMPLE 4

### Acide 4-[4-(7-hydroxyheptyloxy) -3- (5,6,7,8-tétrahydro-5,5,8,8-tétraméthyl-2-naphtyl)phényl]benzoïque.

(a) 4-[4-(7-hydroxyheptyloxy)-3-(5,6,7,8-tétrahydro-5,5,8,8-tétraméthyl-2-naphtyl) phényl]benzoate d'éthyle.
   De manière analogue à l'exemple 2(a), par réaction de 1,50 g (3,5 mmoles) de l'ester éthylique obtenu à l'exemple 1(d), avec 1,06 g (5,4 mmoles) de 7-bromoheptanol, on obtient 1,43 g (75%) du produit attendu, sous forme d'une huile incolore.
   ¹H NMR (CDCl₃) δ 1,18 à 1,24 (m, 2H), 1,33 (s, 12H), 1,41 (t, 3H, *J* = 7,1 Hz), 1,52 à 1,58 (m, 4H), 1,72 (s, 4H), 1,72 à 1,82 (m, 4H), 3,60 à 3,64 (m, 2H), 4,02 (t, 2H, *J=* 6,5 Hz), 4,39 (q, 2H, *J* = 7,1 Hz), 7,04 (d, 1H, *J* = 8,5 Hz), 7,32 à 7,38 (m, 2H), 7,54 (dd, 1H, *J* = 8,4 / 2,4 Hz), 7,59 à 7,63 (m, 2H), 7,65 (d, 2H, *J* = 8,5 Hz), 8,09 (d, 2H, *J* = 8,4 Hz).
(b) acide 4-[4-(7-hydroxyheptyloxy)-3-(5,6,7,8-tétrahydro-5,5,8,8-tétraméthyl-2-naphtyl)phényl]benzoïque.
   De manière analogue à l'exemple 1(e), à partir de 1,40 g (2,6 mmoles) de l'ester éthylique obtenu à l'exemple 4(a), on obtient 1,15 g (87%) d'acide 4-[4-(7-hydroxyheptyloxy)-3-(5,6,7,8-tétrahydro-5,5,8,8-tétraméthyl-2-naphtyl)phényl]benzoïque, sous la forme d'un solide blanc de point de fusion 168-172°C.
   ¹H NMR (CDCl₃) δ 1,21 à 1,43 (m, 6H), 1,33 (s, 12 H), 1,53 à 1,56 (m, 2H), 1,73 (s, 4H), 1,77 à 1,82 (m, 2H), 3,64 (t, 2H, *J* = 6,5 Hz), 4,02 (t, 2H, *J* = 6,5 Hz), 7,05 (d, 1H, *J* = 8,6 Hz), 7,31 à 7,38 (m, 2H), 7,55 (dd, 1H, *J* = 8,5 / 2,4 Hz), 7,59 (s, 1H), 7,64 (d, 1H, *J* = 2,4 Hz), 7,69 (d, 2H, *J* = 8,4 Hz), 8,16 (d, 2H, *J* = 8,4 Hz).

### EXEMPLE 5

### Acide 4-[4-(8-hydroxyoctyloxy)-3-(5,6,7,8-tétrahydro-5,5,8,8-tétraméthyl-2-naphtyl)phényl]benzoïque.

(a) 4-[4-(8-hydroxyoctyloxy)-3-(5,6,7,8-tétrahydro-5,5,8,8-tétraméthyl-2-naphtyl) phényl]benzoate de méthyle.
   De manière analogue à l'exemple 2(a), par réaction de 700 mg (1,7 mmole) de l'ester méthylique obtenu à l'exemple 1(d), avec 1,15 ml (6,7 mmoles) de 8-bromooctanol, on obtient 920 mg (100%) du produit attendu sous la forme d'une huile incolore.
   ¹H NMR (CDCl₃) δ 1,33 (s, 12H), 1,38 à 1,83 (m, 12H), 1,72 (s, 4H), 3,64 (t, 2H, *J* = 6,5 Hz), 3,93 (s, 3H), 4,02 (t, 2H, *J* = 6,5 Hz), 7,05 (d, 1H, *J* = 8,6 Hz), 7,52 à 7,67 (m, 4H), 7,69 (d, 2H, *J* = 8,3 Hz), 8,15 (d, 2H, *J* = 8,3 Hz).
(b) acide 4-[4-(8-hydroxyoctyloxy)-3- (5,6,7,8-tétrahydro-5,5,8,8-tétraméthyl-2-naphtyl)phényl]benzoïque.
   De manière analogue à l'exemple 1(e) à partir de 920 mg (1,7 mmole) de l'ester méthylique obtenu à l'exemple 5(a), on obtient 740 mg (83%) d'acide 4-[4-(8-hydroxyoctyloxy)-3-(5,6,7,8-tétrahydro-5,5,8,8-tétraméthyl-2-naphtyl)phényl]benzoïque, sous la forme de cristaux jaune clair de point de fusion 155-160°C.
   ¹H NMR (CDCl₃) δ 1,33 (s, 12H), 1,38 à 1,83 (m, 12H), 1,72 (s, 4H), 3,64 (t, 2H, *J* = 6,5 Hz), 4,03 (t, 2H, *J* = 6,5 Hz), 7,05 (d, 1H, *J* = 8,6 Hz), 7,52 à 7,67 (m, 4H), 7,69 (d, 2H, *J* = 8,3 Hz), 8,15 (d, 2H, *J* = 8,3 Hz).

### EXEMPLE 6

### Acide 4-[4-(9-hydroxynonyloxy)-3-(5,6,7,8-tétrahydro-5,5,8,8-tétraméthyl-2-naphtyl)phényl]benzoïque.

(a) 4-[4-(9-hydroxynonyloxy)-3-(5,6,7,8-tétrahydro-5,5,8,8-tétraméthyl-2-naphtyl) phényl]benzoate de méthyle.
   De manière analogue à l'exemple 2(a), par réaction de 680 mg (1,6 mmole) de l'ester méthylique obtenu à l'exemple 1(d), avec 1,47 g (6,6 mmoles) de 9-bromononanol, on obtient 920 mg (100%) du produit attendu, sous forme d'une huile brune.
   ¹H NMR (CDCl₃) δ 1,33 (s, 12H), 1,38 à 1,83 (m, 14H), 1,72 (s, 4H), 3,64 (t, 2H, *J* = 6,5 Hz), 3,93 (s, 3H), 4,02 (t, 2H, *J* = 6,5 Hz), 7,05 (d, 1H, *J* = 8,6 Hz), 7,52 à 7,67 (m, 4H), 7,69 (d, 2H, *J* = 8,3 Hz), 8,15 (d, 2H, *J* = 8,3 Hz).
(b) acide 4-[4-(9-hydroxynonyloxy)-3-(5,6,7,8-tétrahydro-5,5,8,8-tétraméthyl-2-naphtyl)phényl]benzoïque.
   De manière analogue à l'exemple 1(e) à partir de 920 mg (1,6 mmole) de l'ester méthylique obtenu à l'exemple 6(a), on obtient 720 mg (81%) d'acide 4-[4-(9-hydroxynonyloxy)-3-(5,6,7,8-tétrahydro-5,5,8,8-tétraméthyl-2-naphtyl)phényl]benzoïque, sous la forme de cristaux jaune clair de point de fusion 147-150°C.
   ¹H NMR (CDCl₃) δ 1,33 (s, 12H), 1,38 à 1,83 (m, 14H), 1,72 (s, 4H), 3,64 (t, 2H, *J* = 6,5 Hz), 4,03 (t, 2H, *J* = 6,5 Hz), 7,05 (d, 1H, *J* = 8, 6 Hz) , 7,52 à 7,67 (m, 4H), 7,69 (d, 2H, *J* = 8,3 Hz), 8,15 (d, 2H, *J* = 8,3 Hz).

### EXEMPLE 7

### Acide 4-[4-méthoxy-3-(5,6,7,8-tétrahydro-5,5,8,8-tétraméthyl-2-naphtyl) phényl]benzoïque.

(a) 4-[4-méthoxy-3-(5,6,7,8-tétrahydro-5,5,8,8-tétraméthyl-2-naphtyl)phényl] benzoate de méthyle.
   Dans un réacteur et sous courant d'azote, on introduit 1,66 g (4,0 mmoles) de l'ester méthylique obtenu à l'exemple 1(d) et 25 ml de DMF. On ajoute par petites quantités 144 mg (4,8 mmoles) d'hydrure de sodium (80% dans l'huile), et agite jusqu'à cessation du dégagement gazeux. On ajoute ensuite 311 µl (5,0 mmoles) d'iodométhane et agite pendant deux heures. On verse le milieu réactionnel dans l'eau, extrait avec de l'éther éthylique, décante la phase organique, sèche sur sulfate de magnésium, évapore. Le résidu obtenu est purifié par chromatographie sur colonne de silice élué avec du dichlorométhane. Après évaporation des solvants, on recueille 1,70 g (100%) du produit attendu sous la forme d'un solide blanc cristallisé.
   ¹H NMR (CDCl₃) δ 1,33 (s, 12H), 1,72 (s, 4H), 3,87 (s, 3H), 3,93 (s, 3H), 7,06 (d, 1H, *J* = 8,4 Hz), 7,36 (s, 2H), 7,52 à 7,61 (m, 3H), 7,66 (d, 2H, *J* = 8,3 Hz), 8,09 (d, 2H, *J* = 8,3 Hz).
(b) acide 4-[4-méthoxy-3-(5,6,7,8-tétrahydro-5,5,8,8-tétraméthyl-2-naphtyl)phényl] benzoïque.
   De manière analogue à l'exemple 1(e), à partir de 1,70 g (4,0 mmoles) de l'ester méthylique obtenu à l'exemple 7(a), on obtient 1,35 g (81%) d'acide 4-[4-méthoxy-3-(5,6,7,8-tétrahydro-5,5,8,8-tétraméthyl-2-naphtyl)phényl]benzoïque de point de fusion 251-255°C.
   ¹H NMR (CDCl₃) δ 1,33 (s, 12H), 1,72 (s, 4H), 3,88 (s, 3H), 7,08 (d, 1H, *J* = 8,4 Hz), 7,37 (s, 2H), 7,53 à 7,62 (m, 3H), 7,70 (d, 2H, *J* = 8,2 Hz), 8,17 (d, 2H, *J* = 8,2 Hz).

### EXEMPLE 8

### Acide 4-[4-méthoxyéthoxyméthoxy-3-(5, 6, 7,8-tétrahydro-5,5,8,8-tétraméthyl-2-naphtyl)phényl]benzoïque.

(a) 4-[4-méthoxyéthoxyméthoxy-3-(5,6,7,8-tétrahydro-5,5,8,8-tétraméthyl-2-naphtyl) phényl]benzoate de méthyle.
   De manière analogue à l'exemple 2(a), par réaction de 1,66 g (4,0 mmoles) de l'ester méthylique obtenu à l'exemple 1(d) avec 571 µl (5,0 mmoles) de chlorure de méthoxyéthoxyméthyle, on obtient 1,99 g (99%) du produit attendu, sous la forme d'un solide blanc cristallisé.
   ¹H NMR (CDCl₃) δ 1,32 (s, 12H), 1,72 (s, 4H), 3,37 (d, 3H, *J* = 0,5 Hz), 3,52 (t, 2H, *J* = 3,9 Hz), 3,77 (t, 2H, *J* = 3,9 Hz), 3,93 (s, 3H), 5,26 (s, 2H), 7,30 à 7,37 (m, 3H), 7,51 à 7,60 (m, 3H), 7,65 (d, 2H, *J* = 8,2 Hz), 8,09 (d, 2H, *J* = 8,1 Hz).
(b) acide 4-[4-méthoxyéthoxyméthoxy-3-(5,6,7,8-tétrahydro-5,5,8,8-tétraméthyl-2-naphtyl)phényl]benzoïque.
   De manière analogue à l'exemple 1(e), à partir de 1,99 g (4,0 mmoles) de l'ester méthylique obtenu à l'exemple 8(a), on obtient 1,62 g (84%) d'acide 4-[4-méthoxyéthoxyméthoxy-3-(5,6,7,8-tétrahydro-5,5,8,8-tétraméthyl-2-naphtyl)phényl]benzoïque, sous la forme d'un solide blanc cristallisé de point de fusion 218-219°C.
   ¹H NMR (CDCl₃) δ 1,32 (s, 12H), 1,72 (s, 4H), 3,37 (s, 3H), 3,52 (t, 2H, *J* = 3,9 Hz), 3,76 (t, 2H, *J* = 3,9 Hz), 5,26 (s, 2H), 7,30 à 7,37 (m, 3H), 7,50 à 7,61 (m, 3H), 7,65 (d, 2H, *J* = 8,3 Hz), 8,11 (d, 2H, *J* = 8,3 Hz).

### EXEMPLE 9

### Acide 4-[4-benzyloxy-3-(5,6,7,8-tétrahydro-5,5,8,8-tétraméthyl-2-naphtyl)phényl]benzoïque.

(a) 4-[4-benzyloxy-3-(5,6,7,8-tétrahydro-5,5,8,8-tétraméthyl-2-naphtyl) phényl]benzoate d'éthyle.
   De manière analogue à l'exemple 2(a), par réaction de 1,20 g (2,8 mmoles) de l'ester éthylique obtenu à l'exemple 1(d) avec 400 µl (3,2 mmoles) de bromure de benzyle, on obtient 1,23 g (85%) du produit attendu, sous la forme d'une huile incolore.
   ¹H NMR (CDCl₃) δ 1,23 (s, 6H), 1,32 (s, 6H), 1,41 (t, 3H, *J* = 7,1 Hz), 1,70 (s, 4H), 4,39 (q, 2H, *J* = 7,1 Hz), 5,38 (s, 2H), 7,11 (d, 1H, *J* = 8,5 Hz), 7,28 à 7,36 (m, 6H), 7,53 (dd, 1H, *J* = 8,5 / 2,4 Hz), 7,58 à 7,64 (m, 2H), 7,65 (d, 2H, *J* = 8,4 Hz), 8,09 (d, 2H, *J* = 8,4 Hz).
(b) acide 4-[4-benzyloxy-3-(5,6,7,8-tétrahydro-5,5,8,8-tétraméthyl-2-naphtyl)phényl]benzoïque.
   De manière analogue à l'exemple 1(e), à partir de 1,20 g (2,3 mmoles) de l'ester éthylique obtenu à l'exemple 9(a), on obtient 970 mg (86%) d'acide 4-[4-benzyloxy-3-(5,6,7,8-tétrahydro-5,5,8,8-tétraméthyl-2-naphtyl)phényl]benzoïque, sous la forme d'un solide blanc cristallisé de point de fusion 241-244°C.
   ¹H NMR (CDCl₃) δ 1,23 (s, 6H), 1,32 (s, 6H), 1,70 (s, 4H), 5,13 (s, 2H), 7,12 (d, 1H, *J* = 8,6 Hz), 7,28 à 7,36 (m, 6H), 7,54 (dd, 1H, *J* = 8,5 / 2,4 Hz), 7,58 (s, 1H), 7,64 (s, 1H), 7,66 (d, 2H, *J* = 8,4 Hz), 8,11 (d, 2H, *J* = 8,4 Hz).

### EXEMPLE 10

### Acide 4'-(2,3-dihydroxy-propoxy)-3'-(5,5,8,8-tétraméthyl-5,6,7,8-tétrahydro-naphthalèn-2-yl)-biphényl-4-carboxylique (racémique).

(a) 4-toluènesulfonate de 2,2-diméthyl-[1,3]dioxolan-4-ylméthyle (racémique).
   Dans un ballon, sous atmosphère d'argon, on introduit 5,29 g (40,0 mmoles) de (2,2-diméthyl-[1,3]dioxolan-4-yl)-méthanol (Solketal® ) et 10 ml de pyridine. On refroidit à 0°C et ajoute 8,39 g (44,0 mmoles) d'acide paratoluènesulfonique et agite seize heures à la température ambiante. On verse le milieu réactionnel sur un mélange HCl 1N / éther éthylique, extrait par de l'éther éthylique, lave à l'eau, sèche sur sulfate de magnésium, évapore. Le résidu obtenu est purifié par chromatographie sur colonne de silice, élué avec du dichlorométhane. Après évaporation des solvants, on recueille 9,70 g (85%) du produit attendu sous la forme de cristaux jaunes de point de fusion 45-47°C.
   ¹H NMR (CDCl₃) δ 1,31 (s, 3H), 1,34 (s, 3H), 2,45 (s, 3H), 3,74 à 3,80 (m, 1H), 3,93 à 4,07 (m, 3H), 4,23 à 4,32 (m, 1H), 7,35 (d, 2H, *J* = 8,1 Hz), 7,80 (d, 2H, *J* = 8,2 Hz).
(b) 4'-(2,2-diméthyl-[1,3]dioxolan-4-ylméthoxy)-3'-(5,5,8,8-tétraméthyl-5,6,7,8-tétrahydro-naphthalèn-2-yl)-biphényl-4-carboxylate d'éthyle (racémique).
   Dans un ballon, sous atmosphère d'argon, on introduit 3,00 g (7,0 mmoles) de l'ester éthylique obtenu à l'exemple 1(d), 2,41 g (8,4 mmoles) du tosylate obtenu à l'exemple 10(a), 1,10 g (7,7 mmoles) de carbonate de potassium et 35 ml de DMF. On chauffe le milieu réactionnel pendant une heure à 100°C, refroidit, verse sur un mélange eau / éther éthylique, extrait par de l'éther éthylique, lave à l'eau, sèche sur sulfate de magnésium, évapore. Le résidu obtenu est purifié par chromatographie sur colonne de silice, élué par un mélange composé de 50% de dichlorométhane et de 50% d'heptane. Après évaporation des solvants, on recueille 2,66 g (70%) du produit attendu, sous la forme d'une huile incolore.
   ¹H NMR (CDCl₃) δ 1,32 (s, 6H), 1,33 (s, 6H), 1,36 (s, 6H), 1,41 (t, 3H, *J* = 7,1 Hz), 1,72 (s, 4H), 3,83 (dd, 1H, *J* = 8,4 / 6,0 Hz), 3,94 à 4,07 (m, 2H), 4,14 (dd, 1H, *J* = 9,5 / 4,9 Hz), 4,39 (q, 2H, *J* = 7,1 Hz), 4,40 (q, 1H, *J* = 5,1 Hz), 7,06 (d, 1H, *J* = 8,5 Hz), 7,28 (dd, 1H, *J* = 8,1 / 1,8 Hz), 7,36 (d, 1H, *J* = 8,2 Hz), 7,53 (d, 1H, *J* = 1,8 Hz), 7,55 (dd, 1H, *J* = 8,5 / 2,3 Hz), 7,61 (d, 1H, *J* = 2,4 Hz), 7,65 (d, 2H, *J* = 8,4 Hz), 8,09 (d, 2H, *J* = 8,4 Hz).
(c) 4'-(2,3-dihydroxy-propoxy)-3'-(5,5,8,8-tétraméthyl-5,6,7,8-tétrahydro-naphthalèn-2-yl)-biphényl-4-carboxylate d'éthyle(racémique).
   Dans un ballon, sous atmosphère d'argon, on introduit 1,66 g (3,2 mmoles) de l'ester obtenu à l'exemple 10(b), 6,12 g (32,2 mmoles) d'acide paratoluène sulfonique, 60 ml de dichlorométhane et 5 ml de THF. On agite seize heures à la température ambiante, verse le milieu réactionnel sur un mélange eau / éther éthylique, extrait par de l'éther éthylique, lave à l'eau, sèche sur sulfate de magnésium, évapore. Le résidu obtenu est purifié par chromatographie sur un cake de silice, élué par un mélange composé de 40% d'acétate d'éthyle et 60% d'heptane. Après évaporation des solvants, on recueille 1,16 g (72%) du produit attendu, sous la forme d'une poudre blanche de point de fusion 56°C.
   ¹H NMR (CDCl₃) δ 1,33 (s, 12H), 1,41 (t, 3H, *J* = 7,1 Hz), 1,73 (s, 4H), 1,87 (t, 1H, *J* = 6,3 Hz), 2,49 (d, 1H, *J* = 4,3 Hz), 3,60 à 3,80 (m, 2H), 4,03 à 4,16 (m, 3H), 4,40 (q, 2H, *J* = 7,1 Hz), 7,07 (d, 1H, *J* = 8,4 Hz), 7,29 (d, 1H, *J* = 1,8 Hz), 7,38 (d, 1H, *J* = 8,1 Hz), 7,48 (d, 1H, *J* = 1,8 Hz), 7,54 à 7,60 (m, 2H), 7,65 (d, 2H, *J* = 8,4 Hz), 8,10 (d, 2H, *J* = 8,5 Hz).
(d) acide 4'-(2,3-dihydroxy-propoxy)-3'-(5,5,8,8-tétraméthyl-5,6,7,8-tétrahydro-naphthalèn-2-yl)-biphényl-4-carboxylique (racémique).
   De manière analogue à l'exemple 1(e), à partir de 1,16 g (2,3 mmoles) de l'ester éthylique obtenu à l'exemple 10(c), on obtient 897 mg (82%) d'acide 4'-(2,3-dihydroxy-propoxy)-3'-(5,5,8,8-tétraméthyl-5,6,7,8-tétrahydro-naphthalèn-2-yl)-biphényl-4-carboxylique, sous la forme d'un solide blanc cristallisé de point de fusion 258°C.
   ¹H NMR (CDCl₃ + 2 gouttes de DMSO D₆) δ 1,12 (s, 12H), 1,52 (s, 4H), 3,38 à 3,53 (m, 2H), 3,79 à 3,94 (m, 3H), 6,91 (d, 1H, *J* = 8,4 Hz), 7,10 à 7,17 (m, 2H), 7,34 à 7,39 (m, 3H), 7,45 (d, 2H, *J* = 8,4 Hz), 7,88 (d, 2H, *J* = 8,4 Hz).

### EXEMPLE 11

### Acide 4'-(2,2-diméthyl-[1,3]dioxolan-4-ylméthoxy)-3'-(5,5,8,8-tétraméthyl-5,6,7,8-tétrahydro-naphthalèn-2-yl)-biphényl-4-carboxylique (racémique).

De manière analogue à l'exemple 1(e) à partir de 1,00 g (1,8 mmole) de l'ester obtenu à l'exemple 10(b), on obtient 805 mg (85%) d'acide 4'-(2,2-diméthyl-[1,3]dioxolan-4-ylméthoxy)-3'-(5,5,8,8-tétraméthyl-5,6,7,8-tétrahydro-naphthalèn-2-yl)-biphényl-4-carboxylique, sous la forme d'un solide blanc cristallisé de point de fusion 206°C.
¹H NMR (CDCl₃) δ 1,33 (s, 12H), 1,36 (s, 6H), 1,73 (s, 4H), 3,84 (dd, 1H, *J* = 8,4 / 6,0 Hz), 3,96 à 4,09 (m, 2H) , 4,15 (dd, 1H, *J* = 9,5 / 4,9 Hz), 4,42 (q, 1H, *J* = 5,1 Hz), 7,08 (d, 1H, *J* = 8,6 Hz), 7,29 (dd, 1H, *J* = 8,1 / 1,7 Hz), 7,36 (d, 1H, *J* = 8,2 Hz), 7,54 (d, 1H, *J* = 1,6 Hz), 7,57 (dd, 1H, *J* = 8,5 / 2,3 Hz), 7,63 (d, 1H, *J* = 2,3 Hz), 7,69 (d, 2H, *J* = 8,4 Hz), 8,17 (d, 2H, *J* = 8,4 Hz).

### EXEMPLE 12

### Acide 4'-(2-morpholin-4-yl-éthoxy)-3'-(5,5,8,8-tétraméthyl-5,6,7,8-tétrahydro-naphthalèn-2-yl)-biphényl-4-carboxylique.

(a) 4'-(2-morpholin-4-yl-éthoxy)-3'-(5,5,8,8-tétraméthyl-5,6,7,8-tétrahydro-naphthalèn-2-yl)-biphényl-4-carboxylate d'éthyle.
   De manière analogue à l'exemple 2(a) par réaction de 1,08 g (2,5 mmoles) de l'ester éthylique obtenu à l'exemple 1(d) avec 1,39 g (7,5 mmoles) de chlorhydrate de 4-(2-chloroéthyl)morpholine, on obtient 500 mg (37%) du produit attendu, sous la forme d'une huile incolore.
   ¹H NMR (CDCl₃) δ 1,32 (s, 12H), 1,41 (t, 3H, *J* = 7,1 Hz), 1,72 (s, 4H), 2,45 (t, 2H, *J* = 4,6 Hz), 2,77 (t, 2H, *J* = 5,8 Hz), 3,64 (t, 2H, *J* = 4,7 Hz), 4,16 (t, 2H, *J* = 5,8 Hz), 4,40 (q, 2H, *J* = 7,2 Hz), 7,05 (d, 1H, *J* = 8,5 Hz), 7,34 (s, 2H), 7,52 (s, 1H), 7,56 (dd, 1H, *J* = 8,4 / 2,4 Hz), 7,61 (d, 1H, *J* = 2,3 Hz), 7,65 (d, 2H, *J* = 8,4 Hz), 8,09 (d, 2H, *J* = 8,4 Hz).
(b) acide 4'-(2-morpholin-4-yl-éthoxy)-3'-(5,5,8,8-tétraméthyl-5,6,7,8-tétrahydro-naphthalèn-2-yl)-biphényl-4-carboxyliquel.

De manière analogue à l'exemple 1(e), à partir de 500 mg (0,92 mmole) de l'ester éthylique obtenu à l'exemple 12(a), on obtient 320 mg (70%) d'acide 4'-(2-morpholin-4-yl-éthoxy)-3'-(5,5,8,8-tétraméthyl-5,6,7,8-tétrahydro-naphthalèn-2-yl)-biphényl-4-carboxylique, sous la forme d'un solide blanc cristallisé de point de fusion 270-272°C.
¹H NMR (CDCl₃ + 2 gouttes de DMSO D₆) δ 1,31 (s, 12H), 1,73 (s, 4H), 2,58 à 2,61 (m, 2H), 3,23 (d, 2H, *J* = 11,9 Hz), 3,38 (br s, 2H), 3,67 (d, 2H, *J* = 12,6 Hz), 4,02 (t, 2H, *J* = 11,9 Hz), 4,61 (br s, 2H), 7,08 (d, 1H, *J* = 8,3 Hz), 7,22 (dd, 1H, *J* = 8,1 / 1,6 Hz), 7,35 (d, 1H, *J* = 8,1 Hz), 7,47 (s, 1H), 7,55 (s, 1H), 7,61 (dd, 1H, *J* = 8,1 / 2,4 Hz), 7,64 (d, 2H, *J* = 8,4 Hz), 8,09 (d, 2H, *J* = 8,3 Hz).

### EXEMPLE 13

### 2'-(5,5,8,8-tétraméthyl-5,6,7,8-tétrahydro-naphthalèn-2-yl)-[1,1';4',1'']terphényl-4''-carboxylate de méthyle.

(a) 3'-(5,5,8,8-tétraméthyl-5,6,7,8-tétrahydro-naphthalèn-2-yl)-4'-trifluorométhanesulfonyloxy-biphényl-4-carboxylate de méthyle.
   Dans un tricol et sous courant d'azote, on introduit 1,66 g (4,0 mmoles) de l'ester méthylique obtenu à l'exemple 1(d), 1,56 g (12,8 mmoles) de 4-diméthylaminopyridine et 40 ml de dichlorométhane. On refroidit à 0°C, ajoute goutte à goutte 701 µl (4,2 mmoles) d'anhydride trifluorométhanesulfonique et agite à la température ambiante pendant une heure. On verse le milieu réactionnel dans un mélange d'acide chlorhydrique (1N) et de dichlorométhane, décante la phase organique, sèche sur sulfate de magnésium, évapore. Le résidu obtenu est purifié par chromatographie sur colonne de silice, élué avec un mélange de dichlorométhane et d'hexane (40/60). On recueille 1,90 g (87%) du produit attendu, sous la forme d'une huile incolore.
   ¹H NMR (CDCl₃) δ 1,31 (s, 6H), 1,32 (s, 6H), 1,73 (s, 4H), 3,95 (s, 3H), 7,23 (dd, 1H, *J* = 8,2 / 1,8 Hz), 7,39 à 7,48 (m, 3H), 7,60 à 7,70 (m, 2H), 7,72 (d, 2H, *J* = 8,5 Hz), 8,13 (d, 2H, *J* = 8,3 Hz).
(b) 2'-(5,5,8,8-tétraméthyl-5,6,7,8-tétrahydro-naphthalèn-2-yl)-[1,1';4',1'']terphényl-4''-carboxylate de méthyle.
   Dans un tricol, on introduit 469 mg (3,8 mmoles) d'acide phénylboronique, 1,91 g (3,5 mmoles) de 4-[4-trifluorométhanesulfonate-3-(5,6,7,8-tétrahydro-5,5,8,8-tétraméthyl-2-naphtyl)phényl]benzoate de méthyle, 4,54 ml (9,1 mmoles) d'une solution de carbonate de sodium (2M), 296 mg de chlorure de lithium et 30 ml de DMF. On dégaze le milieu réactionnel par barbotage d'azote, ajoute 129 mg (0,11 mmole) de tétrakistriphénylphosphinepalladium(0) et chauffe à 90°C pendant vingt heures. On évapore à sec le milieu réactionnel, reprend par l'eau et l'éther éthylique, acidifie. On décante la phase organique, sèche sur sulfate de magnésium, évapore. Le résidu obtenu est purifié par chromatographie sur colonne de silice, élué avec un mélange d'éther éthylique et d'heptane (5/95). On recueille 480 mg (30%) du produit attendu, sous la forme d'une huile jaune.
   ¹H NMR (CDCl₃) δ 0,90 (s, 6H), 1,26 (s, 6H), 1,55 à 1,63 (m, 4H), 3,90 (s, 3H), 6,90 (d, 1H, *J* = 1,7 Hz), 7,14 à 7,28 (m, 7H), 7,49 (d, 1H, *J* = 8,0 Hz), 7,62 (dd, 1H, *J* = 8,0 / 1,9 Hz), 7,70 à 7,73 (m, 1H), 7,71 (d, 2H, *J* = 8,4 Hz), 8,11 (d, 2H, *J* = 8,4 Hz).

### EXEMPLE 14

### Acide 2'-(5,5,8,8-tétraméthyl-5,6,7,8-tétrahydro-naphthalèn-2-yl)-[1,1';4',1'']terphényl-4''-carboxylique.

De manière analogue à l'exemple 1(e), à partir de 950 mg (2,0 mmoles) de l'ester méthylique obtenu à l'exemple 13(b), on recueille 820 mg (89%) d'acide 2'-(5,5,8,8-tétraméthyl-5,6,7,8-tétrahydro-naphthalèn-2-yl)-[1,1';4',1'']terphényl-4''-carboxylique, sous la forme d'une poudre blanche de point de fusion 287-288°C.
¹H NMR (CDCl₃) δ 0,91 (s, 6H), 1,27 (s, 6H), 1,56 à 1,64 (m, 4H), 6,89 (d, 1H, *J* = 1,8 Hz), 7,14 à 7,33 (m, 7H), 7,52 (d, 1H, *J* = 7,9 Hz), 7,67 (dd, 1H, *J* = 8, 0 / 1,9 Hz), 7,72 à 7,73 (m, 1H), 7,75 (d, 2H, *J* = 8,6 Hz), 8,15 (d, 2H, *J* = 8,4 Hz).

### EXEMPLE 15

### Acide 4-méthoxyméthoxy-2'-(5,5,8,8-tétraméthyl-5,6,7,8-tétrahydro-naphthalèn-2-yl)-[1,1';4',1"]terphényl-4"-carboxylique.

(a) 4-méthoxyméthoxy-bromobenzène.
   De manière analogue à l'exemple 7(a), par réaction de 48,84 g (282,3 mmoles) de 4-bromophénol avec 25,0 ml (310,5 mmoles) d'éther méthylique de chlorométhyle, on obtient 63,85 g (100%) du produit attendu, sous la forme d'une huile beige.
   ¹H NMR (CDCl₃) δ 3,46 (s, 3H), 5,14 (s, 2H), 6,92 (d, 2H, *J* = 9,0 Hz), 7,38 (d, 2H, *J* = 9,0 Hz).
(b) acide 4-méthoxyméthoxy-phénylboronique.
   De manière analogue à l'exemple 1(a), à partir de 63,81 g (293,0 mmoles) de 4-méthoxyméthoxy-bromobenzène, on obtient 35,42 g (80%) du produit attendu, sous la forme d'un solide blanc de point de fusion 122°C.
   ¹H NMR (CDCl₃) δ 3,52 (s, 3H), 5,27 (s, 2H), 7,15 (d, 2H, *J* = 8,6 Hz), 8,16 (d, 2H, *J* = 8,6 Hz).
(c) 4-méthoxyméthoxy-2'-(5,5,8,8-tétraméthyl-5,6,7,8-tétrahydro-naphthalèn-2-yl)-[1,1';4',1'')terphényl-4''-carboxylate d'éthyle.
   De manière analogue à l'exemple 13(b), par réaction de 357 mg (2,0 mmoles) du composé obtenu à l'exemple 15(b) avec 1,00 g (1,8 mmoles) de l'analogue (ester éthylique) du composé obtenu à l'exemple 13(a), on obtient 880 mg (13%) du produit attendu, sous la forme d'une huile incolore.
   ¹H NMR (CDCl₃) δ 0,94 (s, 6H), 1,28 (s, 6H), 1,42 (t, 3H, *J* = 7,1 Hz), 1,58 à 1,63 (m, 4H), 3,46 (s, 3H), 4,41 (q, 2H, *J* = 7,1 Hz), 5,14 (s, 2H), 6,90 (d, 1H, *J* = 1,8 Hz), 6,91 (d, 2H, *J* = 8,7 Hz), 7,08 (d, 2H, *J* = 8,7 Hz), 7,17 (dd, 1H, *J* = 8,1 / 1,9 Hz), 7,29 (d, 1H, *J* = 8,1 Hz), 7,50 (d, 1H, *J* = 7,9 Hz), 7,64 (dd, 1H, *J* = 8,0 / 2,0 Hz), 7,72 (d, 1H, *J* = 1,9 Hz), 7,74 (d, 2H, *J* = 8,4 Hz), 8,13 (d, 2H, *J* = 8,4 Hz).
(d) acide 4-méthoxyméthoxy-2'-(5,5,8,8-tétraméthyl-5,6,7,8-tétrahydro-naphthalèn-2-yl)-[1,1';4',1'']terphényl-4''-carboxylique.
   De manière analogue à l'exemple 1(e), à partir de 870 mg (1,6 mmole) de l'ester éthylique obtenu à l'exemple 15(c), on recueille 750 mg (91%) d'acide 4-méthoxyméthoxy-2'-(5,5,8,8-tétraméthyl-5,6,7,8-tétrahydro-naphthalèn-2-yl)-[1,1';4',1'']terphényl-4''-carboxylique, sous la forme d'une poudre blanche de point de fusion 249-251°C.
   ¹H NMR (CDCl₃ + 2 gouttes de DMSO D₆) δ 0,94 (s, 6H), 1,28 (s, 6H), 1,54 à 1, 66 (m, 4H), 3,45 (s, 3H), 5,14 (s, 2H), 6,89 (d, 1H, *J* = 1,9 Hz), 6,91 (d, 2H, *J* = 8,6 Hz), 7,08 (d, 2H, *J* = 8,6 Hz), 7,17 (dd, 1H, *J* = 8,1 / 1,7 Hz), 7,31 (d, 1H, *J* = 8,1 Hz), 7,28 (d, 1H, *J* = 8,1 Hz), 7,50 (d, 1H, *J* = 7,9 Hz), 7,65 (dd, H, *J* = 8,0 / 1,8 Hz), 7,71 (d, 1H, *J* = 1,9 Hz), 7,74 (d, 2H, *J* = 8,3 Hz), 8,14 (d, 2H, *J* = 8,3 Hz).

### EXEMPLE 16

### Acide 4-hydroxy-2'-(5,5,8,8-tétraméthyl-5,6,7,8-tétrahydro-naphthalèn-2-yl)-[1,1';4',1"]terphényl-4"-carboxylique.

(a) 4-hydroxy-2'-(5,5,8,8-tétraméthyl-5,6,7,8-tétrahydro-naphthalèn-2-yl)-[1,1';4',1"]terphényl-4"-carboxylate d'éthyle.
   Dans un tricol de 250 ml, et sous courant d'azote, on introduit 3,81 g (5,5 mmoles) du composé obtenu à l'exemple 15(c), 100 ml d'éthanol et 50 ml de THF. On ajoute goutte à goutte 3,5 ml d'acide sulfurique concentré. Le milieu réactionnel est chauffé pendant quinze minutes à 60°C, puis on ajoute de l'eau, extrait par de l'éther éthylique, lave la phase organique à l'eau jusqu'à pH neutre, sèche sur sulfate de magnésium, filtre et évapore les solvants. Le résidu obtenu est purifié par chromatographie sur colonne de silice, élué par de l'heptane, puis par un mélange composé de 20% d'acétate d'éthyle et 80% d'heptane. Après évaporation des solvants, on recueille 2,60 g (74%) du composé attendu, sous la forme d'une poudre blanche de point de fusion 177-179°C.
   ¹H NMR (CDCl₃) δ 0,98 (s, 6H), 1,27 (s, 6H), 1,42 (t, 3H, *J* = 7,1 Hz), 1,58 à 1,65 (m, 4H), 4,41 (q, 2H, *J* = 7,1 Hz), 4,91 (s, 1H), 6,71 (d, 2H, *J* = 8,6 Hz), 6,94 (d, 1H, *J* = 1,8 Hz), 7,03 (d, 2H, *J* = 8,6 Hz), 7,13 (dd, 1H, *J* = 8,1 / 1,9 Hz), 7,27 (d, 2H, *J* = 7,4 Hz), 7,49 (d, 1H, *J* = 8,0 Hz), 7,64 (dd, 1H, *J* = 8,0 / 1,9 Hz), 7,71 (d, 1H, *J* = 1,9 Hz), 7,73 (d, 2H, *J* = 8,5 Hz), 8,13 (d, 2H, *J* = 8,4 Hz).
(b) acide 4-hydroxy-2'-(5,5,8,8-tétraméthyl-5,6,7,8-tétrahydro-naphthalèn-2-yl)-[1,1';4',1'']terphényl-4''-carboxylique.
   De manière analogue à l'exemple 1(e) à partir de 980 mg (1,9 mmole) de l'ester éthylique obtenu à l'exemple 16(a), on recueille 790 mg (80%) d'acide 4-hydroxy-2'-(5,5,8,8-tétraméthyl-5,6,7,8-tétrahydro-naphthalèn-2-yl)-[1,1';4',1"]terphényl-4"-carboxylique, sous la forme d'une poudre blanche de point de fusion 262-266°C.
   ¹H NMR (CDCl₃) δ 0,99 (s, 6H), 1,27 (s, 6H), 1,55 à 1,67 (m, 4H), 6,72 (d, 2H, *J* = 8,5 Hz), 6,97 (d, 1H, *J* = 1,7 Hz), 6,98 (d, 2H, *J* = 8,5 Hz), 7,12 (dd, 1H, *J* = 8,0 / 1,6 Hz), 7,25 (d, 1H, *J* = 8,1 Hz), 7,49 (d, 1H, *J* = 7,9 Hz), 7,64 (dd, 1H, *J* = 8,0 / 1,6 Hz), 7,69 (d, 1H, *J* = 1,7 Hz), 7,73 (d, 2H, *J* = 8,3 Hz), 8,13 (d, 2H, *J* = 8,3 Hz).

### EXEMPLE 17

### Acide 4-méthoxy-2'-(5,5,8,8-tétraméthyl-5,6,7,8-tétrahydro-naphthalèn-2-yl)-[1,1';4',1"]terphényl-4"-carboxylique.

(a) 4-méthoxy-2'-(5,5,8,8-tétraméthyl-5,6,7,8-tétrahydro-naphthalèn-2-yl)-[1,1';4',1"]terphényl-4"-carboxylate d'éthyle.
   De manière analogue à l'exemple 7(a), par réaction de 1,40 g (2,8 mmoles) du composé obtenu à l'exemple 16(a) avec 250 µl (4,2 mmoles) d'iodure de méthyle, on obtient 1,35 g (96%) du produit attendu, sous la forme d'un solide jaune de point de fusion 112-115°C.
   ¹H NMR (CDCl₃) δ 1,27 (s, 12H), 1,42 (t, 3H, *J* = 7,1 Hz), 1,58 à 1,65 (m, 4H), 3,78 (s, 3H), 4,41 (q, 2H, *J* = 7,1 Hz), 6,78 (d, 2H, *J* = 8,7 Hz), 6,93 (d, 1H, *J* = 1,9 Hz), 7,08 (d, 2H, *J* = 8,7 Hz), 7,14 (dd, 1H, *J* = 8,1 / 1,9 Hz), 7,27 (d, 1H, *J* = 7,0 Hz), 7,50 (d, 1H, *J* = 7,9 Hz), 7,64 (dd, 1H, *J* = 8,0 / 4,0 Hz), 7,71 (d, 1H, *J* = 1,9 Hz), 7,74 (d, 2H, *J* = 8,5 Hz), 8,12 (d, 2H, *J* = 8,4 Hz).
(b) acide 4-méthoxy-2'-(5,5,8,8-tétraméthyl-5,6,7,8-tétrahydro-naphthalèn-2-yl)-[1,1';4',1"]terphényl-4"-carboxylique.
   De manière analogue à l'exemple 1(e), à partir de 1,30 g (2,7 mmoles) de l'ester éthylique obtenu à l'exemple 17(a), on recueille 960 mg (74%) d'acide 4-méthoxy-2'-(5,5,8,8-tétraméthyl-5,6,7,8-tétrahydro-naphthalèn-2-yl)-[1,1';4',1"]terphényl-4"-carboxylique, sous la forme d'une poudre blanche de point de fusion 262-266°C.
   ¹H NMR (CDCl₃) δ 0,96 (s, 6H), 1,27 (s, 6H), 1,55 à 1,67 (m, 4H), 3,78 (s, 3H), 6,78 (d, 2H, *J* = 8,7 Hz), 6,93 (d, 1H, *J* = 1,6 Hz), 7,08 (d, 2H, *J* = 8,6 Hz), 7,14 (dd, 1H, *J* = 8,2 / 1,7 Hz), 7,27 (d, 1H, *J* = 8,1 Hz), 7,50 (d, 1H, *J* = 8,0 Hz), 7,65 (dd, 1H, *J* = 8,0 / 1,8 Hz), 7,72 (d, 1H, *J* = 1,6 Hz), 7,75 (d, 2H, *J* = 8,4 Hz), 8,17 (d, 2H, *J* = 8,3 Hz).

### EXEMPLE 18

### Acide 3-méthoxyméthoxy-2'-(5,5,8,8-tétraméthyl-5,6,7,8-tétrahydro-naphthalèn-2-yl)-[1,1';4',1'']terphényl-4''-carboxylique.

(a) 3-méthoxyméthoxy-bromobenzène.
   De manière analogue à l'exemple 7(a), par réaction de 100,00 g (577,9 mmoles) de 3-bromophénol avec 48,28 g (635,8 mmoles) d'éther méthylique de chlorométhyle, on obtient 135,32 g (100%) du produit attendu, sous la forme d'une huile beige claire.
   ¹H NMR (CDCl₃) δ 3,46 (s, 3H), 5,15 (s, 2H), 6,92 à 7,00 (m, 1H), 7,10 à 7,14 (m, 2H), 7,18 à 7,22 (m, 1H).
(b) acide 3-méthoxyméthoxy-phénylboronique.
   De manière analogue à l'exemple 1(a), à partir de 125,00 g (575,8 mmoles) de 3-méthoxyméthoxy-bromobenzène, on obtient 86,00 g (100%) du produit attendu, sous la forme d'une huile jaune qui sera utilisée directement pour l'étape suivante.
(c) 3-méthoxyméthoxy-2'-(5,5,8,8-tétraméthyl-5,6,7,8-tétrahydro-naphthalèn-2-yl)-[1,1';4',1"]terphényl-4"-carboxylate d'éthyle.
   De manière analogue à l'exemple 13(b), par réaction de 3,50 g (19,3 mmoles) du composé obtenu à l'exemple 18(b) avec 9,00 g (16,1 mmoles) de l'analogue (ester éthylique) du composé obtenu à l'exemple 13(a), on obtient 7,70 g (87%) du produit attendu, sous la forme d'un solide blanc de point de fusion 103-104°C.
   ¹H NMR (CDCl₃) δ 0,96 (s, 6H), 1,27 (s, 6H), 1,42 (t, 3H, *J* = 7,1 Hz), 1,58 à 1,65 (m, 4H), 3,36 (s, 3H), 4,41 (q, 2H, *J* = 7,1 Hz), 4,89 (s, 2H), 6,76 (t, 1H, *J* = 2,1 Hz), 6,85 (dd, 1H, *J* = 8,2 / 2,4 Hz), 6,91 (m, 1H), 6,93 (d, 1H, *J* = 1,6 Hz), 7,13 à 7,30 (m, 3H), 7,54 (d, 1H, *J* = 7,9 Hz), 7,65 (dd, 1H, *J* = 8,0 / 1,9 Hz), 7,72 (s, 1H), 7,74 (d, 2H, *J* = 8,4 Hz), 8,13 (d, 2H, *J* = 8,4 Hz).
(d) acide 3-méthoxyméthoxy-2'-(5,5,8,8-tétraméthyl-5,6,7,8-tétrahydro-naphthalèn-2-yl)-[1,1';4',1"]terphényl-4"-carboxylique.
   De manière analogue à l'exemple 1(e), à partir de 1,20 g (2,2 mmoles) de l'ester éthylique obtenu à l'exemple 18(c), on recueille 1,03 g (90%) d'acide 3-méthoxyméthoxy-2'-(5,5,8,8-tétraméthyl-5,6,7,8-tétrahydro-naphthalèn-2-yl)-[1,1';4',1"]terphényl-4"-carboxylique, sous la forme d'une poudre blanche de point de fusion 212°C.
   ¹H NMR (CDCl₃) δ 0,96 (s, 6H), 1,27 (s, 6H), 1,56 à 1,66 (m, 4H), 3,37 (s, 3H), 4,90 (s, 2H), 6,77 (d, 1H, *J* = 1,9 Hz), 6,85 (dd, 1H, *J* = 8,2 / 1,9 Hz), 6,91 à 6,94 (m, 2H), 7,16 (dd, 1H, *J* = 8,1 / 1,8 Hz), 7,17 à 7,23 (m, 1H), 7,29 (d, 1H, *J* = 8,1 Hz), 7,55 (d, 1H, *J* = 8,0 Hz), 7,68 (dd, 1H, *J* = 8,0 / 1,9 Hz), 7,74 (d, 1H, *J* = 1,8 Hz), 7,78 (d, 2H, *J* = 8,4 Hz), 8,21 (d, 2H, *J* = 8,4 Hz).

### EXEMPLE 19

### Acide 3-hydroxy-2'-(5,5,8,8-tétraméthyl-5,6,7,8-tétrahydro-naphthalèn-2-yl)-[1,1';4',1'']terphényl-4''-carboxylique.

(a) 3-hydroxy-2'-(5,5,8,8-tétraméthyl-5,6,7,8-tétrahydro-naphthalèn-2-yl)-[1,1';4',1'']terphényl-4''-carboxylate d'éthyle.
   De manière analogue à l'exemple 16(a), à partir de 6,26 g (11,4 mmoles) du composé obtenu à l'exemple 18(c), on obtient 5,63 g (98%) du produit attendu, sous la forme d'un solide blanc de point de fusion inférieur à 70°C.
   ¹H NMR (CDCl₃) δ 0,95 (s, 6H), 1,27 (s, 6H), 1,42 (t, 3H, *J* = 7,1 Hz), 1,58 à 1,65 (m, 4H), 4,41 (q, 2H, *J* = 7,1 Hz), 4,95 (s, 1H), 6,63 à 6,76 (m, 3H), 6,94 (d, 1H, *J* = 1,8 Hz), 7,11 (t, 1H, *J* = 7,9 Hz), 7,15 (dd, 1H, *J* = 8,2 / 1,9 Hz), 7,28 (d, 1H, *J* = 8,1 Hz), 7,49 (d, 1H, *J* = 7,9 Hz), 7,63 (dd, 1H, *J* = 8,2 / 1,9 Hz), 7,71 à 7,73 (m, 1H), 7,73 (d, 2H, *J* = 8,4 Hz), 8,13 (d, 2H, *J* = 8,4 Hz).
(b) acide 3-hydroxy-2'-(5,5,8,8-tétraméthyl-5,6,7,8-tétrahydro-naphthalèn-2-yl)-[1,1';4',1"]terphényl-4"-carboxylique.
   De manière analogue à l'exemple 1(e), à partir de 1,05 g (2,1 mmoles) de l'ester éthylique obtenu à l'exemple 19(a), on recueille 820 mg (83%) d'acide 3-hydroxy-2'-(5,5,8,8-tétraméthyl-5,6,7,8-tétrahydro-naphthalèn-2-yl)-[1,1';4',1"]terphényl-4"-carboxylique, sous la forme d'une poudre blanche de point de fusion 260-263°C.
   ¹H NMR (CDCl₃) δ 0,96 (s, 6H), 1,27 (s, 6H), 1,57 à 1,64 (m, 4H), 6,58 (d, 1H, *J* = 7,7 Hz), 6,69 (dd, 1H, *J* = 8,0 / 2,0 Hz), 6,75 (d, 1H, *J* = 2,0 Hz), 6,98 (d, 1H, *J* = 1,7 Hz), 7,03 (t, 1H, *J* = 7,8 Hz), 7,16 (dd, 1H, *J* = 7,9 / 1,8 Hz), 7,26 (d, 1H, *J* = 8,1 Hz), 7,49 (d, 1H, *J* = 7,9 Hz), 7,63 (dd, 1H, *J* = 8,0 / 1,9 Hz), 7,71 (d, 1H, *J* = 1,9 Hz), 7,73 (d, 2H, *J* = 8,4 Hz), 8,14 (d, 2H, *J* = 8,4 Hz).

### EXEMPLE 20

### Acide 3-méthoxy-2'-(5,5,8,8-tétraméthyl-5, 6,7,8-tétrahydro-naphthalèn-2-yl)-[1,1';4',1"]terphényl-4"-carboxylique.

(a) 3-méthoxy-2'-(5,5,8,8-tétraméthyl-5,6,7,8-tétrahydro-naphthalèn-2-yl)-[1,1';4',1"]terphényl-4"-carboxylate d'éthyle.
   De manière analogue à l'exemple 7(a), par réaction de 1,20 g (2,4 mmoles) du composé obtenu à l'exemple 19(a) avec 190 µl (3,1 mmoles) d'iodure de méthyle, on obtient 1,08 g (87%) du produit attendu, sous la forme d'un solide blanc de point de fusion 116-118°C.
   ¹H NMR (CDCl₃) δ 0,96 (s, 6H), 1,27 (s, 6H), 1,42 (t, 3H, *J* = 7,1 Hz), 1,58 à 1,66 (m, 4H), 3,49 (s, 3H), 4,41 (q, 2H, *J* = 7,1 Hz), 6,53 (d, 1H, *J* = 1,3 Hz), 6,74 (dd, 1H, *J* = 7,6 / 2,5 Hz), 6,89 (d, 1H, *J* = 7,6 Hz), 6,95 (d, 1H, *J* = 1,6 Hz), 7,12 à 7,30 (m, 3H), 7,56 (d, 1H, *J* = 8,0 Hz), 7,67 (dd, 1H, *J* = 8,0 / 1,8 Hz), 7,72 à 7,74 (m, 1H), 7,74 (d, 2H, *J* = 8,3 Hz), 8,13 (d, 2H, *J* = 8,3 Hz).
(b) acide 3-méthoxy-2'-(5,5,8,8-tétraméthyl-5,6,7,8-tétrahydro-naphthalèn-2-yl)-[1,1';4',1"]terphényl-4"-carboxylique.
   De manière analogue à l'exemple 1(e), à partir de 1,07 g (2,1 mmoles) de l'ester éthylique obtenu à l'exemple 20(a), on recueille 930 mg (92%) d'acide 3-méthoxy-2'-(5,5,8,8-tétraméthyl-5,6,7,8-tétrahydro-naphthalèn-2-yl)-[1,1';4',1'']terphényl-4''-carboxylique, sous la forme d'une poudre blanche de point de fusion 258-259°C.
   ¹H NMR (CDCl₃) δ 0,96 (s, 6H), 1,27 (s, 6H), 1,58 à 1,65 (m, 4H), 3,49 (s, 3H), 6,54 (d, 1H, *J* = 1,6 Hz), 6,72 (dd, 1H, *J* = 7,6 / 2,1 Hz), 6,89 (d, 1H, *J* = 7, 6 Hz), 6,94 (d, 1H, *J* = 1,7 Hz), 7,14 (d, 1H, *J* = 7,8 Hz), 7,21 (d, 1H, *J* = 7,9 Hz), 7,28 (d, 1H, *J* = 8,1 Hz), 7,56 (d, 1H, *J* = 8,0 Hz), 7,68 (dd, 1H, *J* = 8,0 / 1,9 Hz), 7,68 à 7,70 (m, 1H), 7,74 (d, 2H, *J* = 8,3 Hz), 8,14 (d, 2H, *J* = 8,3 Hz).

### EXEMPLE 21

### Acide 2-méthoxyméthoxy-2'-(5,5,8,8-tétraméthyl-5,6,7,8-tétrahydro-naphthalèn-2-yl)-[1,1';4',1'']terphényl-4''-carboxylique.

(a) 2-méthoxyméthoxy-bromobenzène.
   De manière analogue à l'exemple 7(a), par réaction de 15,00 g (86,7 mmoles) de 2-bromophénol avec 8,40 g (104,0 mmoles) d'éther méthylique de chlorométhyle, on obtient 18,80 g (100%) du produit attendu, sous la forme d'une huile beige.
   ¹H NMR (CDCl₃) δ 3,53 (s, 3H), 5,25 (s, 2H), 6,89 (dt, 1H, *J* = 7,5 / 1,6 Hz), 7,15 (dd, 1H, *J* = 8,3 / 1,6 Hz), 7,25 (dt, 1H, *J* = 7,3 / 1,6 Hz), 7,54 (dd, 1H, *J* = 7,9 / 1,6 Hz).
(b) acide 2-méthoxyméthoxy-phénylboronique.
   De manière analogue à l'exemple 1(a), à partir de 19,00 g (8,7 mmoles) de 2-méthoxyméthoxy-bromobenzène, on obtient 11,00 g (70%) du produit attendu, sous la forme d'un solide blanc de point de fusion 63-66°C.
   ¹H NMR (CDCl₃) δ 3,51 (s, 3H), 5,31 (s, 2H), 6,21 (s, 2H), 7,07 (d, 1H, *J* = 7,3 Hz), 7,14 (d, 1H, *J* = 8,8 Hz), 7,43 (dt, 1H, *J* = 8,6 / 1,9 Hz), 7,87 (dd, 1H, *J* = 7,3 / 1,8 Hz).
(c) 2-méthoxyméthoxy-2'-(5,5,8,8-tétraméthyl-5,6,7,8-tétrahydro-naphthalèn-2-yl)-[1,1';4',1'']terphényl-4''-carboxylate d'éthyle.
   De manière analogue à l'exemple 13(b), par réaction de 2,73 g (15,0 mmoles) du composé obtenu à l'exemple 21(b) avec 7,00 g (12,5 mmoles) de l'analogue (ester éthylique) du composé obtenu à l'exemple 13(a), on obtient 1,50 g (22%) du produit attendu, sous la forme d'un solide blanc de point de fusion 132-135°C.
   ¹H NMR (CDCl₃) δ 0,91 (br s, 6H), 1,24 (s, 6H), 1,42 (t, 3H, *J* = 7,1 Hz), 1,54 à 1,63 (m, 4H), 3,10 (s, 3H), 4,41 (q, 2H, *J* = 7,1 Hz), 4,40 à 4,80 (br s, 2H), 6,96 (d, 1H, *J* = 1,8 Hz), 7,02 à 7,29 (m, 7H), 7,47 (d, 1H, *J* = 8,7 Hz), 7,65 (dd, 1H, *J* = 7,9 / 1,9 Hz), 7,74 (s, 1H), 7,76 (d, 2H, *J* = 8,4 Hz), 8,13 (d, 2H, *J* = 8,4 Hz).
(d) acide 2-méthoxyméthoxy-2'-(5,5,8,8-tétraméthyl-5,6,7,8-tétrahydro-naphthalèn-2-yl)-[1,1';4',1'']terphényl-4''-carboxylique.
   De manière analogue à l'exemple 1(e), à partir de 470 mg (0,86 mmole) de l'ester éthylique obtenu à l'exemple 21(c), on recueille 360 mg (81%) d'acide 2-méthoxyméthoxy-2'-(5,5,8,8-tétraméthyl-5,6,7,8-tétrahydro-naphthalèn-2-yl)-[1,1';4',1"]terphényl-4"-carboxylique, sous la forme d'une poudre blanche de point de fusion 218-221°C.
   ¹H NMR (CDCl₃) δ 0,91 (br s, 6H), 1,24 (s, 6H), 1,52 à 1,64 (m, 4H), 3,10 (s, 3H), 4,40 à 4,80 (br s, 2H), 6,97 (d, 1H, *J* = 1,8 Hz), 7,02 à 7,28 (m, 6H), 7,47 (d, 1H, *J* = 7,9 Hz), 7,67 (dd, 1H, *J* = 7,9 / 1,9 Hz), 7,75 (d, 1H, *J* = 1,8 Hz), 7,80 (d, 2H, *J* = 8,4 Hz), 8,21 (d, 2H, *J* = 8,3 Hz).

### EXEMPLE 22

### Acide 2-hydroxy-2'-(5,5,8,8-tétraméthyl-5,6,7,8-tétrahydro-naphthalèn-2-yl)-(1,1';4',1'']terphényl-4''-carboxylique.

(a) 2-hydroxy-2'-(5,5,8,8-tétraméthyl-5,6,7,8-tétrahydro-naphthalèn-2-yl)-[1,1';4',1'']terphényl-4''-carboxylate d'éthyle.
   De manière analogue à l'exemple 16(a), à partir de 1,10 g (2,0 mmoles) du composé obtenu à l'exemple 21(c), on obtient 1,00 g (99%) du produit attendu, sous la forme d'un solide blanc de point de fusion 161-163°C.
   ¹H NMR (CDCl₃) δ 0,93 (s, 6H), 1,25 (s, 6H), 1,43 (t, 3H, *J* = 7,2 Hz), 1,55 à 1,65 (m, 4H), 4,41 (q, 2H, *J* = 7,1 Hz), 6,81 (d, 1H, *J* = 8,1 Hz), 6,90 (t, 1H, *J* = 7,5 Hz), 6,99 (d, 1H, *J* = 1,9 Hz), 7,10 à 7,20 (m, 3H), 7,28 (d, 1H, *J* = 8,7 Hz), 7,50 (d, 1H, *J* = 7,9 Hz), 7,69 (dd, 1H, *J* = 7,9 / 1,9 Hz), 7,75 (d, 2H, *J* = 8,5 Hz), 7,78 (d, 1H, *J* = 1,9 Hz), 8,15 (d, 2H, *J* = 8,4 Hz).
(b) acide 2-hydroxy-2'-(5,5,8,8-tétraméthyl-5,6,7,8-tétrahydro-naphthalèn-2-yl)-[1,1';4',1"]terphényl-4"-carboxylique.
   De manière analogue à l'exemple 1(e), à partir de 390 mg (0,77 mmole) de l'ester éthylique obtenu à l'exemple 22(a), on recueille 315 mg (86%) d'acide 2-hydroxy-2'-(5,5,8,8-tétraméthyl-5,6,7,8-tétrahydro-naphthalèn-2-yl)-[1,1';4',1"]terphényl-4"-carboxylique, sous la forme d'une poudre blanche de point de fusion 265-269°C.
   ¹H NMR (CDCl₃ + 2 gouttes de DMSO D₆) δ 0,93 (s, 6H), 1,25 (s, 6H), 1,55 à 1,62 (m, 4H), 6,77 à 6,85 (m, 2H), 7,02 à 7,13 (m, 3H), 7,18 à 7,27 (m, 2H), 7,51 (d, 1H, *J* = 7,9 Hz), 7,66 (dd, 1H, *J* = 7,9 / 1,9 Hz), 7,73 (d, 1H, *J* = 7,9 Hz), 7,74 (d, 2H, *J* = 8,3 Hz), 8,14 (d, 2H, *J* = 8,3 Hz).

### EXEMPLE 23

### Acide 2-méthoxy-2'-(5,5,8,8-tétraméthyl-5,6,7,8-tétrahydro-naphthalèn-2-yl)-[1,1';4',1"]terphényl-4"-carboxylique.

(a) 2-méthoxy-2'-(5,5,8,8-tétraméthyl-5,6,7,8-tétrahydro-naphthalèn-2-yl)-[1,1';4',1"]terphényl-4"-carboxylate d'éthyle.
   De manière analogue à l'exemple 7(a), par réaction de 580 mg (1,2 mmole) du composé obtenu à l'exemple 22(a) avec 110 µl (1,5 mmole) d'iodure de méthyle, on obtient 530 mg (89%) du produit attendu, sous la forme d'un solide blanc de point de fusion 133-136°C.
   ¹H NMR (CDCl₃) δ 0,93 (br s, 6H), 1,24 (s, 6H), 1,42 (t, 3H, *J* = 7,1 Hz), 1,56 à 1,63 (m, 4H), 3,27 (s, 3H), 4,40 (q, 2H, *J* = 7,1 Hz), 6,71 (d, 1H, *J* = 8,0 Hz), 6,94 (d, 1H, *J* = 1,8 Hz), 6,98 (d, 1H, *J* = 7,3 Hz), 7,12 (dd, 1H, *J* = 8,1 / 1,9 Hz), 7,19 à 7,26 (m, 3H), 7,47 (d, 1H, *J* = 7,9 Hz), 7,64 (dd, 1H, *J* = 7,9 / 1,9 Hz), 7,71 (d, 1H, *J* = 1,9 Hz), 7,75 (d, 2H, *J* = 8,6 Hz), 8,12 (d, 2H, *J* = 8,4 Hz).
(b) acide 2-méthoxy-2'-(5,5,8,8-tétraméthyl-5,6,7,8-tétrahydro-naphthalèn-2-yl)-[1,1';4',1'']terphényl-4''-carboxylique.
   De manière analogue à l'exemple 1(e), à partir de 530 mg (1,0 mmole) de l'ester éthylique obtenu à l'exemple 23(a), on recueille 435 mg (87%) d'acide 2-méthoxy-2'-(5,5,8,8-tétraméthyl-5,6,7,8-tétrahydro-naphthalèn-2-yl)-[1,1';4',1'']terphényl-4"-carboxylique, sous la forme d'une poudre blanche de point de fusion 239-243°C.
   ¹H NMR (CDCl₃) δ 0,93 (s, 6H), 1,25 (s, 6H), 1,54 à 1,63 (m, 4H), 3,28 (s, 3H), 6,71 (d, 1H, *J* = 8,1 Hz), 6,95 à 7,01 (m, 2H), 7,14 (d, 1H, *J* = 8,1 Hz), 7,20 à 7,25 (m, 3H), 7,49 (d, 1H, *J* = 7,9 Hz), 7,67 (d, 1H, *J* = 7,9 Hz), 7,74 (d, 1H, *J* = 1,8 Hz), 7,79 (d, 2H, *J* = 8,2 Hz), 8,21 (d, 2H, *J* = 8,2 Hz).

### EXEMPLE 24

### Acide 2'-méthoxyméthoxy-5'-(5,5,8,8-tétraméthyl-5,6,7,8-tétrahydro-naphthalèn-2-yl)-biphényl-4-carboxylique.

(a) 4-(5,5,8,8-tétraméthyl-5,6,7,8-tétrahydro-naphthalèn-2-yl)-phénol.
   De manière analogue à l'exemple 1(d), par réaction de 53,00 g (230,3 mmoles) d'acide boronique obtenu à l'exemple 1(a) avec 23,10 g (133,6 mmoles) de 4-bromophénol, on obtient 60,00 g (70%) du composé attendu, sous la forme d'un solide blanc de point de fusion 137-140°C.
   ¹H NMR (CDCl₃) δ 1,31 (s, 6H), 1,33 (s, 6H), 1,71 (s, 4H), 4,77 (s, 1H), 6,89 (d, 2H, *J* = 8,6 Hz), 7,30 (dd, 1H, *J* = 8,2 / 1,9 Hz), 7,36 (d, 1H, *J* = 8,1 Hz), 7,45 (d, 1H, *J* = 1,9 Hz), 7,46 (d, 2H, *J* = 8,6 Hz).
(b) 2-bromo-4-(5,5,8,8-tétraméthyl-5,6,7,8-tétrahydro-naphthalèn-2-yl)-phénol.
   Dans un ballon, on introduit 39,00 g (139,0 mmoles) du composé obtenu à l'exemple 24(a) et 350 ml de dichlorométhane. On ajouté goutte à goutte 23,40 g (146,0 mmoles) de brome en solution dans 50 ml de dichlorométhane, et agite trente minutes à la température ambiante. On évapore à sec le milieu réactionnel, reprend par de l'eau et de l'acétate d'éthyle, décante la phase organique, lave à l'aide d'une solution aqueuse de métabisulfite de sodium, sèche sur sulfate de magnésium, filtre, évapore. On recueille 40,60 g (80%) du produit attendu, sous la forme d'une huile jaune.
   ¹H NMR (CDCl₃) δ 1,31 (s, 6H), 1,34 (s, 6H), 1,71 (s, 4H), 5,49 (s, 1H), 7,07 (d, 1H, *J* = 8,4 Hz), 7,27 (dd, 1H, *J* = 7,7 / 2,0 Hz), 7,36 (d, 1H, *J* = 8,2 Hz), 7,43 (dd, 1H, *J* = 7,8 / 2,0 Hz), 7,51 (d, 1H, *J* = 2,1 Hz), 7,65 (d, 1H, *J* = 2,1 Hz).
(c) 6-(3-bromo-4-méthoxyméthoxy-phényl)-1,1,4,4-tetraméthyl-1,2,3,4-tétrahydro-naphthalène.
   De manière analogue à l'exemple 7(a), par réaction de 40,60 g (113,1 mmoles) du composé obtenu à l'exemple 24(b) avec 10,65 ml (135,0 mmoles) d'éther méthylique de chlorométhyle, on obtient 45,00 g (98%) du produit attendu, sous la forme d'une huile brune.
   ¹H NMR (CDCl₃) δ 1,31 (s, 6H), 1,33 (s, 6H), 1,71 (s, 4H), 3,55 (s, 3H), 5,28 (s, 2H), 7,20 (d, 1H, *J* = 8,5 Hz), 7,27 (dd, 1H, *J* = 8,2 / 1,9 Hz), 7,36 (d, 1H, *J* = 8,2 Hz), 7,43 (dd, 1H, *J* = 7,8 / 2,0 Hz), 7,51 (d, 1H, *J* = 2,1 Hz), 7,75 (d, 1H, *J* = 2,2 Hz).
(d) acide 2-méthoxyméthoxy-5-(5,5,8,8-tétraméthyl-5,6,7,8-tétrahydro-naphthalèn-2-yl)-phénylboronique.
   De manière analogue à l'exemple 1(a), à partir de 45,00 g (112,0 mmoles) du composé obtenu à l'exemple 24(c), on obtient 41,50 g (100%) du produit attendu sous la forme d'une huile brune qui sera utilisée directement pour l'étape suivante.
(e) 2'-méthoxyméthoxy-5'-(5,5,8,8-tétraméthyl-5,6,7,8-tétrahydro-naphthalèn-2-yl)-biphényl-4-carboxylate d'éthyle.
   De manière analogue à l'exemple 1(d), par réaction de 41,20 g (112,0 mmoles) du composé obtenu à l'exemple 24(d) avec 30,90 g (112,0 mmoles) de 4-iodobenzoate d'éthyle, on obtient 18,00 g (34%) du produit attendu, sous la forme d'une huile jaune.
   ¹H NMR (CDCl₃) δ 1,31 (s, 6H), 1,33 (s, 6H), 1,41 (t, 3H, *J* = 7,1 Hz), 1,72 (s, 4H), 3,40 (s, 3H), 4,40 (q, 2H, *J* = 7,1 Hz), 5,15 (s, 2H), 7,23 à 7,39 (m, 3H), 7,44 à 7,59 (m, 3H), 7,65 (d, 2H, *J* = 8,1 Hz), 8,12 (d, 2H, *J* = 8,2 Hz).
(f) acide 2'-méthoxyméthoxy-5'-(5,5,8,8-tétraméthyl-5,6,7,8-tétrahydro-naphthalèn-2-yl)-biphényl-4-carboxylique.

De manière analogue à l'exemple 1(e), à partir de 1,00 g (2,1 mmoles) de l'ester obtenu à l'exemple 24(e), on obtient 660 mg (70%) d'acide 2'-méthoxyméthoxy-5'-(5,5,8,8-tétraméthyl-5,6,7,8-tétrahydro-naphthalèn-2-yl)-biphényl-4-carboxylique, sous la forme de cristaux beiges de point de fusion 183-185°C.
¹H NMR (CDCl₃) δ 1,32 (s, 6H), 1,34 (s, 6H), 1,72 (s, 4H), 3,42 (s, 3H), 5,18 (s, 2H), 7,28 à 7,40 (m, 3H), 7,49 à 7,56 (m, 3H), 7,70 (d, 2H, *J* = 8,2 Hz), 8,19 (d, 2H, *J* = 8,2 Hz).

### EXEMPLE 25

### Acide 2'-méthoxy-5'-(5,5,8,8-tétraméthyl-5,6,7,8-tétrahydro-naphthalèn-2-yl)-biphényl-4-carboxylique.

(a) 2'-hydroxy-5'-(5,5,8,8-tétraméthyl-5,6,7,8-tétrahydro-naphthalèn-2-yl)-biphényl-4-carboxylate d'éthyle.
   De manière analogue à l'exemple 16(a), à partir de 17,00 g (36,0 mmoles) du composé obtenu à l'exemple 24(e), on obtient 15,10 g (98%) du produit attendu, sous la forme d'un solide beige de point de fusion 148-152°C.
   ¹H NMR (CDCl₃) δ 1,31 (s, 6H), 1,33 (s, 6H), 1,42 (t, 3H, *J* = 7,1 Hz), 1,72 (s, 4H), 4,41 (q, 2H, *J* = 7,2 Hz), 5,29 (br s, 1H), 7,04 (d, 1H, *J* = 8,1 Hz), 7,31 à 7,39 (m, 2H), 7,46 à 7,52 (m, 3H), 7,63 (d, 2H, *J* = 8,3 Hz), 8,17 (d, 2H, *J* = 8,3 Hz).
(b) 2'-méthoxy-5'-(5,5,8,8-tétraméthyl-5,6,7,8-tétrahydro-naphthalèn-2-yl)-biphényl-4-carboxylate d'éthyle.
   De manière analogue à l'exemple 7(a), par réaction de 1,53 g (3,6 mmoles) du composé obtenu à l'exemple 25(a) avec 330 µl (5,4 mmoles) d'iodure de méthyle, on obtient 1,40 g (88%) du produit attendu, sous la forme d'une huile brune.
   ¹H NMR (CDCl₃) δ 1,31 (s, 6H), 1,33 (s, 6H), 1,42 (t, 3H, *J* = 7,1 Hz), 3,86 (s, 3H), 1,72 (s, 4H), 4,41 (q, 2H, *J* = 7,2 Hz), 7,04 (d, 1H, *J* = 8,1 Hz), 7,31 à 7,39 (m, 2H), 7,46 à 7,52 (m, 3H), 7,63 (d, 2H, *J* = 8,3 Hz), 8,17 (d, 2H, *J* = 8,3 Hz).
(c) acide 2'-méthoxy-5'-(5,5,8,8-tétraméthyl-5,6,7,8-tétrahydro-naphthalèn-2-yl)-biphényl-4-carboxylique.
   De manière analogue à l'exemple 1(e), à partir de 1,40 g (3,6 mmoles) de l'ester obtenu à l'exemple 25(b), on obtient 1,07 g (72%) d'acide 2'-méthoxy-5'-(5,5,8,8-tétraméthyl-5,6,7,8-tétrahydro-naphthalèn-2-yl)-biphényl-4-carboxylique, sous la forme d'un solide blanc cristallisé de point de fusion 233-235°C.
   ¹H NMR (CDCl₃) δ 1,32 (s, 6H), 1,34 (s, 6H), 1,72 (s, 4H), 3,86 (s, 3H), 7,07 (d, 1H, *J* = 8,4 Hz), 7,32 à 7,39 (m, 2H), 7,49 à 7,58 (m, 3H), 7,65 (d, 2H, *J* = 8,3 Hz), 8,11 (d, 1H, *J* = 8,3 Hz).

### EXEMPLE 26

### Acide 2'-propyloxy-5'-(5,5,8,8-tétraméthyl-5,6,7,8-tétrahydro-naphthalèn-2-yl)-biphényl-4-carboxylique.

(a) 2'-propyloxy-5'-(5,5,8,8-tétraméthyl-5,6,7,8-tétrahydro-naphthalèn-2-yl)-biphényl-4-carboxylate d'éthyle.
   De manière analogue à l'exemple 7(a), par réaction de 1,34 g (3,1 mmoles) du composé obtenu à l'exemple 25(a) avec 460 µl (4,7 mmoles) d'iodure de propyle, on obtient 1,30 g (88%) du produit attendu, sous la forme d'une huile incolore.
   ¹H NMR (CDCl₃) δ 0,98 (t, 3H, *J* = 7,3 Hz), 1,32 (s, 6H), 1,33 (s, 6H), 1,42 (t, 3H, *J* = 7,1 Hz), 1,72 à 1,77 (m, 2H), 1,72 (s, 4H), 3,98 (t, 2H, *J* = 6,4 Hz), 4,41 (q, 2H, *J* = 7,1 Hz), 7,04 (d, 1H, *J* = 9,1 Hz), 7,31 à 7,39 (m, 2H), 7,49 à 7,54 (m, 3H), 7,67 (d, 2H, *J* = 8,4 Hz), 8,09 (d, 2H, *J* = 8,4 Hz).
(b) acide 2'-propyloxy-5'-(5,5,8,8-tétraméthyl-5,6,7,8-tétrahydro-naphthalèn-2-yl)-biphényl-4-carboxylique.
   De manière analogue à l'exemple 1(e), à partir de 1,30 g (3,1 mmoles) de l'ester obtenu à l'exemple 26(a), on obtient 850 mg (61%) d'acide 2'-propyloxy-5'-(5,5,8,8-tétraméthyl-5,6,7,8-tétrahydro-naphthalèn-2-yl)-biphényl-4-carboxylique, sous la forme d'un solide blanc cristallisé de point de fusion 199-204°C.
   ¹H NMR (CDCl₃) δ 0,98 (t, 3H, *J* = 7,3 Hz), 1,71 à 1,81 (m, 2H), 1,72 (s, 4H), 3,99 (t, 2H, *J* = 6,4 Hz), 7,05 (d, 1H, *J* = 9,2 Hz), 7,35 à 7,40 (m, 2H) , 7,49 à 7,56 (m, 3H), 7,72 (d, 2H, *J* = 8,4 Hz), 8,17 (d, 2H, *J* = 8,4 Hz).

### EXEMPLE 27

### Acide 2'-hydroxy-5'-(5,5,8,8-tétraméthyl-5,6,7,8-tétrahydro-naphthalèn-2-yl)-biphényl-4-carboxylique.

De manière analogue à l'exemple 1(e), à partir de 1,00 g (2,3 mmoles) de l'ester obtenu à l'exemple 25(a), on obtient 800 mg (86%) d'acide 2'-hydroxy-5'-(5,5,8,8-tétraméthyl-5,6,7,8-tétrahydro-naphthalèn-2-yl)-biphényl-4-carboxylique, sous la forme d'un solide blanc cristallisé de point de fusion 264-267°C.
¹H NMR (CDCl₃) δ 1,31 (s, 6H), 1,33 (s, 6H), 1,71 (s, 4H), 7,07 (d, 1H, *J* = 8,3 Hz), 7,34 à 7,37 (m, 2H), 7,41 (dd, 1H, *J* = 8,4 / 2,3 Hz), 7,47 à 7,49 (m, 2H), 7,72 (d, 2H, *J* = 8,3 Hz), 8,11 (d, 2H, *J* = 8,4 Hz).

### EXEMPLE 28

### Acide 4'-(5,5,8,8-tétraméthyl-5,6,7,8-tétrahydro-naphthalèn-2-yl)-[1,1';2',1"]terphényl-4"-carboxylique.

(a) 5'-(5,5,8,8-tétraméthyl-5,6,7,8-tétrahydro-naphthalèn-2-yl)-2'-trifluorométhanesulfonyloxy-biphényl-4-carboxylate d'éthyle.
   Dans un tricol et sous courant d'azote, on introduit 2,00 g (4,7 mmoles) de l'ester éthylique obtenu à l'exemple 25(a), 1,30 g (4,9 mmoles) de triflate de 4-nitrophénol, 1,30 g (9,3 mmoles) de carbonate de potassium et 30 ml de N,N-diméthylformamide. On agite à la température ambiante pendant seize heures, verse le milieu réactionnel dans un mélange eau / éther éthylique, lave à l'eau, sèche sur sulfate de magnésium, évapore. On recueille 2,60 g (100%) du produit attendu, sous la forme de cristaux jaunes de point de fusion 110-113°C.
   ¹H NMR (CDCl₃) δ 1,32 (s, 6H), 1,34 (s, 6H), 1,43 (t, 3H, *J* = 7,1 Hz), 1,73 (s, 4H), 4,42 (t, 2H, *J* = 7,2 Hz), 7,34 (dd, 1H, *J* = 8,2 / 1,9 Hz), 7,39 à 7,50 (m, 3H), 7,59 (d, 2H, *J* = 8,4 Hz), 7,60 à 7,65 (m, 2H), 8,17 (d, 2H, *J* = 8,4 Hz).
(b) 4'-(5,5,8,8-tétraméthyl-5,6,7,8-tétrahydro-naphthalèn-2-yl)-[1,1';2',1'']terphényl-4''-carboxylate d'éthyle.
   De manière analogue à l'exemple 13(b), par réaction de 2,60 g (4,6 mmoles) du composé obtenu à l'exemple 28(a) avec 626 mg (5,1 mmoles) d'acide phénylboronique, on obtient 1,10 g (47%) du produit attendu, sous la forme de cristaux jaunes de point de fusion 233-235°C.
   ¹H NMR (CDCl₃) δ 1,21 (t, 3H, *J* = 7,1 Hz), 1,32 (s, 6H), 1,35 (s, 6H), 1,68 (s, 4H), 4,29 (q, 2H, *J* = 7,0 Hz), 7,25 à 7,32 (m, 5H), 7,44 (d, 2H, *J* = 8,2 Hz), 7,54 à 7,70 (m, 3H), 7,89 à 7,91 (m, 3H), 8,06 (d, 2H, *J* = 8,2 Hz).
(c) acide 4'-(5,5,8,8-tétraméthyl-5,6,7,8-tétrahydro-naphthalèn-2-yl)-[1,1';2',1'']terphényl-4''-carboxylique.
   De manière analogue à l'exemple 1(e), à partir de 1,00 g (2,0 mmoles) de l'ester obtenu à l'exemple 28(b), on obtient 700 mg (77%) d'acide 4'-(5,5,8,8-tétraméthyl-5,6,7,8-tétrahydro-naphthalèn-2-yl)-[1,1';2',1'']terphényl-4''-carboxylique, sous la forme de cristaux beiges de point de fusion 258-262°C.
   ¹H NMR (DMSO D₆) δ 1,28 (s, 6H), 1,32 (s, 6H), 1,68 (s, 4H), 7,11 à 7,15 (m, 2H), 7,25 à 7,28 (m, 3H), 7,32 (d, 2H, *J* = 8,2 Hz), 7,43 (d, 1H, *J* = 8,2 Hz), 7,46 (d, 1H, *J* = 1,8 Hz), 7,51 (d, 1H, *J* = 8,0 Hz), 7,62 à 7,64 (m, 2H), 7,74 (dd, 1H, *J* = 8,0 / 1,8 Hz), 7,81 (d, 2H, *J* = 8,3 Hz).

### EXEMPLE 29

### Acide 2'-méthoxyméthoxy-3'-(5,5,8,8-tétraméthyl-5,6,7,8-tétrahydro-naphthalèn-2-yl)-biphényl-4-carboxylique.

(a) 1,3-dibromo-2-méthoxyméthoxy-benzène.
   De manière analogue à l'exemple 7(a), par réaction de 19,16 g (76,1 mmoles) de 2,6-dibromophénol avec 7,35 g (91,3 mmoles) d'éther méthylique de chlorométhyle, on obtient 22,50 g (100%) du produit attendu, sous la forme d'une huile incolore.
   ¹H NMR (CDCl₃) δ 3,73 (s, 3H), 5,18 (s, 2H), 6,88 (t, 1H, *J* = 8,1 Hz), 7,52 (d, 2H, *J* = 8,0 Hz).
(b) 6-(3-bromo-2-méthoxyméthoxy-phényl)-1,1,4,4-tétraméthyl-1,2,3,4-tétrahydro-naphthalène.
   De manière analogue à l'exemple 1(d), par réaction de 21,72 g (73,4 mmoles) du composé obtenu à l'exemple 29(a) avec 18,74 g (80,7 mmoles) d'acide boronique obtenu à l'exemple 1(a), on obtient 4,04 g (14%) du produit attendu, sous la forme d'un solide blanc de point de fusion 74°C.
   ¹H NMR (CDCl₃) δ 1,30 (s, 12H), 1,71 (s, 4H), 3,11 (s, 3H), 4,73 (s, 2H), 7,04 (t, 1H, *J* = 7,8 Hz), 7,23 (dd, 1H, *J* = 8,1 / 1,8 Hz), 7,28 (dd, 1H, *J* = 7,9 / 1,6 Hz), 7,34 (d, 1H, *J* = 8,1 Hz), 7,45 (d, 1H, *J* = 1,8 Hz), 7,53 (dd, 1H, *J* = 7,9 / 1,6 Hz).
(c) acide 2-méthoxyméthoxy-3-(5,5,8,8-tétraméthyl-5,6,7,8-tétrahydro-naphthalèn-2-yl)-phénylboronique.
   De manière analogue à l'exemple 1(a), à partir de 4,04 g (10,0 mmoles) du composé obtenu à l'exemple 29(b), on obtient 3,82 g (100%) du produit attendu, sous la forme d'une huile incolore.
   ¹H NMR (CDCl₃) δ 1,30 (s, 6H), 1,32 (s, 6H), 1,72 (s, 4H), 3,26 (s, 3H), 4,58 (s, 2H), 6,13 (s, 2H), 7,21 à 7,27 (m, 3H), 7,31 à 7,40 (m, 1H), 7,44 à 7,52 (m, 1H), 7,80 (dd, 1H, *J* = 7,3 / 1,8 Hz).
(d) 2'-méthoxyméthoxy-3'-(5,5,8,8-tétraméthyl-5,6,7,8-tétrahydro-naphthalèn-2-yl)-biphényl-4-carboxylate d'éthyle.
   De manière analogue à l'exemple 1(d), par réaction de 3,82 g (10,4 mmoles) du composé obtenu à l'exemple 29(c) avec 2,20 g (8,0 mmoles) de 4-iodobenzoate d'éthyle, on obtient 3,28 g (87%) du produit attendu, sous la forme d'un solide blanc cristallisé de point de fusion 75°C.
   ¹H NMR (CDCl₃) δ 1,32 (s, 6H), 1,33 (s, 6H), 1,42 (t, 3H, *J* = 7,1 Hz), 1,72 (s, 4H), 2,60 (s, 3H), 4,33 (s, 2H), 4,41 (q, 2H, *J* = 7,2 Hz), 7,29 à 7,53 (m, 6H), 7,70 (d, 2H, *J* = 8,4 Hz), 8,11 (d, 2H, *J* = 8,4 Hz).
(e) acide 2'-méthoxyméthoxy-3'-(5,5,8,8-tétraméthyl-5,6,7,8-tétrahydro-naphthalèn-2-yl)-biphényl-4-carboxylique.
   De manière analogue à l'exemple 1(e), à partir de 1,00 g (2,1 mmoles) de l'ester obtenu à l'exemple 29(d), on obtient 500 mg (53%) d'acide 2'-méthoxyméthoxy-3'-(5,5,8,8-tétraméthyl-5,6,7,8-tétrahydro-naphthalèn-2-yl)-biphényl-4-carboxylique, sous la forme d'un solide blanc cristallisé de point de fusion 176°C.
   ¹H NMR (CDCl₃) δ 1,31 (s, 6H), 1,33 (s, 6H), 1,72 (s, 4H), 2,61 (s, 3H), 4,35 (s, 2H), 7,24 à 7,35 (m, 4H), 7,41 (dd, 1H, *J* = 7,3 / 2,3 Hz), 7,54 (s, 1H), 7,75 (d, 2H, *J* = 8,4 Hz), 8,19 (d, 2H, *J* = 8,4 Hz).

### EXEMPLE 30

### Acide 2'-hydroxy-3'-(5,5,8,8-tétraméthyl-5,6,7,8-tétrahydro-naphthalèn-2-yl)-biphényl-4-carboxylique.

(a) 2'-hydroxy-3'-(5,5,8,8-tétraméthyl-5,6,7,8-tétrahydro-naphthalèn-2-yl) -biphényl-4-carboxylate d'éthyle.
   De manière analogue à l'exemple 16(a), à partir de 2,28 g (4,82 mmoles) du composé obtenu à l'exemple 29(d), on obtient 1,59 g (77%) du produit attendu sous la forme d'un solide blanc de point de fusion 121°C.
   ¹H NMR (CDCl₃) δ 1,31 (s, 6H), 1,33 (s, 6H), 1,41 (t, 3H, *J* = 7,1 Hz), 1,73 (s, 4H), 4,40 (q, 2H, *J* = 7,1 Hz), 5,53 (s, 1H), 7,07 (t, 1H, *J* = 7,6 Hz), 7,24 à 7,32 (m, 3H), 7,42 à 7,45 (m, 2H), 7,69 (d, 2H, *J* = 8,4 Hz), 8,12 (d, 2H, *J* = 8,4 Hz).
(b) acide 2'-hydroxy-3'-(5,5,8,8-tétraméthyl-5,6,7,8-tétrahydro-naphthalèn-2-yl)-biphényl-4-carboxylique.
   De manière analogue à l'exemple 1(e), à partir de 700 mg (1,6 mmole) de l'ester obtenu à l'exemple 30(a), on obtient 526 mg (81%) d'acide 2'-hydroxy-3'-(5,5,8,8-tétraméthyl-5,6,7,8-tétrahydro-naphthalèn-2-yl)-biphényl-4-carboxylique, sous la forme d'un solide blanc cristallisé de point de fusion 232°C.
   ¹H NMR (CDCl₃) δ 1,31 (s, 6H), 1,33 (s, 6H), 1,73 (s, 4H), 5,58 (br s, 1H), 7,08 (t, 1H, *J* = 7,6 Hz), 7,24 à 7,35 (m, 3H), 7,43 à 7,46 (m, 2H), 7,74 (d, 2H, *J* = 8,4 Hz), 8,20 (d, 2H, *J* = 8,3 Hz).

### EXEMPLE 31

### Acide 2'-méthoxy-3'-(5,5,8,8-tétraméthyl-5,6,7,8-tétrahydro-naphthalèn-2-yl)-biphényl-4-carboxylique.

(a) 2'-méthoxy-3'-(5,5,8,8-tétraméthyl-5,6,7,8-tétrahydro-naphthalèn-2-yl)-biphényl-4-carboxylate d'éthyle.
   De manière analogue à l'exemple 7(a), par réaction de 890 mg (2,1 mmoles) du composé obtenu à l'exemple 30(a) avec 190 µl (3,1 mmoles) d'iodure de méthyle, on obtient 800 mg (87%) du produit attendu, sous la forme d'une huile incolore.
   ¹H NMR (CDCl₃) δ 1,32 (s, 12H), 1,42 (t, 3H, *J* = 7,1 Hz), 1,72 (s, 4H), 3,20 (s, 3H), 4,41 (q, 2H, *J* = 7,1 Hz), 7,23 à 7,35 (m, 4H), 7,39 (dd, 1H, *J* = 7,3 / 2,1 Hz), 7,56 (d, 1H, *J* = 1,4 Hz), 7,69 (d, 2H, *J* = 8,3 Hz), 8,11 (d, 2H, *J* = 8,3 Hz).
(b) acide 2'-méthoxy-3'-(5,5,8,8-tétraméthyl-5,6,7,8-tétrahydro-naphthalèn-2-yl)-biphényl-4-carboxylique.
   De manière analogue à l'exemple 1(e), à partir de 800 mg (1,8 mmole) de l'ester obtenu à l'exemple 31(a), on obtient 502 mg (67%) d'acide 2'-méthoxy-3'-(5,5,8,8-tétraméthyl-5,6,7,8-tétrahydro-naphthalèn-2-yl)-biphényl-4-carboxylique, sous la forme d'un solide blanc cristallisé de point de fusion 205°C.
   ¹H NMR (CDCl₃) δ 1,33 (s, 12H), 1,73 (s, 4H), 7,25 à 7,36 (m, 4H), 7,41 (dd, 1H, *J* = 7,4 / 2,0 Hz), 7,57 (d, 1H, *J* = 1,2 Hz), 7,74 (d, 2H, *J* = 8,4 Hz), 8,20 (d, 2H, *J* = 8,4 Hz).

### EXEMPLE 32

### Acide 3'-méthoxyméthoxyméthyl-5'-(5,5,8,8-tétraméthyl-5, 6, 7,8-tétrahydro-naphthalèn-2-yl)-biphényl-4-carboxylique.

(a) 3-bromo-5- (5,5,8,8-tétraméthyl-5,6,7,8-tétrahydro-naphthalèn-2-yl)-benzoate de méthyle.
   De manière analogue à l'exemple 1(d), par réaction de 7,35 g (21, 6 mmoles) de 3-bromo-5-iodo benzoate de méthyle avec 7,44 g (32,4 mmoles) de l'acide boronique obtenu à l'exemple 1(a), on obtient 5,12 g (59%) du produit attendu, sous la forme d'une poudre blanche de point de fusion 88°C.
   ¹H NMR (CDCl₃) δ 1,32 (s, 6H), 1,35 (s, 6H), 1,72 (s, 4H), 3,95 (s, 3H), 7,34 (dd, 1H, *J* = 8,2 / 1,9 Hz), 7,40 (d, 1H, *J* = 8,2 Hz), 7,48 (d, 1H, *J* = 1,7 Hz), 7,87 (t, 1H, *J* = 1,8 Hz), 8,11 (t, 1H, *J* = 1,6 Hz), 8,16 (t, 1H, *J* = 1,5 Hz).
(b) acide 3-bromo-5-(5,5,8,8-tétraméthyl-5,6,7,8-tétrahydro-naphthalèn-2-yl) -benzoïque.
   De manière analogue à l'exemple 1(e), à partir de 4,92 g (12,3 mmoles) de l'ester obtenu à l'exemple 32(a), on obtient 3,26 g (70%) du produit attendu, sous la forme d'une poudre blanche de point de fusion 165°C.
   ¹H NMR (CDCl₃) δ 1,33 (s, 6H), 1,36 (s, 6H), 1,73 (s, 4H), 7,35 (dd, 1H, *J* = 8,2 / 1,8 Hz), 7,39 (d, 1H, *J* = 8,2 Hz), 7,50 (d, 1H, *J* = 1,7 Hz), 7,94 (t, 1H, *J* = 1,7 Hz), 8,20 (t, 1H, *J* = 1,6 Hz), 8,24 (t, 1H, *J* = 1,5 Hz).
(c) alcool 3-bromo-5-(5,5,8,8-tétraméthyl-5,6,7,8-tétrahydro-naphthalèn-2-yl)-benzylique.
   Dans un ballon, on introduit 2,76 g (0,87 mmole) du composé obtenu à l'exemple 32(b) .et 60 ml de THF. On refroidit la solution obtenue à 0°C et ajoute goutte à goutte 13,75 ml (13,7 mmoles) d'une solution de borane (1M) dans le THF et agite seize heures à la température ambiante, puis à 50°C pendant deux heures. On ajoute lentement du méthanol, reprend par de l'eau et de l'éther éthylique, décante la phase organique, extrait à l'éther éthylique, sèche sur sulfate de magnésium, filtre, évapore. On recueille 2,92 g (100%) du produit attendu, sous la forme d'une huile incolore.
   ¹H NMR (CDCl₃) δ 1,30 (s, 6H), 1,33 (s, 6H), 1,71 (s, 4H), 2,60 (br s, 1H), 4,68 (s, 2H), 7,28 (dd, 1H, *J* = 8,2 / 1,9 Hz), 7,36 (d, 1H, *J* = 8,2 Hz), 7,45 à 7,47 (m, 3H), 7,60 (s, 1H).
(d) 3-méthoxyméthoxyméthyl-5-(5,5,8,8-tétraméthyl-5,6,7,8-tétrahydro-naphthalèn-2-yl)-bromobenzène.
   De manière analogue à l'exemple 7(a), par réaction de 2,92 g (7,8 mmoles) du bromoalcool obtenu à l'exemple 32(c) avec 650 µl (8,6 mmoles) d'éther méthylique de chlorométhyle, on obtient 2,52 g (77%) du produit attendu, sous la forme d'une huile jaune.
   ¹H NMR (CDCl₃) δ 1,31 (s, 6H), 1,34 (s, 6H), 1,72 (s, 4H), 3,43 (s, 3H), 4,62 (s, 2H), 4,73 (s, 2H), 7,31 (dd, 1H, *J* = 8,2 / 1,9 Hz), 7,38 (d, 1H, *J* = 8,2 Hz), 7,43 à 7,48 (m, 3H), 7,62 (m, 1H, *J* = 1,9 Hz).
(e) acide 3-méthoxyméthoxyméthyl-5-(5,5,8,8-tétraméthyl-5,6,7,8-tétrahydro-naphthalèn-2-yl)-phénylboronique.
   De manière analogue à l'exemple 1(a), à partir de 2,52 g (6,0 mmoles) du composé obtenu à l'exemple 32(d), on obtient 2,60 g (100%) du produit attendu, sous la forme d'une huile jaune qui sera utilisée telle quelle pour l'étape suivante.
(f) 3'-méthoxyméthoxyméthyl-5'-(5,5,8,8-tétraméthyl-5,6,7,8-tétrahydro-naphthalèn-2-yl)-biphényl-4-carboxylate d'éthyle.
   De manière analogue à l'exemple 1(d), par réaction de 2,60 g (6,8 mmoles) du composé obtenu à l'exemple 32(e) avec 2,81 g (6,2 mmoles) de 4-iodobenzoate d'éthyle, on obtient 1,48 g (45%) du produit attendu, sous la forme d'une huile jaune.
   ¹H NMR (CDCl₃) δ 1,33 (s, 6H), 1,35 (s, 6H), 1,42 (t, 3H, *J* = 7,1 Hz), 1,73 (s, 4H), 3,46 (s, 3H), 4,41 (q, 2H, *J* = 7,1 Hz), 4,73 (s, 2H), 4,78 (s, 2H), 7,40 (d, 1H, *J* = 0,8 Hz), 7,55 à 7,58 (m, 3H), 7,71 (d, 2H, *J* = 8,4 Hz), 7,73 (s, 1H), 8,14 (d, 2H, *J* = 8,4 Hz).
(g) acide 3'-méthoxyméthoxyméthyl-5'-(5,5,8,8-tétraméthyl-5,6,7,8-tétrahydro-naphthalèn-2-yl)-biphényl-4-carboxylique.
   De manière analogue à l'exemple 1(e), à partir de 600 mg (1,2 mmole) de l'ester obtenu à l'exemple 32(f), on obtient 560 mg (99%) d'acide 3'-méthoxyméthoxyméthyl-5'-(5,5,8,8-tétraméthyl-5,6,7,8-tétrahydro-naphthalèn-2-yl)-biphényl-4-carboxylique, sous la forme d'un solide blanc cristallisé de point de fusion 165°C.
   ¹H NMR (CDCl₃) δ 1,33 (s, 6H), 1,36 (s, 6H), 1,74 (s, 4H), 3,47 (s, 3H), 4,75 (s, 2H), 4,79 (s, 2H), 7,41 (s, 2H), 7,56 à 7,60 (m, 3H), 7,74 à 7,78 (m, 3H), 8,22 (d, 2H, *J* = 8,3 Hz).

### EXEMPLE 33

### Acide 3'-hydroxyméthyl-5'-(5,5,8,8-tétraméthyl-5,6,7,8-tétrahydro-naphthalèn-2-yl)-biphényl-4-carboxylique.

(a) 3'-hydroxyméthyl-5'-(5,5,8,8-tétraméthyl-5,6,7,8-tétrahydro-naphthalèn-2-yl)-biphényl-4-carboxylate d'éthyle.
   De manière analogue à l'exemple 16(a), à partir de 670 mg (1,4 mmole) du composé obtenu à l'exemple 32(f), on obtient 380 mg (62%) du produit attendu, sous la forme d'une huile jaune.
   ¹H NMR (CDCl₃ + 2 gouttes de DMSO D₆) δ 1,33 (s, 6H), 1,36 (s, 6H), 1,73 (s, 4H), 4,79 (s, 2H), 7,40 à 7,43 (m, 3H), 7,56 (s, 1H), 7,61 (d, 1H, *J* = 7,4 Hz), 7,69 (s, 1H), 7,73 (d, 2H, *J* = 8,4 Hz), 8,13 (d, 2H, *J* = 8,3 Hz).
(b) acide 3'-hydroxyméthyl-5'-(5,5,8,8-tétraméthyl-5,6,7,8-tétrahydro-naphthalèn-2-yl)-biphényl-4-carboxylique.
   De manière analogue à l'exemple 1(e), à partir de 380 mg (0,86 mmole) de l'ester obtenu à l'exemple 33(a), on obtient 260 mg (73%) d'acide 3'-hydroxyméthyl-5'-(5,5,8,8-tétraméthyl-5,6,7,8-tétrahydro-naphthalèn-2-yl)-biphényl-4-carboxylique, sous la forme d'un solide blanc cristallisé de point de fusion 213°C.
   ¹H NMR (DMSO D₆) δ 1,33 (s, 6H), 1,36 (s, 6H), 1,73 (s, 4H), 4,79 (s, 2H), 7,40 à 7,43 (m, 3H), 7,56 (s, 1H), 7,61 (d, 2H, *J* = 7,3 Hz), 7,69 (s, 1H), 7,73 (d, 2H, *J* = 8,4 Hz), 8,13 (d, 2H, *J* = 8,3 Hz).

### EXEMPLE 34

### Acide 2'-(4,4-diméthyl-thiochroman-7-yl)-[1,1';4',1''] ter-phényl-4''-carboxylique.

(a) 3'-méthoxyméthoxy-biphényl-4-carboxylate d'éthyle.
   De manière analogue à l'exemple 1(d), par réaction de 85,00 g (566,7 mmoles) du composé obtenu à l'exemple 18(b) avec 104,30 g (377,8 mmoles) de 4-iodobenzoate d'éthyle, on obtient 162,4 g (100%) du produit attendu, sous la forme d'une huile marron.
   ¹H NMR (CDCl₃) δ 1,42 (t, 3H, *J* = 7,2 Hz), 3,51 (s, 3H), 4,40 (q, 2H, *J* = 7,1 Hz), 5,24 (s, 2H), 7,08 (dt, 1H, *J* = 8,1 / 1,0 Hz), 7,25 à 7,42 (m, 3H), 7,65 (d, 2H, *J* = 8,5 Hz), 8,10 (d, 2H, *J* = 8,5 Hz).
(b) 3'-hydroxy-biphényl-4-carboxylate d'éthyle.
   De manière analogue à l'exemple 16(a), à partir de 162,00 g (566,7 mmoles) du composé obtenu à l'exemple 34(a), on obtient 133,41 g (97%) du produit attendu, sous la forme d'une poudre beige de point de fusion 76°C.
   ¹H NMR (CDCl₃) δ 1,26 (s, 12H), 1,41 (t, 3H, *J* = 7,1 Hz), 4,39 (q, 2H, *J* = 7,1 Hz), 6,88 à 6,91 (m, 1H), 7,09 à 7,13 (m, 2H), 7,25 à 7,33 (m, 1H), 7,64 (m, 2H, *J* = 8,3 Hz), 8,08 (d, 2H, *J* = 8,3 Hz), 8,77 (br s, 1H).
(c) acide 3'-hydroxy-biphényl-4-carboxylique.
   De manière analogue à l'exemple 1(e), à partir de 130,00 g (536,6 mmoles) de l'ester obtenu à l'exemple 34(b), on obtient 40,00 g (35%) du produit attendu, sous la forme d'une poudre beige claire de point de fusion 180°C.
   ¹H NMR (DMSO D₆) δ 6,50 (dd, 1H, *J* = 7,2 / 1,5 Hz), 6,71 à 6,74 (m, 2H), 7,22 à 7,31 (m, 1H), 7,30 (d, 2H, *J* = 8,3 Hz), 7,71 (d, 2H, *J* = 8,3 Hz).
(d) acide 4'-iodo-3'-hydroxy-biphényl-4-carboxylique.
   Dans un tricol de deux litres, et sous courant d'azote, on introduit 40,00 g (186,7 mmoles) du composé obtenu à l'exemple 34(c), 7,47 g (186,7 mmoles) de soude en pastilles, 27,98 g (186,7 mmoles) d'iodure de sodium et 800 ml de méthanol. On refroidit à 0°C et ajoute goutte à goutte, en une heure et cinquante minutes, 111,00 g (186,7 mmoles) d'une solution aqueuse d'hypochlorite de sodium à 12,5%. Le milieu réactionnel est agité pendant cinq heures à 0°C, puis on ajoute une solution de thiosulfate de sodium, acidifie à pH 5, extrait avec de l'éther éthylique, lave la phase organique à l'eau jusqu'à pH neutre, sèche sur sulfate de magnésium, filtre et évapore les solvants. On recueille 54,00 g (85%) du composé attendu, sous la forme d'une poudre de couleur rouille, de point de fusion 174°C.
   ¹H NMR (DMSO D₆) δ 6,83 à 6,89 (m, 1H), 7,11 à 7,24 (m, 1H), 7,38 à 7,41 (m, 1H), 7,60 à 7,76 (m, 2H), 8,06 à 8,17 (m, 2H).
(e) 4'-iodo-3'-hydroxy-biphényl-4-carboxylate de méthyle.
   De manière analogue à l'exemple 1(b), à partir de 54,00 g (158,8 mmoles) du composé obtenu à l'exemple 34(d), on obtient 27,16 g (48%) du produit attendu, sous la forme d'une poudre beige clair de point de fusion 192°C.
   ¹H NMR (DMSO D₆) δ 3,44 (s, 3H), 6,37 (dd, 1H, *J* = 8,1 / 2,1 Hz), 6,70 (d, 1H, *J* = 2,0 Hz), 7,13 (d, 2H, *J* = 8,5 Hz), 7,26 (d, 1H, *J* = 8,1 Hz), 7,58 (d, 2H, *J* = 8,4 Hz), 9,45 (br s, 1H).
(f) 2'-hydroxy-[1,1';4',1"]terphényl-4"-carboxylate de méthyle.
   De manière analogue à l'exemple 1(d), par réaction de 27,16 g (76,6 mmoles) du composé obtenu à l'exemple 34(e) avec 14,03 g (115,0 mmoles) d'acide phénylboronique, on obtient 2,90 g (12%) du produit attendu, sous la forme d'une huile jaune.
   ¹H NMR (DMSO D₆) δ 3,88 (s, 3H), 7,25 à 7,45 (m, 6H), 7,61 (d, 2H, *J* = 7,1 Hz), 7,79 (d, 2H, *J* = 8,3 Hz), 8,06 (d, 2H, *J* = 8,3 Hz), 9,85 (br s, 1H).
(g) 2'-trifluorométhanesulfonyloxy-[1,1';4',1"]terphényl-4"-carboxylate de méthyle.
   De manière analogue à l'exemple 13(a), à partir de 2,90 g (9,5 mmoles) du composé obtenu à l'exemple 34(d), on obtient 3,62 g (87%) du produit attendu, sous la forme d'un poudre beige de point de fusion 95°C.
   ¹H NMR (CDCl₃) δ 3,96 (s, 3H), 7,41 à 7,72 (m, 10H), 8,16 (d, 2H, *J* = 8,5 Hz).
(h) 1-bromo-3-(3-méthyl-but-2-ènylsulfanyl)-benzène.
   Dans un tricol, on introduit 25,00 g (132,0 mmoles) de 3-bromothiophénol, 200 ml de DMF et 18,23 g (138,0 mmoles) de carbonate de potassium. On ajoute goutte à goutte 18,0 ml (157,0 mmoles) de 1-bromo-3-méthyl-2-butène et agite à la température ambiante pendant cinq heures. On verse le milieu réactionnel dans l'eau, extrait avec de l'acétate d'éthyle, décante la phase organique, lave à l'eau, sèche sur sulfate de magnésium, évapore. On recueille 33,00 g (97%) du composé attendu, sous la forme d'une huile jaune.
   ¹H NMR (CDCl₃) δ 1,62 (s, 3H), 1,73 (s, 3H), 3,54 (d, 2H, *J* = 7,7 Hz), 5,28 (t, 1H, *J* = 7,7 Hz), 7,09 à 7,15 (m, 1H), 7,22 à 7,31 (m, 2H), 7,45 (s, 1H).
(i) 7-bromo-4,4-diméthyl-thiochromane.
   Dans un tricol, on introduit 25,00 g (97,0 mmoles) de 1-bromo-3- (3-méthyl-but-2-ènylsulfanyl) -benzène, 200 ml de toluène et 27,75 g (146,0 mmoles) d'acide para-toluène sulfonique. On chauffe à reflux pendant quatre heures et évapore le milieu réactionnel à sec. On reprend par une solution aqueuse d'hydrogénocarbonate de sodium, extrait avec de l'acétate d'éthyle, décante la phase organique, sèche sur sulfate de magnésium, évapore. Le résidu obtenu est purifié par chromatographie sur colonne de silice, élué avec de l'heptane. On recueille 22,57 g (90%) du composé attendu, sous la forme d'une huile jaune.
   ¹H NMR (CDCl₃) δ 1,23 (s, 6H), 1,84 à 1,89 (m, 2H), 2,92 à 2,97 (m, 2H), 7,03 (dd, 1H, *J* = 8,5 / 2,0 Hz), 7,13 (d, 1H, *J* = 8,5 Hz), 7,15 (d, 1H, *J* = 2,0 Hz).
(j) acide 7-bromo-4,4-diméthyl-thiochromane boronique.
   De manière analogue à l'exemple 1(a), à partir de 5,00 g (20,4 mmoles) du composé obtenu à l'exemple 34(i), on obtient 2,63 g (61%) du produit attendu, sous la forme d'un solide beige clair de point de fusion 242°C.
   ¹H NMR (CDCl₃) δ 1,37 (s, 6H), 1,98 à 2,02 (m, 2H), 3,05 à 3,10 (m, 2H), 7,48 (d, 1H, *J* = 7,9 Hz), 7,82 (dd, 1H, *J* = 7,9 / 1,2 Hz), 7,89 (d, 1H, *J* = 1,0 Hz).
(k) 2'-(4,4-diméthyl-thiochroman-7-yl)-[1,1';4',1'']terphényl-4''-carboxylate de méthyle.
   De manière analogue à l'exemple 13(b), par réaction de 1,81 g (4,1 mmoles) du composé obtenu à l'exemple 34(g) avec 1,04 g (5,0 mmoles) de l'acide boronique obtenu à l'exemple 34 (j), on obtient 570 mg (30%) du produit attendu, sous la forme d'un solide blanc de point de fusion 172°C.
   ¹H NMR (CDCl₃) δ 1,29 (s, 6H), 1,96 (t, 2H, *J* = 5,9 Hz), 3,02 (t, 2H, *J* = 5,9 Hz), 3,95 (s, 3H), 6,69 (dd, 1H, *J* = 8,1 / 1,8 Hz), 7,04 (d, 1H, *J* = 1,8 Hz), 7,14 (d, 1H, *J* = 8,2 Hz), 7,19 à 7,26 (m, 5H), 7,50 (d, 1H, *J* = 8,5 Hz), 7,65 à 7,67 (m, 2H), 7,73 (d, 2H, *J=* 8,4 Hz), 8,12 (d, 2H, *J* = 8,3 Hz).
(l) acide 2'-(4,4-diméthyl-thiochroman-7-yl)-[1,1';4',1"] terphényl-4"-carboxylique.
   De manière analogue à l'exemple 1(e), à partir de 570 mg (1,2 mmole) de l'ester obtenu à l'exemple 34(k), on obtient 500 mg (90%) d'acide 2'-(4,4-diméthyl-thiochroman-7-yl)-[1,1';4',1"] terphényl-4"-carboxylique, sous la forme d'un solide blanc cristallisé de point de fusion 261°C.
   ¹H NMR (CDCl₃ + 2 gouttes de DMSO D₆) δ 0,64 (s, 6H), 1,53 (t, 2H, *J* = 5,9 Hz), 2,65 (t, 2H, *J* = 6,0 Hz), 6,63 (d, 1H, *J* = 1,6 Hz), 6,71 (d, 1H, *J* = 8,0 Hz), 6,77 (dd, 1H, *J* = 8,1 / 1,7 Hz), 6,84 à 6,97 (m, 5H), 7,19 (d, 1H, *J* = 8,6 Hz), 7,32 à 7,36 (m, 2H), 7,42 (d, 2H, *J* = 8,3 Hz), 7,81 (d, 2H, *J* = 8,3 Hz).

### EXEMPLE 35

### Acide 2'-(4,4-diméthyl-thiochroman-6-yl)-[1,1';4',1"] terphényl-4"-carboxylique.

(a) 1-bromo-4-(3-méthyl-but-2-ènylsulfanyl)-benzène.
   De manière analogue à l'exemple 34(h), par réaction de 30,00 g (159,0 mmoles) de 4-bromothiophénol avec 26,00 g (175,0 mmoles) de 1-bromo-3-méthyl-2-butène, on obtient 37,40 g (93%) du produit attendu, sous la forme d'une huile jaune.
   ¹H NMR (CDCl₃) δ 1,59 (s, 3H), 1,71 (s, 3H), 3,51 (d, 2H, *J* = 7,7 Hz), 5,27 (t, 1H, *J* = 7,7 Hz), 7,19 (d, 2H, *J* = 8,5 Hz), 7,38 (d, 2H, *J* = 8,5 Hz).
(b) 6-bromo-4, 4-diméthyl-thiochromane.
   De manière analogue à l'exemple 34(i), à partir de 34,00 g (132,0 mmoles) du composé obtenu à l'exemple 35(a), on obtient 21,80 g (64%) du produit attendu, sous la forme d'un solide marron de point de fusion 51°C.
   ¹H NMR (CDCl₃) δ 1,31 (s, 6H), 1,93 (t, 2H, *J* = 6,0 Hz), 3,01 (t, 2H, *J* = 6,1 Hz), 6,94 (d, 1H, *J* = 8,4 Hz), 7,13 (dd, 1H, *J* = 8,4 / 2,2 Hz), 7,45 (d, 1H, *J* = 2,1 Hz).
(c) acide 6-bromo-4,4-diméthyl-thiochromane boronique.
   De manière analogue à l'exemple 1(a), à partir de 5,00 g (20,4 mmoles) du composé obtenu à l'exemple 35(b), on obtient 2,28 g (53%) du produit attendu, sous la forme d'un solide blanc de point de fusion 242°C.
   ¹H NMR (CDCl₃) δ 1,43 (s, 6H), 1,98 à 2,04 (m, 2H), 3,06 à 3,11 (m, 2H), 7,21 (d, 1H, *J* = 7,8 Hz), 7,81 (dd, 1H, *J* = 7,8 / 1,1 Hz), 8,20 (d, 1H, *J* = 1,1 Hz).
(d) 2'-(4,4-diméthyl-thiochroman-6-yl)-[1,1';4',1'']terphényl-4''-carboxylate de méthyle.
   De manière analogue à l'exemple 13(b), par réaction de 1,81 g (4,1 mmoles) du composé obtenu à l'exemple 34(g) avec 1,04 g (5,0 mmoles) de l'acide boronique obtenu à l'exemple 35(c), on obtient 680 mg (35%) du produit attendu, sous la forme d'une huile incolore.
   ¹H NMR (CDCl3) δ 1,27 (s, 6H), 1,83 à 1,88 (m, 2H), 2,94 à 2,99 (m, 2H), 3,94 (s, 3H), 6,96 (d, 1H, *J* = 1,4 Hz), 7,04 à 7,25 (m, 7H), 7,51 (d, 1H, *J* = 7,9 Hz), 7,65 (dd, 1H, *J* = 7,9 / 2,0 Hz), 7,69 (d, 1H, *J* = 1,8 Hz), 7,73 (d, 2H, *J* = 8,5 Hz), 8,13 (d, 2H, *J* = 8,5 Hz).
(e) acide 2'-(4,4-diméthyl-thiochroman-6-yl)-[1,1';4',1''] terphényl-4''-carboxylique.
   De manière analogue à l'exemple 1(e) à partir de 680 mg (1,5 mmole) de l'ester obtenu à l'exemple 35(d), on obtient 280 mg (42%) d'acide 2'-(4,4-diméthyl-thiochroman-6-yl)-[1,1';4',1"]terphényl-4"-carboxylique, sous la forme d'un solide blanc cristallisé de point de fusion 279°C.
   ¹H NMR (CDCl₃) δ 1,29 (s, 6H), 1,95 (t, 2H, *J* = 5,9 Hz), 3,01 (t, 2H, *J* = 5,9 Hz), 6,70 (dd, 1H, *J* = 8,2 / 1,9 Hz), 7,01 (d, 1H, *J* = 1,9 Hz), 7,15 (d, 1H, *J* = 8,3 Hz), 7,17 à 7,42 (m, 5H), 7,50 (d, 1H, *J* = 8,7 Hz), 7,65 à 7,69 (m, 2H), 7,73 (d, 2H, *J* = 8,4 Hz), 8,13 (d, 2H, *J* = 8,4 Hz).

### EXEMPLE 36

### Acide 2'-(3,5,5,8,8-pentaméthyl-5,6,7,8-tétrahydro-naphthalèn-2-yl)-[1,1';4',1"]terphényl-4"-carboxylique.

(a) 6-bromo-1,1,4,4,7-pentaméthyl-1,2,3,4-tétrahydro-naphthalène.
   Dans un tricol, sous atmosphère d'argon, on introduit 18,31 g (100,0 mmoles) de dichloro-2,5-diméthyl-2,5-hexane, 17,10 g (100,0 mmoles) de 2-bromotoluène et 200 ml de 1,2-dichloroéthane. On ajoute rapidement et en une fois 1,33 g (10,0 mmoles) de chlorure d'aluminium et agite le milieu réactionnel pendant trente minutes à la température ambiante. On verse le milieu réactionnel dans l'eau, extrait par du dichlorométhane, lave à l'eau, décante la phase organique, sèche sur sulfate de magnésium, évapore. On recueille, après recristallisation du résidu dans du méthanol, 17,78 g (63%) du composé attendu, sous la forme de fins cristaux blancs de point de fusion 73°C.
   ¹H NMR (CDCl₃) δ 1,25 (s, 12H), 1,65 (s, 4H), 2,33 (s, 3H), 7,14 (s, 1H), 7,42 (s, 1H).
(b) acide 5,6,7,8-tétrahydro-3,5,5,8,8-pentaméthyl-2-naphtylboronique.
   De manière analogue à l'exemple 1(a), à partir de 14,00 g (49,8 mmoles) du composé obtenu à l'exemple 36(a), on obtient 7,36 g (60%) du produit attendu, sous la forme d'une huile incolore.
   ¹H NMR (CDCl₃) δ 1,32 (s, 6H), 1,34 (s, 6H), 1,72 (s, 4H), 2,81 (s, 3H), 7,21 (s, 1H), 8,28 (s, 1H).
(c) 4-[4-hydroxy-3-(5,6,7,8-tétrahydro-3,5,5,8,8-pentaméthyl-2-naphtyl)phényl] benzoate d'éthyle.
   De manière analogue à l'exemple 1(d), par réaction de 2,26 g (7,0 mmoles) du composé obtenu à l'exemple 1(c) avec 2,08 g (8,4 mmoles) de l'acide boronique obtenu à l'exemple 36(b), on obtient 1,00 g (32%) du produit attendu, sous la forme d'une huile incolore.
   ¹H NMR (CDCl₃) δ 1,27 (s, 6H), 1,33 (s, 6H), 1,41 (t, 3H, *J* = 7,1 Hz), 1,71 (s, 4H), 2,17 (s, 3H), 4,39 (q, 2H, *J* = 7,1 Hz), 5,05 (s, 1H), 7,09 (d, 1H, *J* = 8,4 Hz), 7,21 (s, 1H), 7,25 (s, 1H), 7,45 (d, 1H, *J* = 2,3 Hz), 7,56 (dd, 1H, *J* = 8,4 / 2,3 Hz), 7,64 (d, 2H, *J* = 8,4 Hz), 8,08 (d; 2H, *J* = 8,4 Hz).
(d) 3'-(3,5,5,8,8-pentaméthyl-5,6,7,8-tétrahydro-naphthalèn-2-yl)-4'-trifluorométhanesulfonyloxy-biphényl-4-carboxylate d'éthyle.
   De manière analogue à l'exemple 13(a), à partir de 1,00 g (2,3 mmoles) de l'ester obtenu à l'exemple 36(c), on obtient 1,30 g (100%) du produit attendu, sous la forme d'une huile jaune.
   ¹H NMR (CDCl₃) δ 1,26 (d, 6H, *J* = 6,8 Hz), 1,31 (d, 6H, *J* = 6,5 Hz), 1,42 (t, 3H, *J* = 7,1 Hz), 1,71 (s, 4H), 2,15 (s, 3H), 4,40 (q, 2H, *J* = 7,1 Hz), 7,16 (s, 1H), 7,21 (s, 1H), 7,43 à 7,47 (m, 1H), 7,64 à 7,68 (m, 2H), 7,67 (d, 2H, *J* = 8,4 Hz), 8,13 (d, 2H, *J* = 8,4 Hz).
(e) 2'-(3,5,5,8,8-pentaméthyl-5,6,7,8-tétrahydro-naphthalèn-2-yl)-[1,1';4',1"]terphényl-4"-carboxylate d'éthyle.
   De manière analogue à l'exemple 13(b), par réaction de 1,00 g (1,7 mmole) du composé obtenu à l'exemple 36(d) avec 254 mg (2,1 mmoles) d'acide benzène boronique, on obtient 770 mg (88%) du produit attendu sous la forme d'une huile incolore qui sera utilisée directement pour l'étape suivante.
(f) acide 2'-(3,5,5,8,8-pentaméthyl-5,6,7,8-tétrahydro-naphthalèn-2-yl)-[1,1';4',1'']terphényl-4''-carboxylique.
   De manière analogue à l'exemple 1(e), à partir de 770 mg (1,5 mmole) de l'ester obtenu à l'exemple 36(e), on obtient 150 mg (21%) d'acide 2'-(3,5,5,8,8-pentaméthyl-5,6,7,8-tétrahydro-naphthalèn-2-yl)-[1,1';4',1'']terphényl-4''-carboxylique, sous la forme d'une poudre beige de point de fusion 217°C.
   ¹H NMR (CDCl₃ + 2 gouttes de DMSO D₆) δ 1,20 (s, 6H), 1,26 (s, 6H), 1,63 (s, 4H), 2,11 (s, 3H), 6,28 (br s, 1H), 6,83 à 7,16 (m, 5H), 7,23 (s, 1H), 7,37 (d, 1H, *J* = 2,2 Hz), 7,47 (dd, 1H, *J* = 8,4 / 2,4 Hz), 7,57 (d, 2H, *J* = 8,4 Hz), 8,02 (d, 2H, *J* = 8,3 Hz).

### EXEMPLE 37

### Acide 2'-(3-méthoxyméthoxy-5,5,8,8-tetraméthyl-5,6,7,8-tétrahydro-naphthalèn-2-yl)-[1,1';4',1'']terphényl-4''-carboxylique.

(a) 3-bromo-5,5,8,8-tétraméthyl-5,6,7,8-tétrahydro-naphthalèn-2-ol.
   De manière analogue à l'exemple 36(a), par réaction de 67,14 g (388,0 mmoles) de 2-bromophénol avec 71,10 g (388,0 mmoles) de 2,5-dichloro-2,5-diméthylhexane, on obtient 86,13 g (78%) du produit attendu, sous la forme d'un solide blanc de point de fusion 90-94°C.
   ¹H NMR (CDCl₃) δ 1,16 (s, 6H), 1,17 (s, 6H), 1,57 (s, 4H), 5,21 (s, 1H), 6,87 (s, 1H), 7,26 (S, 1H).
(b) 6-bromo-7-méthoxyméthoxy-1,1,4,4-tetraméthyl-1,2,3,4-tétrahydro-naphthalène.
   De manière analogue à l'exemple 7(a), par réaction de 8,00 g (28,2 mmoles) du composé obtenu à l'exemple 37(a) avec 2,36 ml (31,1 mmoles) d'éther méthylique de chlorométhyle, on obtient 9,49 g (100%) du produit attendu, sous la forme d'une huile beige.
   ¹H NMR (CDCl₃) δ 1,24 (s, 6H), 1,26 (s, 6H), 1,65 (s, 4H), 3,53 (s, 3H), 5,20 (s, 2H), 7,06 (s, 1H), 7,42 (s, 1H).
(c) acide 3-méthoxyméthoxy-5,6,7,8-tétrahydro-5,5,8,8-tétraméthyl-2-naphtylboronique.
   De manière analogue à l'exemple 1(a), à partir de 9,49 g (29,0 mmoles) du composé obtenu à l'exemple 37(b), on obtient 8,21 g (97%) du produit attendu, sous la forme d'une huile jaune.
   ¹H NMR (CDCl₃) δ 1,12 (s, 12H), 1,51 (s, 4H), 3,34 (s, 3H), 5,10 (s, 2H), 6,40 (s, 2H), 6,88 (s, 1H), 7,64 (s, 1H).
(d) 4-[4-hydroxy-3-(3-méthoxyméthoxy-5,6,7,8-tétrahydro-5,5,8,8-tétraméthyl-2-naphtyl)phényl] benzoate d'éthyle.
   De manière analogue à l'exemple 1(d), par réaction de 8,10 g (25,2 mmoles) du composé obtenu à l'exemple 1(c) avec 6,15 g (21,0 mmoles) de l'acide boronique obtenu à l'exemple 37(c), on obtient 5,26 g (51%) du produit attendu, sous la forme d'une huile jaune clair.
   ¹H NMR (CDCl₃) δ 1,21 (s, 6H), 1,25 (s, 6H), 1,33 (t, 3H, *J* = 7,1 Hz), 1,63 (s, 4H), 3,31 (s, 3H), 4,31 (q, 2H, *J* = 7,1 Hz), 5,06 (s, 2H), 6,31 (s, 1H), 7,00 (d, H, *J* = 8,3 Hz), 7,09 (s, 1H), 7,20 (s, 1H), 7,45 à 7,48 (dd, 1H, *J* = 8,3 / 2,3 Hz), 7,51 (d, 1H, *J* = 2,3 Hz), 7,57 (d, 2H, *J* = 8,4 Hz), 8,01 (d, 2H, *J* = 8,4 Hz).
(e) 3'-(3-méthoxyméthoxy-5,5,8,8-tétraméthyl-5,6,7,8-tétrahydro-naphthalèn-2-yl)-4'-trifluorométhanesulfonyloxy-biphényl-4-carboxylate d'éthyle.
   Dans un tricol et sous courant d'azote, on introduit 4,76 g (9,7 mmoles) de l'ester éthylique obtenu à l'exemple 37(d), 2,64 g (9,7 mmoles) de triflate de 4-nitrophénol, 2,64 g (1,9 mmoles) de carbonate de potassium et 100 ml de 1,2-diméthoxyéthane. On agite à température ambiante pendant trois heures, verse le milieu réactionnel dans un mélange d'eau et d'éther éthylique, décante la phase organique, extrait par de l'éther éthylique, lave à l'eau jusqu'à disparition du 4-nitrophénol de la phase aqueuse, sèche sur sulfate de magnésium, évapore. On recueille 6,05 g (100%) du produit attendu sous la forme d'une huile beige.
   ¹H NMR (CDCl₃) δ 1,26 (t, 3H, *J* = 7,1 Hz), 1,27 (s, 6H), 1,32 (s, 6H), 1,72 (s, 4H), 3,37 (s, 3H), 3,41 (q, 2H, *J* = 7,1 Hz), 5,10 (s, 2H), 7,17 (s, 1H), 7,19 (s, 1H), 7,42 (d, 1H, *J* = 8,5 Hz), 7,62 à 7,71 (m, 4H), 8,13 (d, 2H, *J* = 8,4 Hz).
(f) 2'-(3-méthoxyméthoxy-5,5,8,8-tetraméthyl-5,6,7,8-tétrahydro-naphthalèn-2-yl)-[1,1';4',1"]terphényl-4"-carboxylate d'éthyle.
   De manière analogue à l'exemple 13(b), par réaction de 6,59 g (10,6 mmoles) du composé obtenu à l'exemple 37(e) avec 1,55 g (12,7 mmoles) d'acide benzène boronique, on obtient 5,30 g (91%) du produit attendu, sous la forme d'une huile incolore.
   ¹H NMR (CDCl₃) δ 1,26 (s, 6H), 1,27 (s, 6H), 1,30 (t, 3H, *J* = 7,1 Hz), 1,60 à 1,64 . (m, 4H), 3,22 (s, 3H), 4,41 (q, 2H, *J* = 7,1 Hz), 4,75 (br s, 2H), 6,93 (s, 1H), 7,02 (s, 1H), 7,16 à 7,19 (m, 2H), 7,44 (d, 1H, *J* = 8,5 Hz), 7,55 (d, 1H, *J* = 8,0 Hz), 7,62 à 7,77 (m, 6H), 8,13 (d, 2H, *J* = 7,6 Hz).
(g) acide 2'-(3-méthoxyméthoxy-5,5,8,8-tetraméthyl-5,6,7,8-tétrahydro-naphthalèn-2-yl)-[1,1';4',1"]terphényl-4"-carboxylique.
   De manière analogue à l'exemple 1(e), à partir de 1,00 g (1,8 mmole) de l'ester obtenu à l'exemple 37(f), on obtient 540 mg (57%) d'acide 2'-(3-méthoxyméthoxy-5,5,8,8-tetraméthyl-5,6,7,8-tétrahydro-naphthalèn-2-yl)-[1,1';4',1"]terphényl-4"-carboxylique, sous la forme d'un solide blanc cristallisé de point de fusion 219-222°C.
   ¹H NMR (CDCl₃) δ 1,03 (br s, 6H), 1,26 (s, 6H), 1,58 à 1,66 (m, 4H), 3,21 (s, 3H), 4,75 (br s, 2H), 6,93 (s, 1H), 7,01 (s, 1H), 7,16 à 7,21 (m, 5H), 7,55 (d, 1H, *J* = 8,0 Hz), 7,68 (dd, 1H, *J* = 8,0 / 1,9 Hz), 7,72 (s, 1H), 7,74 (d, 2H, *J* = 8,4 Hz), 8,14 (d, 2H, *J* = 8,4 Hz).

### EXEMPLE 38

### Acide 2'-(3-hydroxy-5,5, 8, 8-tétraméthyl-5,6, 7,8-tétrahydro-naphthalèn-2-yl)-[1,1';4',1'']terphényl-4''-carboxylique.

(a) 2'-(3-hydroxy-5,5,8,8-tétraméthyl-5,6,7,8-tétrahydro-naphthalèn-2-yl)-[1,1';4',1"]terphényl-4"-carboxylate d'éthyle.
   De manière analogue à l'exemple 16(a), à partir de 4,30 g (7,8 mmoles) du composé obtenu à l'exemple 37(f), on obtient 1,65 g (42%) du produit attendu, sous la forme d'une poudre blanche de point de fusion 145°C.
   ¹H NMR (CDCl₃) δ 0,98 (s, 6H), 1,24 (s, 6H), 1,42 (t, 3H, *J* = 7,1 Hz), 1,56 à 1,64 (m, 4H), 4,41 (q, 2H, *J* = 7,1 Hz), 4,76 (s, 1H), 6,80 (d, 2H, *J* = 7,8 Hz), 7,13 à 7,23 (m, 5H), 7,62 (d, 1H, *J* = 7,9 Hz), 7,72 à 7,73 (m, 1H), 7,74 (d, 2H, *J* = 8,4 Hz), 8,13 (d, 2H, *J* = 8,4 Hz).
(b) acide 2'-(3-hydroxy-5,5,8,8-tétraméthyl-5,6,7,8-tétrahydro-naphthalèn-2-yl)-[1,1';4',1"]terphényl-4"-carboxylique.
   De manière analogue à l'exemple 1(e), à partir de 600 mg (1,1 mmole) de l'ester obtenu à l'exemple 38(a), on obtient 400 mg (70%) d'acide 2'-(3-hydroxy-5,5,8,8-tétraméthyl-5,6,7,8-tétrahydro-naphthalèn-2-yl)-[1,1';4',1"]terphényl-4"-carboxylique, sous la forme d'un solide blanc cristallisé de point de fusion 273°C.
   ¹H NMR (CDCl₃ + 2 gouttes de DMSO D₆) δ 0,91 (s, 6H), 1,23 (s, 6H), 1,53 à 1,61 (m, 4H), 6,70 (s, 1H), 6,79 (s, 1H), 7,13 à 7,18 (m, 5H), 7,54 (d, 1H, *J* = 8,0 Hz), 7,66 (dd, 1H, *J* = 8,0 / 1,9 Hz), 7,74 (d, 2H, *J* = 8,3 Hz), 7,82 (d, 1H, *J* = 1,8 Hz), 8,10 (d, 2H, *J* = 8,3 Hz), 8,18 (br s, 1H).

### EXEMPLE 39

### Acide 2'-(3-méthoxy-5,5,8,8-tétraméthyl-5,6,7,8-tétrahydro-naphthalèn-2-yl)-[1,1';4',1"]terphényl-4"-carboxylique

(a) 2'-(3-méthoxy-5,5,8,8-tétraméthyl-5,6,7,8-tétrahydro-naphthalèn-2-yl)-[1,1';4',1"]terphényl-4"-carboxylate d'éthyle.
   De manière analogue à l'exemple 7(a), par réaction de 530 mg (1,1 mmole) du composé obtenu à l'exemple 38(a) avec 71 µl (1,1 mmole) d'iodure de méthyle, on obtient 544 mg (100%) du produit attendu, sous la forme d'une huile incolore.
   ¹H NMR (CDCl₃) δ 1,04 (s, 6H), 1,27 (s, 6H), 1,42 (t, 3H, *J* = 7,1 Hz), 1,59 à 1,67 (m, 4H), 3,48 (s, 3H), 4,40 (q, 2H, *J* = 7,1 Hz), 6,68 (s, 1H), 6,94 (s, 1H), 7,10 à 7,19 (m, 5H), 7,54 (d, 1H, *J* = 8,0 Hz), 7,67 (dd, 1H, *J* = 8,0 / 2,0 Hz), 7,73 à 7,74 (m, 1H), 7,75 (d, 2H, *J* = 8,4 Hz), 8,12 (d, 2H, *J* = 8,4 Hz).
(b) acide 2'-(3-méthoxy-5,5,8,8-tétraméthyl-5,6,7,8-tétrahydro-naphthalèn-2-yl)-[1,1';4',1"]terphényl-4"-carboxylique.
   De manière analogue à l'exemple 1(e), à partir de 544 mg (1,0 mmole) de l'ester obtenu à l'exemple 39(a), on obtient 490 mg (95%) d'acide 2'-(3-méthoxy-5,5,8,8-tétraméthyl-5,6,7,8-tétrahydro-naphthalèn-2-yl)-[1,1';4',1"]terphényl-4"-carboxylique, sous la forme d'un solide blanc cristallisé de point de fusion 248°C.
   ¹H NMR (CDCl₃ + 2 gouttes de DMSO D₆) δ 1,04 (s, 6H), 1,27 (s, 6H), 1,59 à 1,67 (m, 4H), 3,48 (s, 3H), 6,67 (s, 1H), 6,93 (s, 1H), 7,10 à 7,18 (m, 5H), 7,54 (d, 1H, *J* = 8,0 Hz), 7,67 (dd, 1H, *J* = 8,0 / 2,0 Hz), 7,73 (d, 2H, *J* = 8,3 Hz), 7,74 (s, 1H), 8,13 (d, 2H, *J* = 8,3 Hz).

### EXEMPLE 40

### Acide 2'-(3-propyloxy-5,5,8,8-tétraméthyl-5,6,7,8-tétrahydro-naphthalèn-2-yl)-[1,1';4',1"]terphényl-4"-carboxylique

(a) 2'-(3-propyloxy-5,5,8,8-tétraméthyl-5,6,7,8-tétrahydro-naphthalèn-2-yl)-[1,1';4',1"]terphényl-4"-carboxylate d'éthyle.
   De manière analogue à l'exemple 7(a), par réaction de 450 mg (8,3 mmoles) du composé obtenu à l'exemple 38(a) avec 89 µl (9,2 mmoles) d'iodure de propyle, on obtient 450 mg (92%) du produit attendu, sous la forme d'un solide jaune de point de fusion 163°C, qui sera utilisé directement pour l'étape suivante.
(b) acide 2'-(3-propyloxy-5,5,8,8-tétraméthyl-5,6,7,8-tétrahydro-naphthalèn-2-yl)-[1,1';4',1"]terphényl-4"-carboxylique.
   De manière analogue à l'exemple 1(e), à partir de 450 mg (0,8 mmole) de l'ester obtenu à l'exemple 40(a), on obtient 400 mg (94%) d'acide 2'-(3-propyloxy-5,5,8,8-tétraméthyl-5,6,7,8-tétrahydro-naphthalèn-2-yl)-[1,1';4',1"]terphényl-4"-carboxylique, sous la forme d'un solide blanc cristallisé de point de fusion 234°C.
   ¹H NMR (CDCl₃ + 2 gouttes de DMSO D₆) δ 0,85 (t, 3H, *J* = 7,5 Hz), 0,95 (br s, 6H), 1,27 (s, 6H), 1,54 à 1,70 (m, 6H), 3,72 (t, 2H, *J* = 6,6 Hz), 6,73 (s, 1H), 6,82 (s, 1H), 7,11 à 7,17 (m, 5H), 7,53 (d, 1H, *J* = 8,0 Hz), 7,66 (dd, 1H, *J* = 8,0 / 1,9 Hz), 7,74 (d, 2H, *J* = 8,4 Hz), 7,79 (d, 1H, *J* = 1,8 Hz), 8,13 (d, 2H, *J* = 8,3 Hz).

### EXEMPLE 41

### Acide 3"-méthyl-2'-(5,5,8,8-tétraméthyl-5,6,7,8-tétrahydro-naphthalèn-2-yl)-[1,1';4',1"]terphényl-4"-carboxylique

(a) 2-bromo-4-nitro-phénol.
   Dans un tricol, sous atmosphère d'argon, on introduit 139,40 g (64,7 mmoles) de 4-nitrophénol, et 130 ml de dichlorométhane. On refroidit à 0°C et ajoute goutte à goutte 3,31 ml (67,7 mmoles) de brome. Le milieu réactionnel est agité pendant une heure à 0°C, puis on ajoute 360 mg (6,5 mmoles) de fer en poudre et agite pendant seize heures à la température ambiante. On verse le milieu réactionnel dans l'eau, ajoute une solution saturée de thiosulfate de sodium, extrait par du dichlorométhane, lave à l'eau, décante la phase organique, sèche sur sulfate de magnésium et évapore les solvants. On recueille 13,50 g (96%) du composé attendu, sous la forme d'une poudre beige de point de fusion 105-107°C.
   ¹H NMR (CDCl₃) δ 6,34 (br s, 1H), 7,13 (d, 1H, *J* = 9,0 Hz), 8,16 (dd, 1H, *J* = 9,1 / 2,7 Hz), 8,44 (d, 1H, *J* = 2,7 Hz).
(b) 4-nitro-2-(5,5,8,8-tétraméthyl-5,6,7,8-tétrahydro-naphthalèn-2-yl)-phénol.
   De manière analogue à l'exemple 1(d), par réaction de 160,00 g (672,0 mmoles) d'acide boronique obtenu à l'exemple 1(a) avec 100,00 g (459,0 mmoles) du composé obtenu à l'exemple 41(a), on obtient 81,70 g (55%) du composé attendu, sous la forme d'une huile jaune.
   ¹H NMR (CDCl₃) δ 1,25 (s, 6H), 1,26 (s, 6H), 1,67 (s, 4H), 5,89 (br s, 1H), 7,00 (d, 1H, *J* = 9,7 Hz), 7,13 (dd, 1H, *J* = 8,1 / 1,9 Hz), 7,28 (d, 1H, *J* = 1,9 Hz), 7,41 (d, 1H, *J* = 8,1 Hz), 8,07 à 8,11 (m, 2H).
(c) trifluoro-méthanesulfonate de 4-nitro-2-(5,5,8,8-tétraméthyl-5,6,7,8-tétrahydro-naphthalèn-2-yl)-phényle.
   De manière analogue à l'exemple 13(a), à partir de 78,50 g (241,0 mmoles) du composé obtenu à l'exemple 41(b), on obtient 81,40 g (73%) du produit attendu, sous la forme d'une poudre grise de point de fusion 87-89°C.
   ¹H NMR (CDCl₃) δ 1,31 (s, 6H), 1,32 (s, 6H), 1,73 (s, 4H), 7,21 (dd, 1H, *J* = 8,2 / 2,0 Hz), 7,39 (d, 1H, *J* = 1,9 Hz), 7,44 (d, 1H, *J* = 8,2 Hz), 7,56 (d, 1H, *J* = 9,0 Hz), 8,27 (dd, 1H, *J* = 9,0 / 2,9 Hz), 8,37 (d, 1H, *J* = 2,8 Hz).
(d) 1,1,4,4-tétraméthyl-6-(4-nitro-biphényl-2-yl)-1,2,3,4-tétrahydro-naphthalène.
   De manière analogue à l'exemple 13(b), par réaction de 81,00 g (177,0 mmoles) du composé obtenu à l'exemple 41(c) avec 32,20 g (265,0 mmoles) d'acide phénylboronique, on obtient 62,20 g (91%) du produit attendu, sous la forme d'une poudre beige de point de fusion 181-183°C.
   ¹H NMR (CDCl₃) δ 0,90 (s, 6H), 1,26 (s, 6H), 1,56 à 1,64 (m, 4H), 6,84 (d, 1H, *J* = 1,9 Hz), 7,09 à 7,15 (m, 3H), 7,25 à 7,30 (m, 4H), 7,56 (d, 1H, *J* = 8,4 Hz), 8,21 (dd, 1H, *J* = 8,5 / 2,4 Hz), 8,32 (d, 1H, *J* = 2,4 Hz).
(e) 2-(5,5,8,8-tétraméthyl-5,6,7,8-tétrahydro-naphthalèn-2-yl)-biphényl-4-ylamine.
   Dans un hydrogénateur de deux litres, on introduit 62,00 g (160,0 mmoles) du composé obtenu à l'exemple 41(d) et un litre de méthanol. On purge à l'azote, ajoute 1,85 g de palladium sur charbon à 5%, purge à l'hydrogène et agite le milieu réactionnel pendant six heures à 60°C sous une pression de sept bars d'hydrogène. Après refroidissement du milieu réactionnel et filtration sur Célite® , les solvants sont évaporés et le produit purifié par chromatographie sur colonne de silice, élué par un mélange composé de 80% d'heptane et de 20% d'acétate d'éthyle. Après évaporation des solvants, on recueille 43,00 g (75%) du composé attendu, sous la forme d'une huile orange.
   ¹H NMR (CDCl₃) δ 1,25 (s, 12H), 1,53 à 1,63 (m, 4H), 3,74 (br s, 2H), 6,72 (dd, 1H, *J* = 8,1 / 2,5 Hz), 6,79 (d, 1H, *J* = 2,4 Hz), 6,86 (d, 1H, *J* = 1,9 Hz), 7,04 à 7,24 (m, 8H).
(f) 6-(4-iodo-biphényl-2-yl)-1,1,4,4-tétraméthyl-1,2,3,4-tétrahydro-naphthalène.
   Dans un ballon de 500 ml, sous atmosphère d'argon, on introduit 40,00 g (113,0 mmoles) du composé obtenu à l'exemple 41(e) et 113 ml (113 mmoles) d'une solution de diiodométhane 1M. On coule goutte à goutte 45,5 ml de nitrite d'isoamyle et chauffe le milieu réactionnel pendant vingt minutes à 60°C. Après évaporation à sec, le produit est purifié par chromatographie sur colonne de silice, élué par un mélange composé de 90% d'heptane et de 10% d'acétate d'éthyle. Après évaporation des solvants, on recueille 21,00 g (40%) du composé attendu, sous la forme d'une poudre blanc cassé de point de fusion 120-122°C.
   ¹H NMR (CDCl₃) δ 0,89 (s, 6H), 1,25 (s, 6H), 1,54 à 1,62 (m, 4H), 6,80 (d, 1H, *J* = 1,9 Hz), 7,03 à 7,24 (m, 8H), 7,70 (dd, 1H, *J* = 8,1 / 1,9 Hz), 7,80 (d, 1H, *J* = 1,8 Hz).
(g) acide 3-(5,5,8,8-tétraméthyl-5,6,7,8-tétrahydro-naphthalèn-2-yl)-4-phényl-benzène boronique.
   De manière analogue à l'exemple 1(a), à partir de 18,00 g (38,0 mmoles) du composé obtenu à l'exemple 41(f), on obtient 11,90 g (81%) du produit attendu, sous la forme d'un solide blanc-rosé de point de fusion 257-259°C.
   ¹H NMR (CDCl₃) δ 0,93 (s, 6H), 1,28 (s, 6H), 1,58 à 1,63 (m, 4H), 6,92 (d, 1H, *J* = 1,7 Hz), 7,20 à 7,31 (m, 7H), 7,56 (d, 1H, *J* = 7,6 Hz), 8,28 (dd, 1H, *J* = 8,7 / 1,1 Hz), 8,34 (s, 1H).
(h) 3"-méthyl-2'-(5,5,8,8-tétraméthyl-5,6,7,8-tétrahydro-naphthalèn-2-yl)-[1,1';4',1"]terphényl-4"-carboxylate de méthyle.
   De manière analogue à l'exemple 1(d), par réaction de 700 mg (1,8 mmole) du composé obtenu à l'exemple 41(g) avec 380 mg (1,7 mmole) de 2-méthyl-4-bromobenzoate de méthyle, on obtient 740 mg (91%) du produit attendu, sous la forme d'un solide blanc de point de fusion 130-132°C.
   ¹H NMR (CDCl₃) δ 0,91 (s, 6H), 1,27 (s, 6H), 1,54 à 1,64 (m, 4H), 2,68 (s, 3H), 3,92 (s, 3H), 6,89 (d, 1H, *J* = 1,7 Hz), 7,15 à 7,29 (m, 7H), 7,50 à 7,56 (m, 3H), 7,65 (dd, 1H, *J* = 7,9 / 1,8 Hz), 7,72 (d, 1H, *J* = 1,7 Hz), 8,02 (d, 1H, *J* = 8,7 Hz).
(i) acide 3''-méthyl-2'-(5,5,8,8-tétraméthyl-5,6,7,8-tétrahydro-naphthalèn-2-yl)-[1,1';4',1'']terphényl-4''-carboxylique.
   De manière analogue à l'exemple 1(e), à partir de 700 mg (1,4 mmole) de l'ester obtenu à l'exemple 41(h), on obtient 537 mg (79%) d'acide 3''-méthyl-2'-(5,5,8,8-tétraméthyl-5,6,7,8-tétrahydro-naphthalèn-2-yl)-[1,1';4',1'']terphényl-4''-carboxylique, sous la forme d'un solide blanc cristallisé de point de fusion 237-239°C.
   ¹H NMR (CDCl₃) δ 0,91 (s, 6H), 1,27 (s, 6H), 1,57 à 1,65 (m, 4H), 2,75 (s, 3H), 6,90 (d, 1H, *J* = 1,8 Hz), 7,15 à 7,30 (m, 7H), 7,53 (d, 1H, *J* = 7,9 Hz), 7,59 à 7,61 (m, 2H), 7,66 (dd, 1H, *J* = 8,0 / 1,9 Hz), 7,74 (d, 1H, *J* = 1,8 Hz), 8,19 (d, 1H, *J* = 8,7 Hz).

### EXEMPLE 42

### Acide 2"-hydroxy-2'-(5,5,8,8-tétraméthyl-5,6,7,8-tétrahydro-naphthalèn-2-yl)-[1,1';4',1'']terphényl-4''-carboxylique.

(a) acide 3-hydroxy-4-iodo-benzoïque.
   Dans un tricol de un litre, et sous courant d'azote, on introduit 25,00 g (180,0 mmoles) d'acide 3-hydroxybenzoïque, 7,20 g (180,0 mmoles) de soude en pastilles, 27,13 g (180,0 mmoles) d'iodure de sodium et 500 ml de méthanol. On refroidit à 0°C et ajoute goutte à goutte, en une heure et cinquante minutes, 374,30 g (180,0 mmoles) d'une solution aqueuse d'hypochlorite de sodium. Le milieu réactionnel est agité pendant deux heures à 0°C, puis on ajoute une solution de thiosulfate de sodium, acidifie à pH 5, extrait avec de l'éther éthylique, lave la phase organique à l'eau jusqu'à pH neutre, sèche sur sulfate de magnésium, filtre et évapore les solvants. On recueille 43,80 g (92%) du composé attendu, sous la forme d'une poudre beige de point de fusion 198°C.
   ¹H NMR (DMSO D₆) δ 7,13 (dd, 1H, *J* = 8,1 / 1,9 Hz), 7,43 (d, 1H, *J* = 1,8 Hz), 7,80 (d, 1H, *J* = 8,1 Hz), 10,69 (br s, 1H), 12,98 (br s, 1H).
(b) 3-hydroxy-4-iodo-benzoate de méthyle.
   De manière analogue à l'exemple 1(b), à partir de 43,80 g (166,0 mmoles) de l'acide obtenu à l'exemple 42(a), on obtient 43,54 g (94%) de 3-hydroxy-4-iodo-benzoate de méthyle, sous la forme d'une poudre beige de point de fusion 153°C.
   ¹H NMR (CDCl₃) δ 3,89 (s, 3H), 7,25 (dd, 1H, *J* = 8,2 / 1,9 Hz), 7,58 (d, 1H, *J* = 1,9 Hz), 7,77 (d, 1H, *J* = 8,2 Hz), 8,79 (br s, 1H).
(c) 2"-hydroxy-2'-(5,5,8,8-tétraméthyl-5,6,7,8-tétrahydro-naphthalèn-2-yl)-[1,1';4',1'']terphényl-4"-carboxylate de méthyle.
   De manière analogue à l'exemple 1(d), par réaction de 2,80 g (7,3 mmoles) de l'acide boronique obtenu à l'exemple 41(g) avec 1,84 g (6,6 mmoles) du composé obtenu à l'exemple 42(b), on obtient 2,00 g (62%) du produit attendu, sous la forme d'un solide blanc de point de fusion 183-185°C.
   ¹H NMR (CDCl₃) δ 0,89 (s, 6H), 1,26 (s, 6H), 1,56 à 1,64 (m, 4H), 3,94 (s, 3H), 5,51 (s, 1H), 6,89 (d, 1H, *J* = 1,9 Hz), 7,18 à 7,26 (m, 7H), 7,42 (d, 1H, *J* = 8,3 Hz), 7,53 à 7,55 (m, 2H), 7,59 à 7,60 (m, 1H), 7,68 à 7,71 (m, 2H).
(d) acide 2"-hydroxy-2'-(5,5,8,8-tétraméthyl-5,6,7,8-tétrahydro-naphthalèn-2-yl)-[1,1';4',1"]terphényl-4"-carboxylique.
   De manière analogue à l'exemple 1(e), à partir de 500 mg (1,0 mmole) de l'ester obtenu à l'exemple 42(c), on obtient 480 mg (99%) d'acide 2''-hydroxy-2'-(5,5,8,8-tétraméthyl-5,6,7,8-tétrahydro-naphthalèn-2-yl)-[1,1';4',1'']terphényl-4''-carboxylique, sous la forme d'un solide blanc cristallisé de point de fusion 282-284°C.
   ¹H NMR (CDCl₃ + 2 gouttes de DMSO D₆) δ 0,90 (s, 6H), 1,25 (s, 6H), 1,55 à 1,63 (m, 4H), 6,88 (d, 1H, *J* = 1,5 Hz), 7,12 à 7,25 (m, 7H), 7,43 (d, 1H, *J* = 8,1 Hz), 7,47 (d, 1H, *J* = 8,7 Hz), 7,61 (s, 1H), 7,65 à 7,67 (m, 1H), 7,71 (d, 2H, *J* = 7,6 Hz).

### EXEMPLE 43

### Acide 2"-méthoxyméthoxy-2'-(5,5,8,8-tétraméthyl-5,6,7,8-tétrahydro-naphthalèn-2-yl)-[1,1';4',1'']terphényl-4''-carboxylique.

(a) 2"-méthoxyméthoxy-2'-(5,5,8,8-tétraméthyl-5,6,7,8-tétrahydro-naphthalèn-2-yl)-[1,1';4',1"]terphényl-4"-carboxylate de méthyle.
   De manière analogue à l'exemple 7(a), par réaction de 580 mg (1,2 mmole) du composé obtenu à l'exemple 42(c) avec 103 µl (1,3 mmole) d'éther méthylique de chlorométhyle, on obtient 630 mg (100%) du produit attendu, sous la forme d'une huile orange.
   ¹H NMR (CDCl₃) δ 0,91 (s, 6H), 1,26 (s, 6H), 1,56 à 1,63 (m, 4H), 3,48 (s, 3H), 3,94 (s, 3H), 5,26 (s, 2H), 6,91 (d, 1H, *J* = 1,8 Hz), 7,10 (dd, 1H, *J* = 7,9 / 1,9 Hz), 7,19 à 7,25 (m, 6H), 7,46 à 7,51 (m, 2H), 7,61 (dd, 1H, *J* = 7,8 / 1,7 Hz), 7,65 (d, 1H, *J* = 1,7 Hz), 7,79 (dd, 1H, *J* = 7, 9 / 1,8 Hz), 7,89 (d, 1H, *J* = 1,5 Hz).
(b) acide 2"-méthoxyméthoxy-2'-(5,5,8,8-tétraméthyl-5,6,7,8-tétrahydro-naphthalèn-2-yl)-[1,1';4',1"]terphényl-4"-carboxylique.
   De manière analogue à l'exemple 1(e), à partir de 620 mg (1,2 mmole) de l'ester obtenu à l'exemple 43(a), on obtient 556 mg (92%) d'acide 2"-méthoxyméthoxy-2'-(5,5,8,8-tétraméthyl-5,6,7,8-tétrahydro-naphthalèn-2-yl)-[1,1';4',1"]terphényl-4"-carboxylique, sous la forme d'un solide blanc cristallisé de point de fusion 204-206°C.
   ¹H NMR (CDCl₃) δ 0,92 (s, 6H), 1,26 (s, 6H), 1,56 à 1,64 (m, 4H), 3,49 (s, 3H), 5,28 (s, 2H), 6,92 (d, 1H, *J* = 1,7 Hz), 7,12 (dd, 1H, *J* = 7,9 / 1,8 Hz), 7,18 à 7,26 (m, 6H), 7,49 (d, 1H, *J* = 7,9 Hz), 7,54 (d, 1H, *J* = 8,0 Hz), 7,62 (dd, 1H, *J* = 8,0 / 1,8 Hz), 7,67 (d, 1H, *J* = 1,6 Hz), 7,88 (dd, 1H, *J* = 7,9 / 1,8 Hz), 7,99 (d, 1H, *J* = 1,4 Hz).

### EXEMPLE 44

### Acide 2"-méthoxy-2'-(5,5,8,8-tétraméthyl-5,6,7,8-tétrahydro-naphthalèn-2-yl)-[1,1';4',1"]terphényl-4"-carboxylique

(a) 2"-méthoxy-2'-(5,5,8,8-tétraméthyl-5,6,7,8-tétrahydro-naphthalèn-2-yl)-[1,1';4',1"]terphényl-4"-carboxylate de méthyle.
   De manière analogue à l'exemple 7(a), par réaction de 500 mg (1,0 mmole) du composé obtenu à l'exemple 42(c) avec 70 µl (1,1 mmole) d'iodure de méthyle, on obtient 510 mg (99%) du produit attendu, sous la forme d'une poudre beige clair de point de fusion 144-146°C.
   ¹H NMR (CDCl₃) δ 0,90 (s, 6H), 1,26 (s, 6H), 1,56 à 1,63 (m, 4H), 3,93 (s, 3H), 3,95 (s, 3H), 6,90 (d, 1H, *J* = 1,8 Hz), 7,14 à 7,26 (m, 7H), 7,46 (s, 1H), 7,49 (s, 1H), 7,61 (dd, 1H, *J* = 7,9 / 1,8 Hz), 7,66 (d, 1H, *J* = 8,0 Hz), 7,67 (d, 1H, *J* = 1,3 Hz), 7,73 (d, 1H, *J* = 7,8 Hz).
(b) acide 2"-méthoxy-2'-(5,5,8,8-tétraméthyl-5,6,7,8-tétrahydro-naphthalèn-2-yl)-[1,1',4',1'']terphényl-4''-carboxylique.
   De manière analogue à l'exemple 1(e), à partir de 500 mg (1,0 mmole) de l'ester obtenu à l'exemple 44(a), on obtient 420 mg (86%) d'acide 2''-méthoxy-2'-(5,5,8,8-tétraméthyl-5,6,7,8-tétrahydro-naphthalèn-2-yl)-[1,1';4',1'']terphényl-4''-carboxylique, sous la forme d'un solide blanc cristallisé de point de fusion 272-274°C.
   ¹H NMR (CDCl₃ + 2 gouttes de DMSO D₆) δ 0,91 (s, 6H), 1,26 (s, 6H), 1,55 à 1,63 (m, 4H), 3,92 (s, 3H), 6,90 (d, 1H, *J* = 1,8 Hz), 7,13 à 7,25 (m, 7H), 7,45 (d, 1H, *J* = 1,4 Hz), 7,49 (d, 1H, *J* = 1,3 Hz), 7,62 (dd, 1H, *J* = 7,9 / 1,8 Hz), 7,65 (d, 1H, *J* = 1,6 Hz), 7,71 (s, 1H), 7,75 (d, 1H, *J* = 8,1 Hz).

### EXEMPLE 45

### Acide 2"-propyloxy-2'-(5,5,8,8-tétraméthyl-5,6,7,8-tétrahydro-naphthalèn-2-yl)-[1,1';4',1"]terphényl-4"-carboxylique

(a) 2"-propyloxy-2'-(5,5,8,8-tétraméthyl-5,6,7,8-tétrahydro-naphthalèn-2-yl)-[1,1';4',1'']terphényl-4''-carboxylate de méthyle.
   De manière analogue à l'exemple 7(a), par réaction de 500 mg (1,0 mmole) du composé obtenu à l'exemple 42(c) avec 110 µl (1,1 mmole) d'iodure de propyle, on obtient 530 mg (98%) du produit attendu, sous la forme d'une huile marron.
   ¹H NMR (CDCl₃) δ 0,91 (s, 6H), 1,03 (t, 3H, *J* = 7,5 Hz), 1,25 (s, 6H), 1,55 à 1,63 (m, 4H), 1,86 (sext, 2H, *J* = 6,8 Hz), 3,94 (s, 3H), 4,06 (t, 2H, *J* = 6,5 Hz), 6,90 (d, 1H, *J* = 1,8 Hz), 7,12 (dd, 1H, *J* = 8,0 / 1,8 Hz), 7,17 à 7,26 (m, 6H), 7,47 (d, 1H, *J* = 7,9 Hz), 7,50 (d, 1H, *J* = 7,9 Hz), 7,64 à 7,69 (m, 4H).
(b) acide 2''-propyloxy-2'-(5,5,8,8-tétraméthyl-5,6,7,8-tétrahydro-naphthalèn-2-yl)-[1,1';4',1'']terphényl-4''-carboxylique.
   De manière analogue à l'exemple 1(e), à partir de 520 mg (1,0 mmole) de l'ester obtenu à l'exemple 45(a), on obtient 385 mg (77%) d'acide 2"-propyloxy-2'-(5,5,8,8-tétraméthyl-5,6,7,8-tétrahydro-naphthalèn-2-yl)-[1,1';4',1"]terphényl-4"-carboxylique, sous la forme d'un solide blanc cristallisé de point de fusion 216-218°C.
   ¹H NMR (CDCl₃) δ 0,92 (s, 6H), 1,04 (t, 3H, *J* = 7,5 Hz), 1,26 (s, 6H), 1,56 à 1,64 (m, 4H), 1,87 (sext, 2H, *J* = 6,9 Hz), 4,08 (t, 2H, *J* = 6,5 Hz), 6,92 (d, 1H, *J* = 1,8 Hz), 7,14 à 7,25 (m, 7H), 7,48 (d, 1H, *J* = 7,9 Hz), 7,55 (d, 1H, *J* = 7,9 Hz), 7,66 à 7,74 (m, 3H), 7,82 (d, 1H, *J* = 8,0 Hz).

### EXEMPLE 46

### Acide 3"-hydroxy-2'-(5,5,8,8-tétraméthyl-5,6,7,8-tétrahydro-naphthalèn-2-yl)-[1,1';4',1"]terphényl-4"-carboxylique

(a) 3"-hydroxy-2'-(5,5,8,8-tétraméthyl-5,6,7,8-tétrahydro-naphthalèn-2-yl)-[1,1';4',1"]terphényl-4"-carboxylate de méthyle.
   De manière analogue à l'exemple 1(d), par réaction de 700 mg (1,8 mmole) de l'acide boronique obtenu à l'exemple 41(g) avec 420 mg (1,5 mmole) de 4-iodosalicylate de méthyle, on obtient 550 mg (75%) du produit attendu, sous la forme de cristaux jaunes de point de fusion 134-136°C.
   ¹H NMR (CDCl₃) δ 0,91 (s, 6H), 1,27 (s, 6H), 1,55 à 1,65 (m, 4H), 3,98 (s, 3H), 6,88 (d, 1H, *J* = 1,9 Hz), 7,14 à 7,31 (m, 9H), 7,51 (d, 1H, *J* = 7,9 Hz), 7,65 (dd, 1H, *J* = 7,9 / 2,0 Hz), 7,72 (d, 1H, *J* = 1,9 Hz), 7,91 (d, 1H, *J* = 8,0 Hz), 10,82 (s, 1H).
(b) acide 3"-hydroxy-2'-(5,5,8,8-tétraméthyl-5,6,7,8-tétrahydro-naphthalèn-2-yl)-[1,1';4',1"]terphényl-4"-carboxylique.
   De manière analogue à l'exemple 1(e), à partir de 550 mg (1,1 mmole) de l'ester obtenu à l'exemple 46(a), on obtient 277 mg (52%) d'acide 3"-hydroxy-2'-(5,5,8,8-tétraméthyl-5,6,7,8-tétrahydro-naphthalèn-2-yl)-[1,1';4',1"]terphényl-4"-carboxylique, sous la forme d'un solide blanc cristallisé de point de fusion 266-268°C.
   ¹H NMR (CDCl₃) δ 0,91 (s, 6H), 1,27 (s, 6H), 1,56 à 1,64 (m, 4H), 6,89 (d, 1H, *J* = 1,7 Hz), 7,14 à 7,25 (m, 9H), 7,28 (d, 1H, *J* = 2,9 Hz), 7,50 (d, 1H, *J* = 8,0 Hz), 7,65 (dd, 1H, *J* = 8,0 / 1,8 Hz), 7,72 (d, 1H, *J* = 1,7 Hz), 7,95 (d, 1H, *J* = 8,2 Hz).

### EXEMPLE 47

### Acide 6-[2-(5,5,8,8-tétraméthyl-5,6,7,8-tétrahydro-naphthalèn-2-yl)-biphényl-4-yl]-nicotinique.

(a) 6-[2-(5,5,8,8-tétraméthyl-5,6,7,8-tétrahydro-naphthalèn-2-yl)-biphényl-4-yl]-nicotinate d'éthyle.
   De manière analogue à l'exemple 1(d), par réaction de 700 mg (1,8 mmole) de l'acide boronique obtenu à l'exemple 41(g) avec 460 mg (1,7 mmole) de 6-iodonicotinate d'éthyle, on obtient 650 mg (80%) du produit attendu, sous la forme d'un solide blanc de point de fusion 105-107°C.
   ¹H NMR (CDCl₃) δ 0,92 (s, 6H), 1,28 (s, 6H), 1,45 (t, 3H, *J* = 7,1 Hz), 1,57 à 1,65 (m, 4H), 4,44 (q, 2H, *J* = 7,1 Hz), 6,89 (d, 1H, *J* = 1,8 Hz), 7,18 à 7,30 (m, 6H), 7,57 (d, 1H, *J* = 7,9 Hz), 7,89 (d, 1H, *J* = 8,3 Hz), 8,10 à 8,15 (m, 2H), 8,36 (dd, 1H, *J* = 8,3 / 2,2 Hz), 9,31 (d, 1H, *J* = 2,1 Hz).
(b) acide 6-[2-(5,5,8,8-tétraméthyl-5,6,7,8-tétrahydro-naphthalèn-2-yl)-biphényl-4-yl]-nicotinique.
   De manière analogue à l'exemple 1(e), à partir de 650 mg (1,3 mmole) de l'ester obtenu à l'exemple 47(a), on obtient 490 mg (80%) d'acide 6-[2-(5,5,8,8-tétraméthyl-5,6,7,8-tétrahydro-naphthalèn-2-yl)-biphényl-4-yl]-nicotinique, sous la forme d'un solide blanc cristallisé en fine aiguilles, de point de fusion 319-321°C.
   ¹H NMR (CDCl₃) δ 1,04 (s, 6H), 1,26 (s, 6H), 1,57 à 1,63 (m, 4H), 7,21 à 7,41 (m, 7H), 7,58 (s, 1H), 7,65 (d, 1H, *J* = 8,1 Hz), 8,15 (d, 1H, *J* = 8,3 Hz), 8,41 (dd, 1H, *J* = 8,1 / 1,9 Hz), 8,59 (d, 1H, *J* = 1,8 Hz), 8,69 (d, 1H, *J* = 2,2 Hz), 9,82 (d, 1H, *J* = 1,9 Hz).

### EXEMPLE 48

### Acide 5-[2-(5,5,8,8-tétraméthyl-5,6,7,8-tétrahydro-naphthalèn-2-yl)-biphényl-4-yl]-pyridine-2-carboxylique.

(a) 5-[2-(5,5,8,8-tétraméthyl-5,6,7,8-tétrahydro-naphthalèn-2-yl)-biphényl-4-yl]-pyridine-2-carboxylate de méthyle.
   De manière analogue à l'exemple 1(d), par réaction de 700 mg (1,8 mmole) de l'acide boronique obtenu à l'exemple 41(g) avec 430 mg (1,7 mmole) de 5-iodopyridine-2-carboxylate de méthyle, on obtient 600 mg (77%) du produit attendu, sous la forme d'un solide blanc de point de fusion 160-162°C.
   ¹H NMR (CDCl₃) δ 0,91 (s, 6H), 1,27 (s, 6H), 1,55 à 1,65 (m, 4H), 4,05 (s, 3H), 6,90 (d, 1H, *J* = 1,8 Hz), 7,16 à 7,30 (m, 7H), 7,57 (d, 1H, *J* = 7,9 Hz), 7,67 (dd, 1H, *J* = 8,0 / 1,9 Hz), 7,72 (d, 1H, *J* = 1,9 Hz), 8,10 (dd, 1H, *J* = 8,2 / 2,2 Hz), 8,24 (d, 1H, *J* = 8,2 Hz), 9,06 (d, 1H, *J* = 2,1 Hz).
(b) acide 5-[2-(5,5,8,8-tétraméthyl-5,6,7,8-tétrahydro-naphthalèn-2-yl)-biphényl-4-yl]-pyridine-2-carboxylique.
   De manière analogue à l'exemple 1(e), à partir de 600 mg (1,3 mmole) de l'ester obtenu à l'exemple 48(a), on obtient 490 mg (84%) d'acide 5-[2-(5,5,8,8-tétraméthyl-5,6,7,8-tétrahydro-naphthalèn-2-yl)-biphényl-4-yl]-pyridine-2-carboxylique, sous la forme d'une poudre beige de point de fusion 222-224°C.
   ¹H NMR (CDCl₃) δ 0,92 (s, 6H), 1,27 (s, 6H), 1,57 à 1,65 (m, 4H), 6,89 (d, 1H, *J* = 1,6 Hz), 7,13 à 7,30 (m, 7H), 7,58 (d, 1H, *J* = 7,9 Hz), 7,68 (dd, 1H, *J* = 8,0 / 1,5 Hz), 7,73 (s, 1H),

### EXEMPLE 49

### Acide 2'-(5,5,8,8-tétraméthyl-5,6,7,8-tétrahydro-naphthalèn-2-yl)-[1,1';4',1"]terphényl-4"-hydroxamique.

Dans un tricol et sous courant d'azote, on introduit successivement 2,00 g (4,3 mmoles) de l'acide obtenu à l'exemple 14, 30 ml d'éthanol et 290 mg (5,2 mmoles) de potasse en poudre. Le milieu réactionnel est agité pendant trente minutes à la température ambiante, puis on évapore à sec. On reprend le résidu par 80 ml de dichlorométhane et ajoute 673 mg (4,8 mmoles) de O-(triméthylsilyl)hydroxylamine ainsi que 645 mg (4,8 mmoles) de 1-hydroxybenzotriazole (HOBT). Après refroidissement du milieu réactionnel à 0°C, on ajoute 915 mg (4,8 mmoles) de chlorhydrate de 1-(3-diméthylaminopropyl)-3-éthylcarbodiimide (EDCI), agite la solution obtenue pendant une heure à 0°C, puis pendant seize heures à la température ambiante. Le milieu réactionnel est versé sur un mélange eau / dichlorométhane, extrait par du dichlorométhane, la phase organique est lavée à l'eau jusqu'à pH neutre, séchée sur sulfate de magnésium et évaporée. Le résidu obtenu est purifié par chromatographie sur colonne de silice, élué par un mélange composé de 20% d'acétate d'éthyle et de 80% d'heptane. Après évaporation des solvants, on recueille 530 mg (22%) du produit attendu, sous la forme d'un solide beige de point de fusion 105-108°C.
¹H NMR (CDCl₃) δ 0,90 (s, 6H), 1,26 (s, 6H), 1,55 à 1,63 (m, 6H), 4,70 à 5,20 (m, 2H), 6,88 (s, 1H), 7,12 à 7,27 (m, 7H), 7,49 (d, 1H, *J* = 7,9 Hz), 7,60 (d, 1H, *J* = 8,0 Hz), 7,70 (d, 1H, *J* = 1,3 Hz), 7,73 (d, 2H, *J* = 8,2 Hz), 7,84 (d, 2H, *J* = 8,1 Hz).

### EXEMPLE 50

### 2'-(5,5,8,8-tétraméthyl-5,6,7,8-tétrahydro-naphthalèn-2-yl)-[1,1';4',1'']terphényl-4''-ol.

De manière analogue à l'exemple 1(d), par réaction de 700 mg (1,8 mmole) de l'acide boronique obtenu à l'exemple 41(g) avec 287 mg (1,7 mmole) de 4-bromophénol, on obtient 560 mg (89%) de 2'-(5,5,8,8-tétraméthyl-5,6,7,8-tétrahydro-naphthalèn-2-yl)-[1,1';4',1'']terphényl-4''-ol, sous la forme d'une huile incolore.
¹H NMR (CDCl₃) δ 0,91 (s, 6H), 1,26 (s, 6H), 1,56 à 1,64 (m, 4H), 4,88 (s, 1H), 6,90 à 6,94 (m, 3H), 7,14 à 7,22 (m, 7H), 7,47 (d, 1H, *J* = 7,9 Hz), 7,57 (d, 2H, *J* = 8,0 Hz), 7,57 à 7,59 (m, 1H), 7,65 (d, 1H, *J* = 1,9 Hz).

### EXEMPLE 51

### [2'-(5,5,8,8-tétraméthyl-5,6,7,8-tétrahydro-naphthalèn-2-yl)-[1,1';4',1'']terphényl-4''-yl]-méthanol.

Dans un tricol de deux litres et sous courant d'azote, on introduit 1,80 g (3,7 mmoles) de l'ester obtenu à l'exemple 13(b) et 30 ml de toluène. La solution obtenue est refroidie à-78°C, et on coule goutte à goutte 14,7 ml (14,7 mmoles) d'une solution (1M dans le toluène) d'hydrure de diisobutylaluminium. Le milieu réactionnel est agité pendant une heure à -78°C, hydrolysé à l'aide d'acide chlorhydrique 1N, et filtré. La phase organique est lavée à l'eau jusqu'à pH neutre, séchée sur sulfate de magnésium, filtrée, et les solvants évaporés. Après évaporation des solvants, on recueille 1,31 g (79%) de [2'-(5,5,8,8-tétraméthyl-5,6,7,8-tétrahydro-naphthalèn-2-yl)-[1,1':4',1"]terphényl-4"-yl]-méthanol, sous la forme d'un solide orangé de point de fusion 134-136°C.
¹H NMR (CDCl₃) δ 0,91 (s, 6H), 1,27 (s, 6H), 1,57 à 1,64 (m, 4H), 1,72 (br s, 1H), 4,75 (d, 2H, *J* = 3,4 Hz), 6,90 (d, 1H, *J* = 1,9 Hz), 7,14 à 7,28 (m, 7H), 7,44 à 7,51 (m, 3H), 7,63 (dd, 1H, *J* = 8,0 / 1,9 Hz), 7,67 à 7,71 (m, 3H).

### EXEMPLE 52

### 2'-(5,5,8,8-tétraméthyl-5,6,7,8-tétrahydro-naphthalén-2-yl)-[1,1';4',1"]terphényl-4"-carbaldéhyde.

Dans un ballon de 500 ml, on mélange 640 mg (1,4 mmole) de l'alcool obtenu à l'exemple 51, 2,50 g (28,7 mmoles) d'oxyde de manganèse et 50 ml de dichlorométhane. Le milieu réactionnél est agité pendant vingt heures à la température ambiante, puis on filtre l'oxyde de manganèse et évapore le dichlorométhane. Le résidu obtenu est purifié par chromatographie sur colonne de silice, élué par un mélange composé de 80% d'heptane et de 20% d'acétate d'éthyle. Après évaporation des solvants, on recueille 90 mg (14%) du composé attendu, sous la forme d'une poudre blanche de point de fusion 120-122°C.
¹H NMR (CDCl₃) δ 0,91 (s, 6H), 1,27 (s, 6H), 1,56 à 1,64 (m, 4H), 6,90 (d, 1H, *J* = 1,8 Hz), 7,14 à 7,29 (m, 7H), 7,54 (d, 1H, *J* = 8,0 Hz), 7,66 (dd, 1H, *J* = 7,9 / 2,0 Hz), 7,74 (d, 1H, *J* = 1,9 Hz), 7,84 (d, 2H, *J* = 8,3 Hz), 7,97 (d, 2H, *J* = 8,3 Hz), 10,07 (s, 1H).

### EXEMPLE 53

### Acide 4'-méthoxycarbonylméthoxy-3'-(5,5,8,8-tétraméthyl-5,6,7,8-tétrahydro-naphthalèn-2-yl)-biphényl-4-carboxylique.

(a) 4-[4-hydroxy-3-(5,6,7,8-tétrahydro-5,5,8,8-tétraméthyl-2-naphtyl)phényl] benzoate de benzyle.
   Dans un ballon et sous courant d'azote, on introduit 6,00 g (15,0 mmoles) du composé obtenu à l'exemple 1(e) et 140 ml de DMF. On refroidit à 0°C, ajoute par petites quantités 502 mg (15,7 mmoles) d'hydrure de sodium (80% dans l'huile), et agite jusqu'à cessation du dégagement gazeux. On ajoute ensuite 1,87 ml (15,7 mmoles) de bromure de benzyle et agite pendant une heure à 0°C, puis seize heures à la température ambiante. On verse le milieu réactionnel dans un mélange HCl 2N / acétate d'éthyle, extrait par de l'acétate d'éthyle, décante la phase organique, sèche sur sulfate de magnésium, évapore. Le résidu obtenu est purifié par chromatographie sur colonne de silice, élué par un mélange composé de 20% d'acétate d'éthyle et de 80% d'heptane. Après évaporation des solvants, on recueille 5,21 g (71%) du produit attendu, sous la forme d'un solide jaune cristallisé de point de fusion 90-91°C.
   ¹H NMR (CDCl₃) δ 1,34 (s, 6H), 1,36 (s, 6H), 1,76 (s, 4H), 5,40 (s, 2H), 5,47 (s, 1H), 7,11 (d, 1H, *J* = 8,8 Hz), 7,27 à 7,30 (m, 1H), 7,38 à 7,56 (m, 9H), 7,66 (d, 2H, *J* = 8,4 Hz), 8,14 (d, 2H, *J* = 8,4 Hz).
(b) 4'-méthoxycarbonylméthoxy-3'-(5,5,8,8-tétraméthyl-5,6,7,8-tétrahydro-naphthalèn-2-yl)-biphényl-4-carboxylate de benzyle.
   De manière analogue à l'exemple 2(a), par réaction de 1,20 g (2,44 mmoles) du composé obtenu à l'exemple 53(a) avec 280 µl (2,9 mmoles) de bromoacétate de méthyle, on obtient 950 mg (70%) du produit attendu, sous la forme d'un solide blanc de point de fusion 104-106°C.
   ¹H NMR (CDCl₃) δ 1,27 (s, 6H), 1,33 (s, 6H), 1,72 (s, 4H), 3,80 (s, 3H), 4,67 (s, 2H), 5,38 (s, 2H), 6,95 (d, 1H, *J* = 8,5 Hz), 7,37 à 7,54 (m, 8H), 7,60 à 7,62 (m, 2H), 7,64 (d, 2H, *J* = 8,5 Hz), 8,12 (d, 2H, *J* = 8,5 Hz).
(c) acide 4'-méthoxycarbonylméthoxy-3'-(5,5,8,8-tétraméthyl-5,6,7,8-tétrahydro-naphthalèn-2-yl)-biphényl-4-carboxylique.
   Dans un tricol et sous courant d'argon, on introduit 300 mg (0,53 mmole) du composé obtenu à l'exemple 53(b), 20 ml de méthanol et 10 ml de THF. On dégaze le milieu à l'argon et introduit 60,0 mg de palladium sur charbon à 15%, purge à l'hydrogène et agite le milieu réactionnel sous atmosphère d'hydrogène (légère surpression), pendant 22 heures. On filtre le catalyseur sur Celite® , évapore les solvants, cristallise le produit obtenu dans un mélange composé de 10% d'éther éthylique et de 90% d'heptane, et recueille 142 mg (57%) du produit attendu, sous la forme d'un solide blanc cristallisé de point de fusion 234-238°C.
   ¹H NMR (CDCl₃) δ 1,33 (s, 12H), 1,72 (s, 4H), 3,80 (s, 3H), 4,68 (s, 2H), 6,95 (d, 1H, *J* = 8,5 Hz), 7,33 à 7,40 (m, 2H), 7,53 (dd, 1H, *J* = 8,5 / 2,3 Hz), 7,60 à 7,66 (m, 4H), 8,11 (br d, 2H, *J* = 7,8 Hz).

### EXEMPLE 54

### Acide 4'-carboxyméthoxy-3'-(5,5,8,8-tétraméthyl-5,6,7,8-tétrahydro-naphthalèn-2-yl)-biphényl-4-carboxylique.

De manière analogue à l'exemple 1(e), à partir de 650 mg (1,2 mmole) du diester obtenu à l'exemple 53(b), on obtient 470 mg (88%) d'acide 4'-carboxyméthoxy-3'-(5,5,8,8-tetraméthyl-5,6,7,8-tétrahydro-naphthalèn-2-yl)-biphényl-4-carboxylique, sous la forme d'un solide blanc cristallisé de point de fusion 279-281°C.
¹H NMR (CDCl₃ + 2 gouttes de DMSO D₆) δ 1,33 (s, 12H), 1,72 (s, 4H), 4,65 (s, 2H), 6,99 (d, 1H, *J* = 8,6 Hz), 7,38 (d, 1H, *J* = 7,3 Hz), 7,41 (d, 1H, *J* = 8,2 Hz), 7,53 (dd, 1H, *J* = 8,5 / 2,4 Hz), 7,60 à 7,62 (m, 2H), 7,64 (d, 2H, *J* = 8,4 Hz), 8,09 (d, 2H, *J* = 8,4 Hz).

### EXEMPLE 55

### Acide 4'-(5-éthoxycarbonyl-pentyloxy)-3'-(5,5,8,8-tétraméthyl-5,6,7,8-tétrahydro-naphthalèn-2-yl)-biphényl-4-carboxylique.

(a) 4'-(5-éthoxycarbonyl-pentyloxy)-3'-(5,5,8,8-tétraméthyl-5,6,7,8-tétrahydro-naphthalèn-2-yl)-biphényl-4-carboxylate de benzyle.
   De manière analogue à l'exemple 2(a), par réaction de 1,20 g (2,4 mmoles) du composé obtenu à l'exemple 53(a). avec 520 µl (2,9 mmoles) de 6-bromohexanoate d'éthyle, on obtient 1,52 g (100%) du produit attendu, sous la forme d'une huile jaune.
   ¹H NMR (CDCl₃) δ 1,24 (t, 3H, *J* = 7,1 Hz), 1,32 (s, 6H), 1,33 (s, 6H), 1,42 à 1,49 (m, 2H), 1,64 (quint, 2H, *J* = 8, 0 Hz), 1,72 (s, 4H), 1,78 (quint, 2H, *J* = 7,1 Hz), 2,27 (t, 2H, *J* = 7,6 Hz), 4,02 (t, 2H, *J* = 6,5 Hz), 4,11 (q, 2H, *J* = 7,1 Hz), 5,38 (s, 2H), 7,03 (d, 1H, *J* = 8,6 Hz), 7,32 à 7,57 (m, 8H), 7,57 (d, 1H, *J* = 1,5 Hz), 7,61 (d, 1H, *J* = 2,4 Hz), 7,65 (d, 2H, *J* = 8,4 Hz), 8,12 (d, 2H, *J* = 8,5 Hz).
(b) acide 4'-(5-éthoxycarbonyl-pentyloxy)-3'-(5,5,8,8-tétraméthyl-5,6,7,8-tétrahydro-naphthalèn-2-yl)-biphényl-4-carboxylique.
   De manière analogue à l'exemple 53(c), à partir de 620 mg (1,0 mmole) du diester obtenu à l'exemple 55(a), on obtient 420 mg (80%) d'acide 4'-(5-éthoxycarbonyl-pentyloxy)-3'-(5,5,8,8-tétraméthyl-5,6,7,8-tétrahydro-naphthalèn-2-yl)-biphényl-4-carboxylique, sous la forme d'un solide blanc cristallisé de point de fusion 177°C.
   ¹H NMR (CDCl₃) δ 1,25 (t, 3H, *J* = 7,2 Hz), 1,32 (s, 6H), 1,33 (s, 6H), 1,41 à 1,49 (m, 2H), 1,59 à 1,68 (m, 2H), 1,73 (s, 4H), 1,78 à 1,83 (m, 2H), 2,27 (t, 2H, *J* = 7,6 Hz), 4,03 (t, 2H, *J* = 6,5 Hz), 4,12 (q, 2H, *J* = 7,1 Hz), 7,04 (d, 1H, *J* = 8,6 Hz), 7,30 à 7,38 (m, 2H), 7,53 (d, 1H, *J* = 2,1 Hz), 7,58 (d, 1H, *J* = 1,4 Hz), 7,63 (d, 1H, *J* = 2,3 Hz), 7,69 (d, 2H, *J* = 7,9 Hz), 8,16 (br d, 2H, *J* = 6,7 Hz).

### EXEMPLE 56

### Acide 4'-(5-carboxy-pentyloxy)-3'-(5,5,8,8-tétraméthyl-5,6,7,8-tétrahydro-naphthalén-2-yl)-biphényl-4-carboxylique.

De manière analogue à l'exemple 1(e), à partir de 750 mg (1,2 mmole) du diester obtenu à l'exemple 55(a), on obtient 610 mg (100%) d'acide 4'-(5-carboxy-pentyloxy)-3'-(5,5,8,8-tétraméthyl-5,6,7,8-tétrahydro-naphthalèn-2-yl)-biphényl-4-carboxylique, sous la forme d'un solide blanc cristallisé de point de fusion 245°C.
¹H NMR (DMSO D₆) δ 1,28 (s, 12H), 1,36 à 1,44 (m, 2H), 1,46 à 1,55 (m, 2H), 1,68 (s, 4H), 1,69 à 1,73 (m, 2H), 2,18 (t, 2H, *J* = 7,0 Hz), 4,04 (t, 2H, *J* = 6,0 Hz), 7,20 (d, 1H, *J* = 8,6 Hz), 7,30 (dd, 1H, *J* = 8,0 / 1,2 Hz), 7,37 (d, 1H, *J* = 8,2 Hz), 7,56 (d, 1H, *J* = 1,1 Hz), 7,61 (d, 1H, *J* = 2,2 Hz), 7,66 (dd, 1H, *J* = 8,6 / 2,1 Hz), 7,81 (d, 2H, *J* = 8,4 Hz), 7,99 (d, 2H, *J* = 8,3 Hz).

### EXEMPLE 57

### 2'-(5,5,8,8-tétraméthyl-5,6,7,8-tétrahydro-naphthalèn-2-yl)-[1,1';4',1"]terphényl-4"-carboxamide.

(a) chlorure de 2'-(5,5,8,8-tétraméthyl-5,6,7,8-tétrahydro-naphthalèn-2-yl)-[1,1';4',1"]terphényl-4"-carbonyle.
   Dans un tricol et sous courant d'azote, on introduit 6,00 g (12,9 mmoles) de l'acide obtenu à l'exemple 14 et 240 ml de dichlorométhane. On coule goutte à goutte 2,63 ml (13,5 mmoles) de dicyclohexylamine et agite la solution obtenue pendant dix minutes à la température ambiante. On coule goutte à goutte 984 µl (13,5 mmoles) de chlorure de thionyle et agite la solution obtenue pendant quinze minutes à la température ambiante. Le milieu réactionnel est évaporé à sec, repris par de l'éther éthylique, filtré et le filtrat évaporé à sec. Le chlorure d'acide ainsi obtenu est utilisé directement pour l'étape suivante.
(b) 2'-(5,5,8,8-tétraméthyl-5,6,7,8-tétrahydro-naphthalèn-2-yl)-[1,1';4',1'']terphényl-4''-carboxamide.
   1,03 g (2,1 mmoles) du chlorure d'acide obtenu à l'étape précédente est solubilisé dans 100 ml de THF. La solution ainsi obtenue est coulée goutte à goutte sur une solution composée de 2,6 ml (43,0 mmoles) d'une solution aqueuse d'ammoniaque à 32% et 20 ml de THF. Le milieu réactionnel est agité pendant une heure à la température ambiante, versé dans l'eau et extrait à l'éther éthylique. La phase organique est lavée à l'eau jusqu'à pH neutre, séchée sur sulfate de magnésium, filtrée, et les solvants évaporés. Le résidu obtenu est trituré dans l'heptane, filtré et séché. On recueille 940 mg (95%) de 2'-(5,5,8,8-tétraméthyl-5,6,7,8-tétrahydro-naphthalèn-2-yl)-[1,1';4',1'']terphényl-4''-carboxamide, sous la forme d'une poudre beige de point de fusion 220°C.
   ¹H NMR (CDCl₃) δ 0,91 (s, 6H), 1,26 (s, 6H), 1,56 à 1,64 (m, 4H), 6,20 (br s, 2H), 6,89 (d, 1H, *J* = 1,3 Hz), 7,14 à 7,29 (m, 7H), 7,51 (d, 1H, *J* = 7,9 Hz), 7,64 (dd, 1H, *J* = 7,9 / 1,5 Hz), 7,72 (s, 1H), 7,74 (d, 2H, *J* = 8,2 Hz), 7,91 (d, 2H, *J* = 8,2 Hz).

### EXEMPLE 58

### N-éthyl-2'-(5,5,8,8-tétraméthyl-5,6,7,8-tétrahydro-naphthalèn-2-yl)-[1,1';4',1'']terphényl-4''-carboxamide.

De manière analogue à l'exemple 57(b), à partir de 1,30 g (2,7 mmoles) du chlorure d'acide obtenu à l'exemple 57(a) et 4,4 ml (54,3 mmoles) d'une solution aqueuse d'éthylamine à 70%, on obtient 1,20 g (91%) de N-éthyl-2'-(5,5,8,8-tétraméthyl-5,6,7,8-tétrahydro-naphthalèn-2-yl)-[1,1';4',1'']terphényl-4''-carboxamide, sous la forme d'une poudre beige de point de fusion 183°C.
¹H NMR (CDCl₃) δ 0,91 (s, 6H), 1,27 (s, 6H), 1,28 (t, 3H, *J* = 5,7 Hz), 1,56 à 1,63 (m, 4H), 3,53 (q, 2H, *J* = 5,3 Hz), 6,18 (br s, 1H), 6,89 (d, 1H, *J* = 1,9 Hz), 7,14 à 7,29 (m, 7H), 7,51 (d, 2H, *J* = 7,9 Hz), 7,64 (dd, 1H, *J* = 7,9 / 1,9 Hz), 7,71 (s, 1H), 7,73 (d, 2H, *J* = 8,4 Hz), 7,86 (d, 2H, *J* = 8,4 Hz).

### EXEMPLE 59

### N,N-diéthyl-2'-(5,5,8,8-tétraméthyl-5,6,7,8-tétrahydro-naphthalèn-2-yl)-[1,1';4',1'']terphényl-4''-carboxamide.

De manière analogue à l'exemple 57(b), à partir de 1,30 g (2,7 mmoles) du chlorure d'acide obtenu à l'exemple 57 (a) et 5,6 ml (54,0 mmoles) de diéthylamine, on obtient 930 mg (67%) de N,N-diéthyl-2'-(5,5,8,8-tétraméthyl-5, 6,7,8-tétrahydro-naphthalèn-2-yl)-[1,1';4',1'']terphényl-4''-carboxamide, sous la forme d'une poudre beige de point de fusion 113°C.
¹H NMR (CDCl₃) δ 0,85 (s, 6H), 1,25 (m, 6H), 1,27 (s, 6H), 1,56 à 1,64 (m, 4H), 3,35 (br s, 2H), 3,56 (br s, 2H), 6,90 (s, 1H), 7,14 à 7,28 (m, 7H), 7,47 (d, 2H, *J* = 8,2 Hz), 7,52 (s, 1H), 7,63 (dd, 1H, *J* = 8,0 / 1,4 Hz), 7,68 à 7,71 (m, 3H).

### EXEMPLE 60

### Morpholin-4-yl-[2'-(5,5,8,8-tétraméthyl-5,6,7,8-tétrahydro-naphthalèn-2-yl)-[1,1';4',1'']terphényl-4"-yl]-méthanone.

De manière analogue à l'exemple 57(b), à partir de 1,03 g (2,1 mmoles) du chlorure d'acide obtenu à l'exemple 57(a) et 945 µl (43,0 mmoles) de morpholine, on obtient 900 mg (80%) de morpholin-4-yl-[2'-(5,5,8,8-tétraméthyl-5,6,7,8-tétrahydro-naphthalèn-2-yl)-[1,1';4',1"]terphényl-4"-yl]-méthanone, sous la forme d'une poudre blanche de point de fusion 223°C.
¹H NMR (CDCl₃) δ 0,91 (s, 6H), 1,27 (s, 6H), 1,56 à 1,64 (m, 4H), 3,60 à 4,00 (m, 8H), 6,90 (d, 1H, *J* = 1,7 Hz), 7,13 à 7,26 (m, 9H), 7,49 (s, 1H), 7,50 (d, 2H, *J* = 8,4 Hz), 7,63 (dd, 1H, *J* = 7,9 / 1,8 Hz), 7,72 (d, 2H, *J* = 8,4 Hz).

### EXEMPLE 61

### (4-Hydroxy-phényl)-2'-(5,5,8,8-tétraméthyl-5,6,7,8-tétrahydro-naphthalèn-2-yl)-[1,1';4',1'']terphényl-4''-carboxamide.

De manière analogue à l'exemple 57(b), à partir de 1,04 g (2,2 mmoles) du chlorure d'acide obtenu à l'exemple 57(a), 260 mg (23,9 mmoles) de 4-aminophénol et 362 µl (2,7 mmoles) de triéthylamine, on obtient 1,15 g (95%) de (4-hydroxy-phényl)-2'-(5,5,8,8-tétraméthyl-5,6,7,8-tétrahydro-naphthalèn-2-yl)-[1,1';4',1"]terphényl-4"-carboxamide, sous la forme d'une poudre grise de point de fusion 231°C.
¹H NMR (CDCl₃) δ 0,91 (s, 6H), 1,26 (s, 6H), 1,56 à 1,64 (m, 4H), 6,84 (d, 2H, *J* = 8,5 Hz), 6,89 (d, 1H, *J* = 1,2 Hz), 7,14 à 7,28 (m, 7H), 7,41 à 7,44 (m, 3H), 7,51 (d, 1H, *J* = 7,8 Hz), 7,64 (d, 1H, *J* = 7,8 Hz), 7,72 (s, 1H), 7,75 (d, 2H, *J* = 8,0 Hz), 7,95 (d, 2H, *J* = 8,0 Hz), 8,06 (s, 1H).

### EXEMPLE 62

### Acide 3-(5,5,8,8-tétraméthyl-5,6,7,8-tétrahydro-naphthalèn-2-yl)-biphényl-4-carboxyméthyle-4'-carboxylique.

(a) 2'-(5,5,8,8-tétraméthyl-5,6,7,8-tétrahydro-naphthalèn-2-yl)-4-trifluorométhanesulfonyloxy-[1,1';4',1"]terphényl-4"-carboxylate de benzyle.
   De manière analogue à l'exemple 13(a), à partir de 2,00 g (4,1 mmoles) du composé obtenu à l'exemple 53(a), on obtient 2,33 g (90%) du produit attendu, sous la forme d'une huile jaune.
   ¹H NMR (CDCl₃) δ 1,31 (s, 6H), 1,32 (s, 6H), 1,73 (s, 4H), 5,39 (s, 2H), 7,24 à 7,26 (m, 2H), 7,37 à 7,48 (m, 6H), 7,60 à 7,69 (m, 2H), 7,66 (d, 2H, *J* = 8,3 Hz), 8,16 (d, 2H, *J* = 8,3 Hz).
(b) 3-(5,5,8,8-tétraméthyl-5,6,7,8-tétrahydro-naphthalèn-2-yl)-biphényl-4-carboxyméthyle-4'-carboxylate de benzyle.
   Dans une bombe à hydrogéner, on introduit successivement 1,80 g (2,9 mmoles) du triflate obtenu à l'exemple 62(a), 120 mg (0,29 mmole) de 1,3-bis(diphéylphosphino)propane (DPPP), 32 mg (0,14 mmole) d'acétate de palladium, 50 ml de méthanol, 800 µl (5,8 mmoles) de triéthylamine et 5 ml de THF. Le milieu réactionnel est confiné sous une pression de six bars de monoxyde de carbone et chauffé sous agitation à 70°C pendant sept heures. Le mélange est refroidi, évaporé au maximum, repris par une solution saturée de chlorure de sodium, extrait par de l'acétate d'éthyle, lavé à l'aide d'une solution diluée d'acide chlorhydrique, puis à l'eau, la phase organique est séchée sur sulfate de magnésium et évaporée. Le résidu obtenu est purifié par chromatographie sur colonne de silice élué avec de l'heptane. Après évaporation des solvants, on recueille 1,36 g (88%) du composé attendu, sous la forme d'une huile jaune.
   ¹H NMR (CDCl₃) δ 1,21 (s, 6H), 1,25 (s, 6H), 1,64 (s, 4H), 3,58 (s, 3H), 5,32 (s, 2H), 7,09 (dd, 1H, *J* = 8,1 / 2,0 Hz), 7,18 (d, 1H, *J* = 2,2 Hz), 7,27 à 7,38 (m, 6H), 7,53 à 7,56 (m, 2H), 7,62 (d, 2H, *J* = 8,5 Hz), 7,79 (d, 1H, *J* = 7,6 Hz), 8,08 (d, 2H, *J* = 8,5 Hz).
(c) acide 3-(5,5,8,8-tétraméthyl-5,6,7,8-tétrahydro-naphthalèn-2-yl)-biphényl-4-carboxyméthyle-4'-carboxylique.
   De manière analogue à l'exemple 53(c), à partir de 450 mg (0,84 mmole) de l'ester benzylique obtenu à l'exemple 62(b), on recueille 330 mg (89%) d'acide 3-(5,5,8,8-tétraméthyl-5,6,7,8-tétrahydro-naphthalèn-2-yl)-biphényl-4-carboxyméthyle-4'-carboxylique, sous la forme d'une poudre blanche de point de fusion 258-261°C.
   ¹H NMR (DMSO D₆) δ 1,25 (s, 6H), 1,29 (s, 6H), 1,67 (s, 4H), 3,64 (s, 3H), 7,23 (dd, 1H, *J* = 8, 0 / 1,8 Hz), 7,25 (s, 1H), 7,74 (s, 1H), 7,80 (s, 1H), 7,92 (d, 2H, *J* = 8,4 Hz), 8,05 (d, 2H, *J* = 8,4 Hz).

### EXEMPLE 63

### Acide 3- (5,5,8,8-tétraméthyl-5, 6, 7,8-tétrahydro-naphthalèn-2-yl)-biphényl-4,4'-dicarboxylique.

De manière analogue à l'exemple 1(e), à partir de 850 mg (1,6 mmole) du diester obtenu à l'exemple 62(b), on obtient 600 mg (88%) d'acide 3-(5,5,8,8-tétraméthyl-5,6,7,8-tétrahydro-naphthalèn-2-yl)-biphényl-4,4'-dicarboxylique, sous la forme d'un solide blanc cristallisé de point de fusion 343°C.
¹H NMR (DMSO D₆) δ 1,27 (s, 6H), 1,28 (s, 6H), 1,67 (s, 4H), 7,25 (dd, 1H, *J* = 7,9 / 1,9 Hz), 7,31 (s, 1H), 7,69 (s, 1H), 7,78 (s, 1H), 7,91 (d, 2H, *J* = 8,4 Hz), 8,04 (d, 2H, *J* = 8,4 Hz).

### EXEMPLES DE FORMULATION

Dans les exemples suivants, on a illustré diverses formulations pharmaceutiques et cosmétiques à base des composés selon l'invention.

### A - VOIE ORALE

***(a) Comprimé de 0,2 g***
   - Composé préparé à l'exemple 2 10,001 g
   - Amidon 0,114 g
   - Phosphate bicalcique 0,020 g
   - Silice 0,020 g
   - Lactose 0,030 g
   - Talc 0,010 g
   - Stéarate de magnésium 0,005 g

   Dans cet exemple, le composé selon l'exemple 2 peut être remplacé par la même quantité d'un des composés des exemples 4, 8, 14, 17, 29 et 34.
***(b) Suspension buvble en ampoules de 5 ml***
   - Composé préparé à l'exemple 1 20,001 g
   - Glycérine 0,500 g
   - Sorbitol à 70 % 0,500 g
   - Saccharinate de sodium 0,010 g
   - p-hydroxybenzoate de méthyle 0,040 g
   - Arôme qs
   - Eau purifiée q.s.p 5 ml
***(c) Comprimé de 0,8 g***
   - Composé de l'exemple 4 0,500 g
   - Amidon prégélatinisé 0,100 g
   - Cellulose microcristalline 0,115 g
   - Lactose 0,075 g
   - Stéarate de magnésium 0,010 g

   Dans cet exemple, le composé selon l'exemple 4 peut être remplacé par la même quantité d'un des composés des exemples 11, 18, 21, 24, 39 et 48.
***(d) Suspension buvable en ampoules de 10 ml***
   - Composé de l'exemple 5 0,200 g
   - Glycérine 1,000 g
   - Sorbitol à 70 % 1,000 g
   - Saccharinate de sodium 0,010 g
   - p-hydroxybenzoate de méthyle 0,080 g
   - Arôme qs
   - Eau purifiée q.s.p. 10 ml

### B - VOIE TOPIQUE

***(a) Onguent***
   - Composé de l'exemple 3 20,020 g
   - Myristate d'isopropyle 81,700 g
   - Huile de vaseline fluide 9,100 g
   - Silice ("Aérosil 200" vendue par Degussa) 9,180 g

   Dans cet exemple, le composé selon l'exemple 3 peut être remplacé par la même quantité d'un des composés des exemples 7, 14, 27, 36 et 53.
***(b) Onguent***
   - Composé de l'exemple 6 0,300 g
   - Vaseline blanche codex 100 g
***(c) Crème eau-dans-l'huile non ionique***
   - Composé de l'exemple 2 0,100 g
   - Mélange d'alcools de lanoline émulsifs, de cires et d'huiles ("Eucérine anhydre" vendu par BDF) 39,900 g
   - p-hydroxybenzoate de méthyle 0,075 g
   - p-hydroxybenzoate de propyle 0,075 g
   - Eau déminéralisée stérile q.s.p. 100 g
***(d) Lotion***
   - Composé de l'exemple 3 0,100 g
   - Polyéthylène glycol (PEG 400) 69,900 g
   - Ethanol à 95 % 30,000 g

   Dans cet exemple, le composé selon l'exemple 3 peut être remplacé par la même quantité d'un des composés des exemples 8, 18, 24, 32, 35, 43 et 46.
***(e) Onguent hydrophobe***
   - Composé de l'exemple 1 0,300 g
   - Myristate d'isopropyle 36,400 g
   - Huile de silicone ("Rhodorsil 47V300" vendu par Rhône-Poulenc) 36,400 g
   - Cire d'abeille 13,600 g
   - Huile de silicone ("Abil 300.000 cst" vendu par Goldschmidt) 100 g
***(f) Crème huile-dans-l'eau non ionique***
   - Composé de l'exemple 5 1,000 g
   - Alcool cétylique 4,000 g
   - Monostéarate de glycérol 2,500 g
   - Stéarate de PEG 50 2,500 g
   - Beurre de karité 9,200 g
   - Propylène glycol 2,000 g
   - p-hydroxybenzoate de méthyle 0,075 g
   - p-hydroxybenzoate de propyle 0,075 g
   - Eau déminéralisée stérile 100 g

Dans cet exemple, le composé selon l'exemple 5 peut être remplacé par la même quantité d'un des composés des exemples 29, 49, 51, 52, 58 et 62.

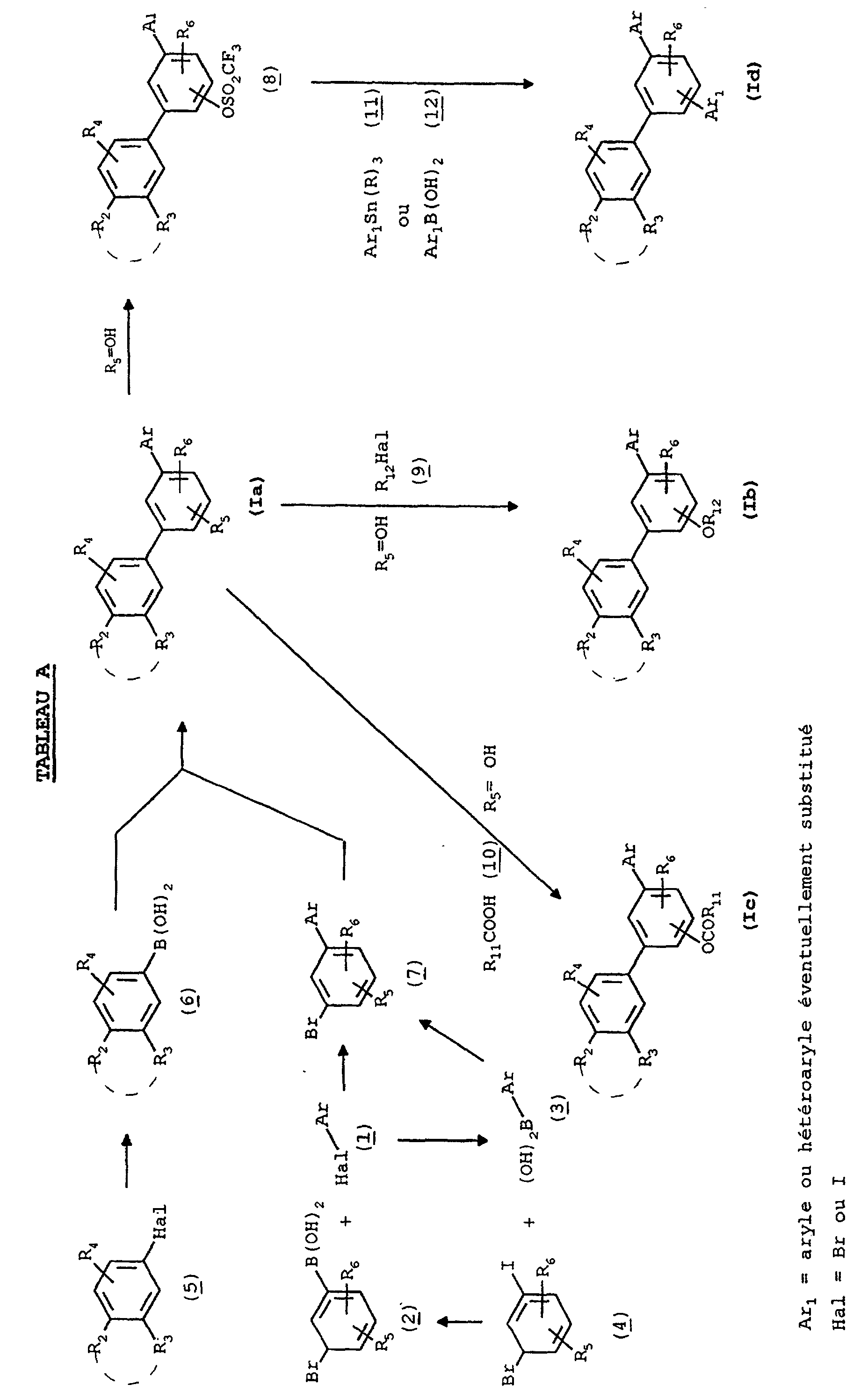

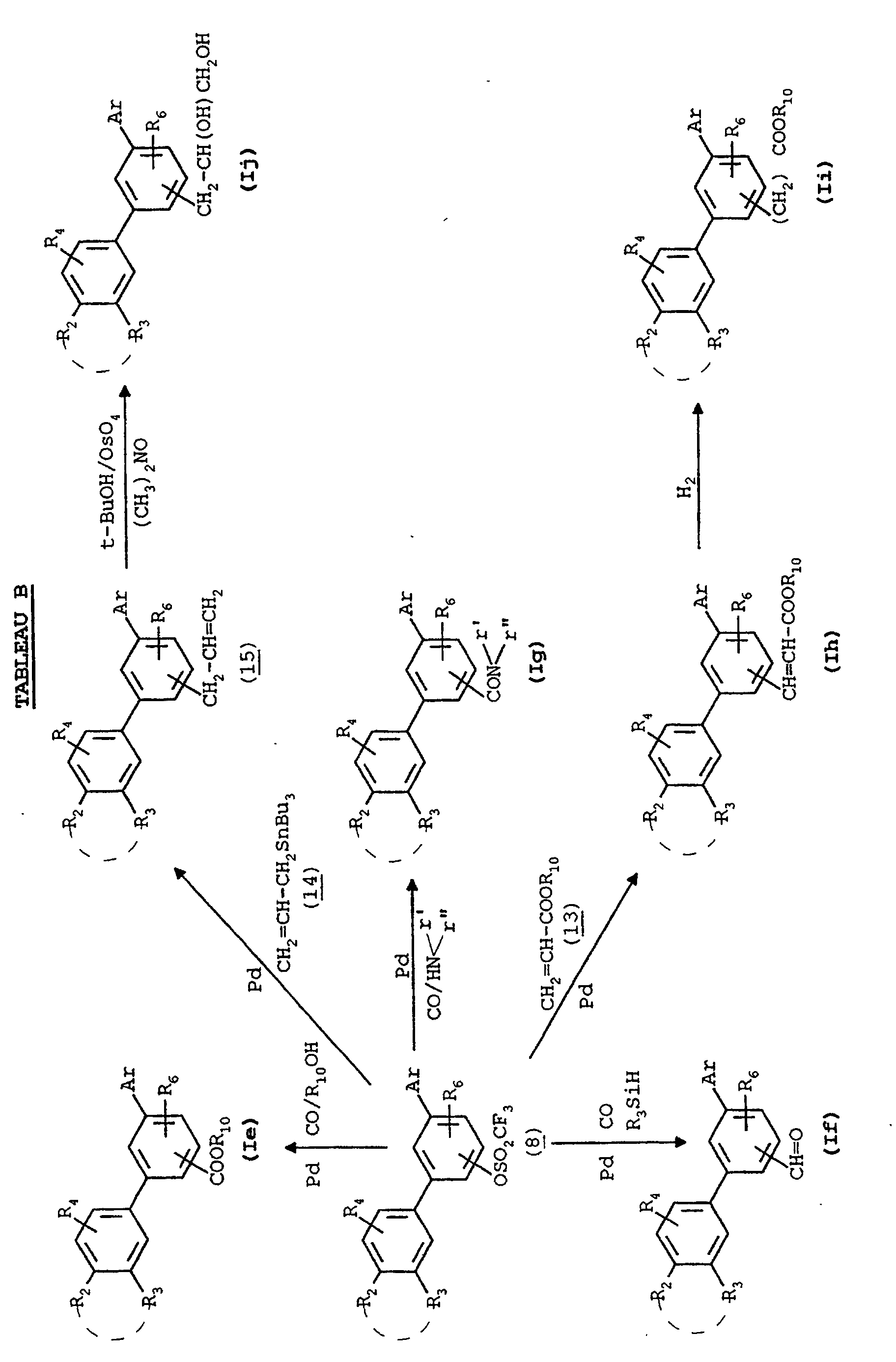

## Revendications

1. Composés biphényliques substitués par un radical aromatique ou hétéroaromatique, **caractérisés en ce qu'**ils répondent à la formule générale (I) suivante : 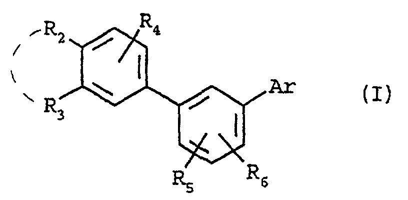 dans laquelle :
Ar représente un radical aromatique ou hétéroaromatique choisi parmi : 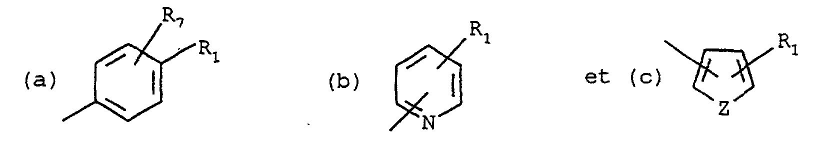
Z étant O ou S,
R₁ représente -CH₃, -CH₂-OH, -OR₈ ou -COR₉,
R₂ et R₃, identiques ou différents, représentent H, alkyle, linéaire ou ramifié, en C₁-C₁₅, cycloalkyle, -ZR₁₀ ou un radical polyéther, l'un au moins de R₂ ou R₃ représentant un radical alkyle, linéaire ou ramifié, en C₁-C₁₅, ou
R₂ et R₃, pris ensemble forment un cycle à 5 ou 6 chaînons, éventuellement substitué par au moins un méthyle et/ou éventuellement interrompu par un atome d'oxygène ou de soufre ou par un radical SO ou SO₂,
R₄ représente H, un atome d'halogène, alkyle, linéaire ou ramifié, en C₁-C₂₀, -OR₁₀, -OCOR₁₁, ou un radical polyéther,
R₅ représente H, un atome d'halogène, alkyle, linéaire ou ramifié, en C₁-C₂₀, -OCOR₁₁, -OR₁₂, mono- ou polyhydroxyalkyle, -NO₂, 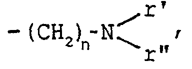 - (CH₂)ₙ-NHCOCH₃, -CH=CH-COR₁₃, -(CH₂)ₙCOR₁₃, n étant 0 à 6, -O-(CH₂)ₘCOR₁₃, -O-(CH₂)ₘOH, m étant 1 à 12, aryle éventuellement substitué, aralkyle éventuellement substitué, hétéroaryle éventuellement substitué, un radical polyéther, ou un radical -CH₂-polyéther.
R₆ représente H, alkyle inférieur en C1-C6 ou -OR₁₀,
R₇ représente H, un atome d'halogène, alkyle, linéaire ou ramifié en C₁-C₂₀, -OR₁₀ ou -OCOR₁₁ ou un radical polyéther,
R₈ représente H, alkyle inférieur en C₁-C₆ ou -COR₁₁,
R₉ représente H, alkyle inférieur en C₁-C₆, -OR₁₄ ou 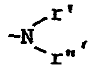
R₁₀ représente H ou alkyle inférieur en C₁-C₆,
R₁₁ représente alkyle inférieur en C₁-C₆,
R₁₂ représente H, alkyle, linéaire ou ramifié, en C₁-C₂₀, mono- ou polyhydroxyalkyle, aryle ou aralkyle éventuellement substitué,
R₁₃ représente H, alkyle inférieur en C₁-C₆, -OR₁₀, aryle ou 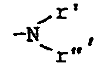
R₁₄ représente H, alkyle, linéaire ou ramifié, en C₁-C₂₀, alkényle, mono- ou polyhydroxyalkyle, aryle ou aralkyle éventuellement substitué, ou un reste de sucre,
r' et r", identiques ou différents, représentent H, OH, alkyle inférieur en C₁-C₆, mono- ou polyhydroxyalkyle, aryle éventuellement substitué, un reste d'aminoacide, un reste de peptide ou r' et r", pris ensemble, forment un hétérocycle,
et les sels des composés de formule (I) lorsque R₁ représente une fonction acide carboxylique ainsi que les isomères optiques et géométriques desdits composés de formule (I).

2. Composés selon la revendication 1, **caractérisés en ce qu'**ils se présentent sous forme d'un sel d'un métal alcalin ou alcalino-terreux, ou encore de zinc ou d'une amine organique.

3. Composés selon l'une des revendications 1 ou 2, **caractérisés en ce que** le radical alkyle inférieur en C₁-C₆ est choisi dans le groupe constitué par les radicaux méthyle, éthyle, isopropyle, butyle, tertiobutyle, et hexyle.

4. Composés selon l'une quelconque des revendications précédentes, **caractérisés en ce que** le radical alkyle, linéaire ou ramifié, lorsqu'il est en C₁-C₁₅, est choisi dans le groupe constitué par les radicaux méthyle, éthyle, propyle, 2-éthyl-hexyle, octyle et dodécyle, et lorsqu'il est en C₁-C₂₀ est choisi en outre parmi les radicaux hexadécyle et octadécyle.

5. Composés selon l'une quelconque des revendications précédentes, **caractérisés en ce que** le radical monohydroxyalkyle est choisi dans le groupe constitué par les radicaux hydroxyméthyle, 2-hydroxyéthyle, 2-hydroxypropyle ou 3-hydroxypropyle.

6. Composés selon l'une quelconque des revendications précédentes, **caractérisés en ce que** le radical polyhydroxyalkyle est choisi dans le groupe constitué par les radicaux 2,3-dihydroxypropyle, 2,3,4-trihydroxybutyle, 2,3,4,5-tétrahydroxypentyle ou le reste du pentaérythritol.

7. Composés selon l'une quelconque des revendications précédentes, **caractérisés en ce que** le radical polyéther est choisi dans le groupe constitué par les radicaux méthoxyméthoxy, méthoxyéthoxy ou méthoxyéthoxyméthoxy.

8. Composés selon l'une quelconque des revendications précédentes, **caractérisés en ce que** le radical -CH₂-polyéther est choisi dans le groupe constitué par les radicaux méthoxyméthoxyméthyle, éthoxyméthoxyméthyle et méthoxyéthoxyméthoxyméthyle.

9. Composés selon l'une quelconque des revendications précédentes, **caractérisés en ce que** le radical aryle est un radical phényle éventuellement substitué par au moins un halogène, un hydroxyle, une fonction nitro, un radical polyéther ou une fonction amino éventuellement protégée par un groupe acétyle ou éventuellement substitué par au moins un alkyle inférieur en C₁-C₆ ou un alkoxy en C₁-C₆.

10. Composés selon l'une quelconque des revendications précédentes, **caractérisés en ce que** le radical aralkyle est choisi dans le groupe constitué par les radicaux benzyle ou phénéthyle éventuellement substitué par au moins un halogène, un hydroxyle, une fonction nitro, un radical polyéther ou une fonction amino éventuellement protégée par un groupe acétyle ou éventuellement substitué par au moins un alkyle inférieur en C₁-C₆ ou un alkoxy en C₁-C₆.

11. Composés selon l'une quelconque des revendications précédentes, **caractérisés en ce que** le radical hétéroaryle est choisi dans le groupe constitué par les radicaux pyridyle, furyle ou thiényle, éventuellement substitué par au moins un halogène, un alkyle inférieur en C₁-C₆, un hydroxyle, un alkoxy en C₁-C₃, une fonction nitro, un radical polyéther ou une fonction amino éventuellement protégée par un groupe acétyle ou éventuellement substitué par au moins un alkyle inférieur ou un alkoxy en C₁-C₆.

12. Composés selon l'une quelconque des revendications précédentes, **caractérisés en ce que** le radical alkényle est choisi dans le groupe constitué par les radicaux contenant de 2 à 5 atomes de carbone et présentant une ou deux insaturation(s) éthylénique(s), en particulier le radical allyle.

13. Composés selon l'une quelconque des revendications précédentes, **caractérisés en ce que** le reste de sucre est choisi dans le groupe constitué par les restes de glucose, de galactose, de mannose ou d'acide glucuronique.

14. Composés selon l'une quelconque des revendications précédentes, **caractérisés en ce que** le reste d'aminoacide est choisi dans le groupe constitué par les restes dérivant de la lysine, de la glycine ou de l'acide aspartique.

15. Composés selon l'une quelconque des revendications précédentes, **caractérisés en ce que** le reste de peptide est choisi dans le groupe constitué par les restes de dipeptides ou de tripeptides.

16. Composés selon l'une quelconque des revendications précédentes, **caractérisés en ce que** lorsque r' et r" forment un hétérocycle, celui-ci est choisi dans le groupe constitué par les radicaux pipéridino, morpholino, pyrrolidino ou pipérazino, éventuellement substitué en position 4 par un alkyle en C₁-C₆ ou un mono- ou polyhydroxyalkyle.

17. Composés selon l'une quelconque des revendications précédentes, **caractérisés en ce que** l'atome d'halogène est choisi dans le groupe constitué par le fluor, le chlore et le brome.

18. Composés selon l'une quelconque des revendications précédentes, **caractérisés par le fait qu'**ils répondent aux formules générales (II) et (III) suivantes : 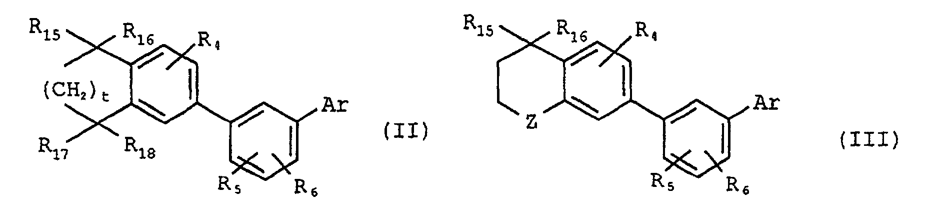 dans lesquelles :
Ar représente un radical de formule (a) ou (b) suivante : 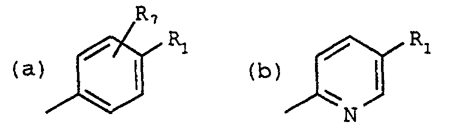
R₁, R₄, R₅, R₆, R₇ et Z ayant les mêmes significations que celles données à la revendication 1,
R₁₅, R₁₆, R₁₇ et R₁₈, identiques ou différents, représentent H ou -CH₃, et
t est 1 ou 2.

19. Composés selon l'une quelconque des revendications précédentes, **caractérisés par le fait qu'**ils sont pris dans le groupe constitué par :
- acide 4-[4-hydroxy-3-(5,6,7,8-tétrahydro-5,5,8,8-tétra-méthyl-2-naphtyl)phényl]benzoïque, et son ester méthylique,
- acide 4-[4-(5-hydroxypentyloxy)-3-(5,6,7,8-tétrahydro-5,5,8,8-tétraméthyl-2-naphtyl)phényl]benzoïque, et son ester méthylique,
- acide 4-[4-(6-hydroxyhexyloxy)-3-(5,6,7,8-tétrahydro-5,5,8,8-tétraméthyl-2-naphtyl)phényl]benzoïque, et son ester méthylique,
- acide 4-[4-(7-hydroxyheptyloxy)-3-(5,6,7,8-tétrahydro-5,5,8,8-tétraméthyl-2-naphtyl)phényl]benzoïque,
- acide 4-[4-(8-hydroxyoctyloxy)-3-(5,6,7,8-tétrahydro-5,5,8,8-tétraméthyl-2-naphtyl)phényl]benzoïque,
- acide 4-[4-(9-hydroxynonyloxy)-3-(5,6,7,8-tétrahydro-5,5,8,8-tétraméthyl-2-naphtyl)phényl]benzoïque,
- acide 4-[4-méthoxy-3-(5,6,7,8-tétrahydro-5,5,8,8-tétraméthyl-2-naphtyl) phényl]benzoïque,
- acide 4-[4-méthoxyéthoxyméthoxy-3-(5,6,7,8-tétrahydro-5,5,8,8-tétraméthyl-2-naphtyl)phényl]benzoïque,
- acide 4-[4-benzyloxy-3-(5,6,7,8-tétrahydro-5,5,8,8-tétraméthyl-2-naphtyl)phényl]benzoïque,
- acide 4'-(2,3-dihydroxy-propoxy)-3'-(5,5,8,8-tétraméthyl-5,6,7,8-tétrahydro-naphthalèn-2-yl)-biphényl-4-carboxylique (racémique),
- acide 4'-(2,2-diméthyl-[1,3]dioxolan-4-ylméthoxy)-3'-(5,5,8,8-tétraméthyl-5,6,7,8-tétrahydro-naphthalèn-2-yl)-biphényl-4-carboxylique (racémique),
- acide 4'-(2-morpholin-4-yl-éthoxy)-3'-(5,5,8,8-tétraméthyl-5,6,7,8-tétrahydro-naphthalèn-2-yl)-biphényl-4-carboxylique,
- 2'-(5,5,8,8-tétraméthyl-5,6,7,8-tétrahydro-naphthalèn-2-yl)-[1,1';4',1'']terphényl-4''-carboxylate de méthyle,
- acide 2'-(5,5,8,8-tétraméthyl-5,6,7,8-tétrahydro-naphthalèn-2-yl)-[1,1';4',1'']terphényl-4''-carboxylique,
- acide 4-méthoxyméthoxy-2'-(5,5,8,8-tétraméthyl-5,6,7,8-tétrahydro-naphthalèn-2-yl)-[1,1';4',1'']terphényl-4''-carboxylique,
- acide 4-hydroxy-2'-(5,5,8,8-tétraméthyl-5,6,7,8-tétrahydro-naphthalèn-2-yl)-[1,1';4',1'']terphényl-4''-carboxylique,
- acide 4-méthoxy-2'-(5,5,8,8-tétraméthyl-5,6,7,8-tétrahydro-naphthalèn-2-yl)-[1,1';4',1'']terphényl-4''-carboxylique,
- acide 3-méthoxyméthoxy-2'-(5,5,8,8-tétraméthyl-5,6,7,8-tétrahydro-naphthalèn-2-yl)-[1,1';4',1'']terphényl-4''-carboxylique,
- acide 3-hydroxy-2'-(5,5,8,8-tétraméthyl-5,6,7,8-tétrahydro-naphthalèn-2-yl)-[1,1';4',1'']terphényl-4''-carboxylique,
- acide 3-méthoxy-2'-(5,5,8,8-tétraméthyl-5,6,7,8-tétrahydro-naphthalèn-2-yl)-[1,1';4',1"]terphényl-4"-carboxylique,
- acide 2-méthoxyméthoxy-2'-(5,5,8,8-tétraméthyl-5,6,7,8-tétrahydro-naphthalèn-2-yl)-[1,1';4',1"]terphényl-4"-carboxylique,
- acide 2-hydroxy-2'-(5,5,8,8-tétraméthyl-5,6,7,8-tétrahydro-naphthalèn-2-yl)-[1,1';4',1"]terphényl-4"-carboxylique,
- acide 2-méthoxy-2'-(5,5,8,8-tétraméthyl-5,6,7,8-tétrahydro-naphthalèn-2-yl)-[1,1';4',1"]terphényl-4"-carboxylique,
- acide 2'-méthoxyméthoxy-5'-(5,5,8,8-tétraméthyl-5,6,7,8-tétrahydro-naphthalèn-2-yl)-biphényl-4-carboxylique,
- acide 2'-méthoxy-5'-(5,5,8,8-tétraméthyl-5,6,7,8-tétrahydro-naphthalèn-2-yl)-biphényl-4-carboxylique,
- acide 2'-propyloxy-5'-(5,5,8,8-tétraméthyl-5,6,7,8-tétrahydro-naphthalèn-2-yl)-biphényl-4-carboxylique,
- acide 2'-hydroxy-5'-(5,5,8,8-tétraméthyl-5,6,7,8-tétrahydro-naphthalèn-2-yl)-biphényl-4-carboxylique,
- acide 4'-(5,5,8,8-tétraméthyl-5,6,7,8-tétrahydro-naphthalèn-2-yl)-[1,1';2',1'']terphényl-4''-carboxylique,
- acide 2'-méthoxyméthoxy-3'-(5,5,8,8-tétraméthyl-5,6,7,8-tétrahydro-naphthalèn-2-yl)-biphényl-4-carboxylique,
- acide 2'-hydroxy-3'-(5,5,8,8-tétraméthyl-5,6,7,8-tétrahydro-naphthalèn-2-yl)-biphényl-4-carboxylique,
- acide 2'-méthoxy-3'-(5,5,8,8-tétraméthyl-5,6,7,8-tétrahydro-naphthalèn-2-yl)-biphényl-4-carboxylique,
- acide 3'-méthoxyméthoxyméthyl-5'-(5,5,8,8-tétraméthyl-5,6,7,8-tétrahydro-naphthalèn-2-yl)-biphényl-4-carboxylique,
- acide 3'-hydroxyméthyl-5'-(5,5,8,8-tétraméthyl-5,6,7,8-tétrahydro-naphthalèn-2-yl)-biphényl-4-carboxylique,
- acide 2'-(4,4-diméthyl-thiochroman-7-yl)-[1,1';4',1'']terphényl-4''-carboxylique,
- acide 2'-(4,4-diméthyl-thiochroman-6-yl)-[1,1';4',1'']terphényl-4''-carboxylique,
- acide 2'-(3,5,5,8,8-pentaméthyl-5,6,7,8-tétrahydro-naphthalèn-2-yl)-[1,1';4',1'']terphényl-4''-carboxylique,
- acide 2'-(3-méthoxyméthoxy-5,5,8,8-tetraméthyl-5,6,7,8-tétrahydro-naphthalèn-2-yl)-[1,1';4',1'']terphényl-4''-carboxylique,
- acide 2'-(3-hydroxy-5,5,8,8-tétraméthyl-5,6,7,8-tétrahydro-naphthalèn-2-yl)-[1,1';4',1'']terphényl-4''-carboxylique,
- acide 2'-(3-méthoxy-5,5,8,8-tétraméthyl-5,6,7,8-tétrahydro-naphthalèn-2-yl)-[1,1';4',1'']terphényl-4''-carboxylique,
- acide 2'-(3-propyloxy-5,5,8,8-tétraméthyl-5,6,7,8-tétrahydro-naphthalèn-2-yl)-[1,1';4',1'']terphényl-4''-carboxylique,
- acide 3''-méthyl-2'-(5,5,8,8-tétraméthyl-5,6,7,8-tétrahydro-naphthalèn-2-yl)-[1,1';4',1'']terphényl-4''-carboxylique,
- acide 2"-hydroxy-2'-(5,5,8,8-tétraméthyl-5,6,7,8-tétrahydro-naphthalèn-2-yl)-[1,1';4',1"]terphényl-4"-carboxylique,
- acide 2''-méthoxyméthoxy-2'-(5,5,8,8-tétraméthyl-5,6,7,8-tétrahydro-naphthalèn-2-yl)-[1,1';4',1'']terphényl-4''-carboxylique,
- acide 2''-méthoxy-2'-(5,5,8,8-tétraméthyl-5,6,7,8-tétrahydro-naphthalèn-2-yl)-[1,1';4',1'']terphényl-4''-carboxylique,
- acide 2''-propyloxy-2'-(5,5,8,8-tétraméthyl-5,6,7,8-tétrahydro-naphthalèn-2-yl)-[1,1';4',1'']terphényl-4''-carboxylique,
- acide 3''-hydroxy-2'-(5,5,8,8-tétraméthyl-5,6,7,8-tétrahydro-naphthalèn-2-yl)-[1,1';4',1'']terphényl-4''-carboxylique,
- acide 6-[2-(5,5,8,8-tétraméthyl-5,6,7,8-tétrahydro-naphthalèn-2-yl)-biphényl-4-yl]-nicotinique,
- acide 5-[2-(5,5,8,8-tétraméthyl-5,6,7,8-tétrahydro-naphthalèn-2-yl)-biphényl-4-yl]-pyridine-2-carboxylique,
- acide 2'-(5,5,8,8-tétraméthyl-5,6,7,8-tétrahydro-naphthalèn-2-yl)-[1,1';4',1'']terphényl-4''-hydroxamique,
- 2'-(5,5,8,8-tétraméthyl-5,6,7,8-tétrahydro-naphthalèn-2-yl)-[1,1';4',1'']terphényl-4''-ol,
- [2'-(5,5,8,8-tétraméthyl-5,6,7,8-tétrahydro-naphthalèn-2-yl)-[1,1';4',1"]terphényl-4"-yl]-méthanol,
- 2'-(5,5,8,8-tétraméthyl-5,6,7,8-tétrahydro-naphthalèn-2-yl)-[1,1';4',1"]terphényl-4"-carbaldéhyde,
- acide 4'-méthoxycarbonylméthoxy-3'-(5,5,8,8-tétraméthyl-5,6,7,8-tétrahydro-naphthalèn-2-yl)-biphényl-4-carboxylique,
- acide 4'-carboxyméthoxy-3'-(5,5,8,8-tétraméthyl-5,6,7,8-tétrahydro-naphthalèn-2-yl)-biphényl-4-carboxylique,
- acide 4'-(5-éthoxycarbonyl-pentyloxy)-3'-(5,5,8,8-tétraméthyl-5,6,7,8-tétrahydro-naphthalèn-2-yl)-biphényl-4-carboxylique,
- acide 4'-(5-carboxy-pentyloxy)-3'-(5,5,8,8-tétraméthyl-5,6,7,8-tétrahydro-naphthalèn-2-yl)-biphényl-4-carboxylique,
- 2'-(5,5,8,8-tétraméthyl-5,6,7,8-tétrahydro-naphthalèn-2-yl)-[1,1';4',1'']terphényl-4''-carboxamide,
- N-éthyl-2'-(5,5,8,8-tétraméthyl-5,6,7,8-tétrahydro-naphthalèn-2-yl)-[1,1';4',1'']terphényl-4''-carboxamide,
- N,N-diéthyl-2'-(5,5,8,8-tétraméthyl-5,6,7,8-tétrahydro-naphthalèn-2-yl)-[1,1';4',1"]terphényl-4"-carboxamide,
- morpholin-4-yl-[2'-(5,5,8,8-tétraméthyl-5,6,7,8-tétrahydro-naphthalèn-2-yl)-[1,1';4',1"]terphényl-4"-yl]-méthanone,
- (4-hydroxy-phényl)-2'-(5,5,8,8-tétraméthyl-5,6,7,8-tétrahydro-naphthalèn-2-yl)-[1,1',4',1'']terphényl-4''-carboxamide,
- acide 3-(5,5,8,8-tétraméthyl-5,6,7,8-tétrahydro-naphthalèn-2-yl)-biphényl-4-carboxyméthyle-4'-carboxylique,
- acide 3-(5,5,8,8-tétraméthyl-5,6,7,8-tétrahydro-naphthalèn-2-yl)-biphényl-4,4'-dicarboxylique,
- acide 3'-méthoxyméthoxy-5'-(5,5,8,8-tétraméthyl-5,6,7,8-tétrahydro-naphthalèn-2-yl)-biphényl-4-carboxylique,
- acide 3'-méthoxy-5'-(5,5,8,8-tétraméthyl-5,6,7,8-tétrahydro-naphthalèn-2-yl)-biphényl-4-carboxylique,
- acide 3'-propyloxy-5'-(5,5,8,8-tétraméthyl-5,6,7,8-tétrahydro-naphthalèn-2-yl)-biphényl-4-carboxylique,
- acide 3'-hydroxy-5'-(5,5,8,8-tétraméthyl-5,6,7,8-tétrahydro-naphthalèn-2-yl)-biphényl-4-carboxylique,
- acide 4'-(5,5,8,8-tétraméthyl-5,6,7,8-tétrahydro-naphthalèn-2-yl)-[1,1';3",1"]terphényl-4"-carboxylique,
- acide 4'-(5-carboxamido-pentyloxy)-3'-(5,5,8,8-tétraméthyl-5,6,7,8-tétrahydro-naphthalèn-2-yl)-biphényl-4-carboxylique,
- acide 3'-méthoxycarbonyl-5'-(5,5,8,8-tétraméthyl-5,6,7,8-tétrahydro-naphthalèn-2-yl)-biphényl-4-carboxylique,
- acide 3'-carboxyl-5'-(5,5,8,8-tétraméthyl-5,6,7,8-tétrahydro-naphthalèn-2-yl)-biphényl-4-carboxylique,
- acide 2'-(4-hydroxy-5,5,8,8-tétraméthyl-5,6,7,8-tétrahydro-naphthalèn-2-yl)-[1,1';4',1"]terphényl-4"-carboxylique,
- acide 2'-(4-méthoxy-5,5,8,8-tétraméthyl-5,6,7,8-tétrahydro-naphthalèn-2-yl)-[1,1';4',1"]terphényl-4"-carboxylique,
- acide 2'-(4-propyloxy-5,5,8,8-tétraméthyl-5,6,7,8-tétrahydro-naphthalèn-2-yl)-[1,1';4',1"]terphényl-4"-carboxylique,
- acide 2'-(4-méthoxyméthoxy-5,5,8,8-tétraméthyl-5,6,7,8-tétrahydro-naphthalèn-2-yl)-[1,1';4',1"]terphényl-4"-carboxylique,
- acide 2-[2-(5,5,8,8-tétraméthyl-5,6,7,8-tétrahydro-naphthalèn-2-yl)-biphényl-4-yl]-thiophene-4-carboxylique.

20. Composés selon l'une quelconque des revendications précédentes pour une utilisation comme médicament.

21. Composés selon la revendication 20 pour une utilisation comme médicament destiné au traitement des affections dermatologiques, des affections dermatologiques à composante inflammatoire et/ou immuno-allergique du type rhumatismale ou respiratoire, des affections cardio-vasculaires et des troubles ophtalmologiques.

22. Utilisation de l'un au moins des composés tels que définis selon l'une quelconque des revendications 1 à 19 pour la préparation d'un médicament destiné au traitement des affections dermatologiques, des affections dermatologiques à composante inflammatoire et/ou immuno-allergique du type rhumatismale ou respiratoire, des affections cardio-vasculaires et des troubles ophtalmologiques.

23. Composition pharmaceutique, **caractérisée par le fait qu'**elle comprend, dans un support pharmaceutiquement acceptable, au moins un composé tel que défini selon l'une quelconque des revendications 1 à 19.

24. Composition selon la revendication 23, **caractérisée en ce que** la concentration en au moins un composé selon l'une des revendications 1 à 19 est comprise entre 0,001 % et 5 % en poids par rapport au poids total de la composition.

25. Composition cosmétique, **caractérisée par le fait qu'**elle contient, dans un support cosmétiquement acceptable, au moins un composé tel que défini selon l'une quelconque des revendications 1 à 19.

26. Composition selon la revendication 25, **caractérisée en ce que** la concentration en au moins un composé selon l'une quelconque des revendications 1 à 19 est comprise entre 0,001 et 3 % en poids par rapport au poids total de la composition.

27. Utilisation d'une composition cosmétique telle que définie selon l'une quelconque des revendications 25 ou 26 pour l'hygiène corporelle ou capillaire.

## Patentansprüche

1. Mit einem aromatischen oder heteroaromatischen Rest substituierte Biphenylverbindungen, **dadurch gekennzeichnet, dass** sie der folgenden allgemeinen Formel (I) entsprechen: 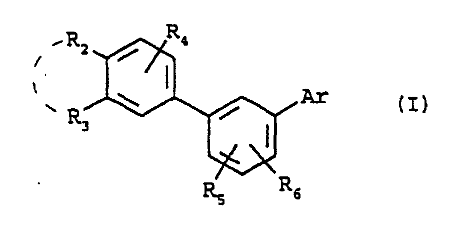 worin:
Ar für einen aromatischen oder heteroaromatischen Rest steht, ausgewählt unter: 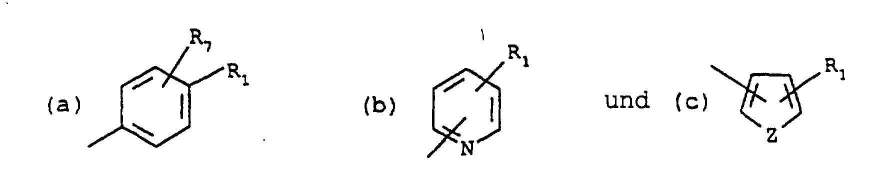
Z O oder S ist,
R₁ für -CH₃, -CH₂-OH, -OR₈ oder -COR₉ steht,
R₂ und R₃, gleich oder verschieden, für H, lineares oder verzweigtes C₁-C₁₅-Alkyl, Cycloalkyl, -ZR₁₀ oder einem Polyetherrest stehen, wobei mindestens einer der Reste R₂ oder R₃ für einen linearen oder verzweigten C₁-C₁₅-Alkylrest steht, oder
R₂ und R₃ zusammengenommen einen 5- oder 6-gliedrigen Ring bilden, gegebenenfalls substituiert mit mindestens einem Methyl und/oder gegebenenfalls unterbrochen durch ein Sauerstoff- oder Schwefelatom oder durch einen Rest SO oder SO₂,
R₄ für H, ein Halogenatom, lineares oder verzweigtes C₁-C₂₀-Alkyl, -OR₁₀, -OCOR₁₁ oder einen Polyetherrest steht,
R₅ für H, ein Halogenatom, lineares oder verzweigtes C₁-C₂₀-Alkyl, -OCOR_{11,} -OR₁₂, Mono- oder Polyhydroxyalkyl, -NO₂, 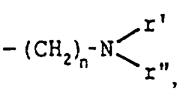 -(CH₂)ₘ-NHCOCH₃, -CH=CH-COR₁₃, -(CH₂)nCOR₁₃, worin n 0 bis 6 ist, -O-(CH₂)ₘCOR₁₃, -O-(CH₂)ₘOH worin m 1 bis 12 ist, gegebenenfalls substituiertes Aryl, gegebenenfalls substituiertes Aral kyl, gegebenenfalls substituiertes Heteroaryl, einen Polyetherrest oder einen -CH₂ Polyether-Rest steht,
R₆ für H, niederes C₁-C₆-Alkyl oder -OR₁₀ steht,
R₇ für H, ein Halogenatom, lineares oder verzweigtes C₁-C₂₀-Alkyl, -OR₁₀ oder -OCOR₁₁ oder einen Polyetherrest steht,
R₈ für H, niederes C₁-C₆-Alkyl oder -COR₁₁ steht,
R₉ für H, niederes C₁-C₆-Alkyl, -OR₁₄ oder 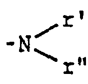 steht,
R₁₀ für H oder niederes C₁-C₆-Alkyl steht,
R₁₁ für niederes C₁-C₆-Alkyl steht,
R₁₂ für H, lineares oder verzweigtes C₁-C₂₀-Alkyl, Mono- oder Polyhydroxyalkyl, Aryl oder Aralkyl, gegebenenfalls substituiert, steht,
R₁₃ für H, niederes C₁-C₆-Alkyl, -OR₁₀, Aryl oder 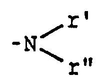 steht,
R₁₄ für H, lineares oder verzweigtes C₁-C₂₀-Alkyl, Alkenyl, Mono- oder Polyhydroxyalkyl, Aryl oder Aralkyl, gegebenenfalls substituiert, oder einen Zuckerrest steht,
r' und r" gleich oder verschieden sind und für H, OH, niederes C₁-C₆-Alkyl, Mono- oder Polyhydroxyalkyl, gegebenenfalls substituiertes Aryl, einen Aminosäurerest, einen Peptidrest stehen oder worin r' und r" zusammengenommen einen Heterocyclus bilden,
und Salze der Verbindungen der Formel (I), wenn R₁ eine Carbonsäurefunktion darstellt, wie auch die optischen und geometrischen Isomeren der Verbindungen der Formel (I).

2. Verbindungen gemäß Anspruch 1, **dadurch gekennzeichnet, dass** sie in Form eines Salzes eines Alkalimetalls oder Erdalkalimetalls oder auch von Zink oder eines organischen Amins vorliegen.

3. Verbindungen gemäß einem der Ansprüche 1 oder 2, **dadurch gekennzeichnet, dass** das niedere C₁-C₆-Alkyl ausgewählt ist aus der Gruppe, bestehend aus den Resten Methyl, Ethyl, Isopropyl, Butyl, tert.-Butyl und Hexyl.

4. Verbindungen gemäß einem der vorstehenden Ansprüche, **dadurch gekennzeichnet, dass** der lineare oder verzweigte Alkylrest, wenn er C₁-C₁₅ ist, ausgewählt ist aus der Gruppe, bestehend aus den Resten Methyl, Ethyl, Propyl, 2-Ethylhexyl, Octyl und Dodecyl, und wenn er C₁-C₂₀ ist, er zusätzlich ausgewählt ist unter den Resten Hexadecyl und Octadecyl.

5. Verbindungen gemäß einem der vorstehenden Ansprüche, **dadurch gekennzeichnet, dass** der Monohydroxyalkylrest ausgewählt ist aus der Gruppe, bestehend aus den Resten Hydroxymethyl, 2-Hydroxyethyl, 2-Hydroxypropyl oder 3-Hydroxypropyl.

6. Verbindungen gemäß einem der vorstehenden Ansprüche, **dadurch gekennzeichnet, dass** der Polyhydroxyalkylrest ausgewählt ist aus der Gruppe, bestehend aus den Resten 2,3-Dihydroxypropyl, 2,3,4-Trihydroxybutyl, 2,3,4,5-Tetrahydroxypentyl oder dem Pentaerythritrest.

7. Verbindungen gemäß einem der vorstehenden Ansprüche, **dadurch gekennzeichnet, dass** der Polyetherrest ausgewählt ist aus der Gruppe, bestehend aus den Resten Methoxymethoxy, Methoxyethoxy oder Methoxyethoxymethoxy.

8. Verbindungen gemäß einem der vorstehenden Ansprüche, **dadurch gekennzeichnet, dass** der -CH₂-Polyether-Rest ausgewählt ist aus der Gruppe, bestehend aus den Resten Methoxymethoxymethyl, Ethoxymethoxymethyl und Methoxyethoxymethoxymethyl.

9. Verbindungen gemäß einem der vorstehenden Ansprüche, **dadurch gekennzeichnet, dass** der Arylrest ein Phenylrest ist, gegebenenfalls substituiert mit mindestens einem Halogen, einem Hydroxyl, einer Nitrofunktion, einem Polyetherrest oder einer Aminfunktion, gegebenenfalls geschützt mit einer Acetylgruppe oder gegebenenfalls substituiert mit mindestens einem niederen C₁-C₆-Alkyl oder einem C₁-C₆-Alkoxy.

10. Verbindungen gemäß einem der vorstehenden Ansprüche, **dadurch gekennzeichnet, dass** der Aralkylrest ausgewählt ist aus der Gruppe, bestehend aus den Resten Benzyl oder Phenethyl, gegebenenfalls substituiert mit mindestens einem Halogen, einem Hydroxyl, einer Nitrofunktion, einem Polyetherrest oder einer Aminfunktion, gegebenenfalls geschützt mit einer Acetylgruppe oder gegebenenfalls substituiert mit mindestens einem niederen C₁-C₆-Alkyl oder einem C₁-C₆-Alkoxy.

11. Verbindungen gemäß einem der vorstehenden Ansprüche, **dadurch gekennzeichnet, dass** der Heteroarylrest ausgewählt ist aus der Gruppe, bestehend aus den Resten Pyridyl, Furyl oder Thienyl, gegebenenfalls substituiert mit mindestens einem Halogen, einem niederen C₁-C₆-Alkyl, einem Hydroxyl, einem C₁-C₃-Alkoxy, einer Nitrofunktion, einem Polyetherrest oder einer Aminfunktion, gegebenenfalls geschützt mit einer Acetylgruppe oder gegebenenfalls substituiert mit mindestens einem niederen Alkyl oder einem C₁-C₆-Alkoxy.

12. Verbindungen gemäß einem der vorstehenden Ansprüche, **dadurch gekennzeichnet, dass** der Alkenylrest ausgewählt ist aus der Gruppe, bestehend aus den Resten, enthaltend 2 bis 5 Kohlenstoffatome und mit ein oder zwei ethylenischen Unsättigungen, insbesondere dem Allylrest.

13. Verbindungen gemäß einem der vorstehenden Ansprüche, **dadurch gekennzeichnet, dass** der Zuckerrest ausgewählt ist aus der Gruppe, bestehend aus den Resten von Glukose, Galactose, Mannose oder Glucuronsäure.

14. Verbindungen gemäß einem der vorstehenden Ansprüche, **dadurch gekennzeichnet, dass** der Aminosäurerest ausgewählt ist aus der Gruppe, bestehend aus den Resten, die sich von Lysin, Glycin oder der Asparaginsäure ableiten.

15. Verbindungen gemäß einem der vorstehenden Ansprüche, **dadurch gekennzeichnet, dass** der Peptidrest ausgewählt ist aus der Gruppe, bestehend aus Dipeptidresten oder Tripeptidresten.

16. Verbindungen gemäß einem der vorstehenden Ansprüche, **dadurch gekennzeichnet, dass** dann, wenn r' und r" einen Heterocyclus bilden, dieser ausgewählt ist aus der Gruppe, bestehend aus den Resten Piperidino, Morpholino, Pyrrolidino oder Piperazino, gegebenenfalls substituiert in Position 4 mit einem C₁-C₆-Alkyl oder einem Mono- oder Polyhydroxyalkyl.

17. Verbindungen gemäß einem der vorstehenden Ansprüche, **dadurch gekennzeichnet, dass** das Halogenatom ausgewählt ist aus der Gruppe, bestehend aus Fluor, Chlor und Brom.

18. Verbindungen gemäß einem der vorstehenden Ansprüche, **dadurch gekennzeichnet, dass** sie den folgenden allgemeinen Formeln (II) und (III) entsprechen: 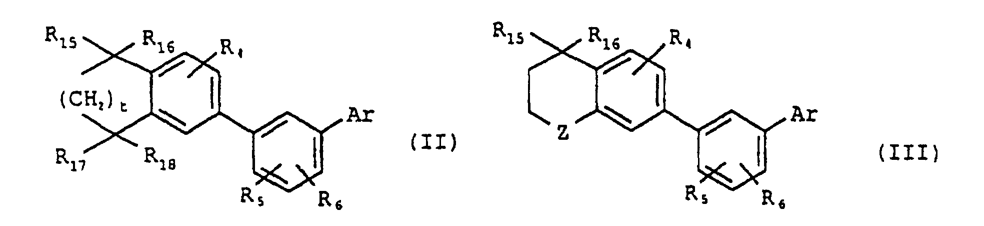 worin:
Ar für einen Rest der folgenden Formel (a) oder (b) steht: 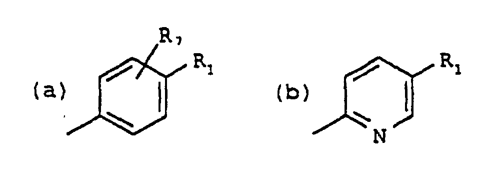
R₁, R₄, R₅, R₆, R₇ und Z dieselben Bedeutungen aufweisen, wie sie in Anspruch 1 gegeben sind,
R₁₅, R₁₆, R₁₇ und R₁₈, gleich oder verschieden, für H oder -CH₃ stehen, und
t 1 oder 2 ist.

19. Verbindungen gemäß einem der vorstehenden Ansprüche, **dadurch gekennzeichnet, dass** sie enthalten sind in der Gruppe, bestehend aus:
- 4-[4-Hydroxy-3-(5,6,7,8-tetrahydro-5,5,8,8-tetramethyl-2-naphtyl)phenyl]benzoesäure und deren Methylester,
- 4-[4-(5-Hydroxypentyloxy)-3-(5,6,7,8-tetrahydro-5,5,8,8-tetramethyl-2-naphtyl)phenyl]benzoesäure und deren Methylester,
- 4-[4-(6-Hydroxyhexyloxy)-3-(5,6,7,8-tetrahydro-5,5,8,8-tetramethyl-2-naphtyl)phenyl]benzoesäure und deren Methylester,
- 4-[4-(7-Hydroxyheptyloxy)-3-(5,6,7,8-tetrahydro-5,5,8,8-tetramethyl-2-naphtyl)phenyl]benzoesäure,
- 4-[4-(8-Hydroxyoctyloxy)-3-(5,6,7,8-tetrahydro-5,5,8,8-tetramethyl-2-naphtyl)phenyl]benzoesäure,
- 4-[4-(9-Hydroxynonyloxy)-3-(5,6,7,8-tetrahydro-5,5,8,8-tetramethyl-2-naphtyl)phenyl]benzosäure,
- 4-[4-Methoxy-3-(5,6,7,8-tetrahydro-5,5,8,8-tetramethyl-2-naphtyl)phenyl]-benzoesäure,
- 4-[4-Methoxyethoxymethoxy-3-(5,6,7,8-tetrahydro-5,5,8,8-tetramethyl-2-naphtyl)phenyl]benzoesäure,
- 4-[4-Benzyloxy-3-(5,6,7,8-tetrahydro-5,5,8,8-tetramethyl-2-naphtyl)phenyl]benzoesäure,
- 4'-(2,3-Dihydroxypropoxy)-3'-(5,5,8,8-tetramethyl-5,6,7,8-tetrahydronaphthalin-2-yl)-biphenyl-4-carbonsäure (Racemat),
- 4'-(2,2-Dimethyl-[1,3]dioxolan-4-ylmethoxy)-3'-(5,5,8,8-tetramethyl-5,6,7,8-tetrahydronaphthalin-2-yl)-biphenyl-4-carbonsäure (Racemat),
- 4'-(2-Morpholin-4-yl-ethoxy)-3'-(5,5,8,8-tetramethyl-5,5,8,8-tetrahydronaphthalin-2-yl)-biphenyl-4-carbonsäure,
- 2'-(5,5,8,8-Tetramethyl-5,6,7,8-tetrahydronaphthalin-2-yl)-[1,1';4',1"]terphenyl-4"-carbonsäuremethylester,
- 2'-(5,5,8,8-Tetramethyl-5,6,7,8-tetrahydronaphthalin-2-yl)-[1,1';4',1"]terphenyl-4"-carbonsäure,
- 4-Methoxymethoxy-2'-(5,5,8,8-tetramethyl-5,6,7,8-tetrahydronaphthalin-2-yl)-[1,1';4',1"]terphenyl-4"-carbonsäure,
- 4-Hydroxy-2'-(5,5,8,8-tetramethyl-5,6,7,8-tetrahydronaphthalin-2-yl)-[1,1';4',1"]terphenyl-4"-carbonsäure,
- 4-Methoxy-2'-(5,5,8,8-tetramethyl-5,6,7,8-tetrahydronaphthalin-2-yl)-[1,1';4',1"]terphenyl-4"-carbonsäure,
- 3-Methoxymethoxy-2'-(5,5,8,8-tetramethyl-5,6,7,8-tetrahydronaphthalin-2-yl)-[1,1';4',1"]terphenyl-4"-carbonsäure,
- 3-Hydroxy-2'-(5,5,8,8-tetramethyl-5,6,7,8-tetrahydronaphthalin-2-yl)-[1,1';4',1"]terphenyl-4"-carbonsäure,
- 3-Methoxy-2'-(5,5,8,8-tetramethyl-5,6,7,8-tetrahydronaphthalin-2-yl)-[1,1';4',1"]terphenyl-4"-carbonsäure,
- 2-Methoxymethoxy-2'-(5,5,8,8-tetramethyl-5,6,7,8-tetrahydronaphthalin-2-yl)-[1,1';4',1"]terphenyl-4"-carbonsäure,
- 2-Hydroxy-2'-(5,5,8,8-tetramethyl-5,6,7,8-tetrahydronaphthalin-2-yl)-[1,1';4',1"]terphenyl-4"-carbonsäure,
- 2-Methoxy-2'-(5,5,8,8-tetramethyl-5,6,7,8-tetrahydronaphthalin-2-yl)-[1,1';4',1"]terphenyl-4 "-carbonsäure,
- 2'-Methoxymethoxy-5'-(5,5,8,8-tetramethyl-5,6,7,8-tetrahydronaphthalin-2-yl)-biphenyl-4-carbonsäure,
- 2'-Methoxy-5'-(5,5,8,8-tetramethyl-5,6,7,8-tetrahydronaphthalin-2-yl)-biphenyl-4-carbonsäure,
- 2'-Propyloxy-5'-(5,5,8,8-tetramethyl-5,6,7,8-tetrahydronaphthalin-2-yl)-biphenyl-4-carbonsäure,
- 2'-Hydroxy-5'-(5,5,8,8-tetramethyl-5,6,7,8-tetrahydronaphthalin-2-yl)-biphenyl-4-carbonsäure,
- 4'-(5,5,8,8-Tetramethyl-5,6,7,8-tetrahydronaphthalin-2-yl)-[1,1';2',1"]terphenyl-4"-carbonsäure,
- 2'-Methoxymethoxy-3'-(5,5,8,8-tetramethyl-5,6,7,8-tetrahydronaphthalin-2-yl)-biphenyl-4-carbonsäure,
- 2'-Hydroxy-3'-(5,5,8,8-tetramethyl-5,6,7,8-tetrahydronaphthalin-2-yl)-biphenyl-4-carbonsäure,
- 2'-Methoxy-3'-(5,5,8,8-tetramethyl-5,6,7,8-tetrahydronaphthalin-2-yl)-biphenyl-4-carbonsäure,
- 3'-Methoxymethoxymethyl-5'-(5,5,8,8-tetramethyl-5,6,7,8-tetrahydronaphthalin-2-yl)-biphenyl-4-carbonsäure,
- 3'-Hydroxymethyl-5'-(5,5,8,8-tetramethyl-5,6,7,8-tetrahydronaphthalin-2-yl)-biphenyl-4-carbonsäure,
- 2'-(4,4-Dimethyl-thiochroman-7-yl)-[1,1';4',1"]terphenyl-4"-carbonsäure,
- 2'-(4,4-Dimethyl-thiochroman-6-yl)-[1,1';4' ,1"]terphenyl-4"-carbonsäure,
- 2'-(3,5,5,8,8-Pentamethyl-5,6,7,8-tetrahydronaphthalin-2-yl)-[1,1';4',1"]terphenyl-4"-carbonsäure,
- 2'-(3-Methoxymethoxy-5,5,8,8-tetramethyl-5,6,7,8-tetrahydronaphthalin-2-yl)-[1,1';4' ,1"]terphenyl-4"-carbonsäure,
- 2'-(3-Hydroxy-5,5,8,8-tetramethyl-5,6,7,8-tetrahydronaphthalin-2-yl)-[1,1';4',1"]terphenyl-4"-carbonsäure,
- 2'-(3-Methoxy-5,5,8,8-tetramethyl-5,6,7,8-tetrahydronaphthalin-2-yl)-[1,1';4',1"]terphenyl-4"-carbonsäure,
- 2'-(3-Propyloxy-5,5,8,8-tetramethyl-5,6,7,8-tetrahydronaphthalin-2-yl)-1,1';4',1"]terphenyl-4"-carbonsäure,
- 3"-Methyl-2'-(5,5,8,8-tetramethyl-5,6,7,8-tetrahydronaphthalin-2-yl)-[1,1';4',1"]terphenyl-4"-carbonsäure,
- 2"-Hydroxy-2'-(5,5,8,8-tetramethyl-5,6,7,8-tetrahydronaphthalin-2-yl)-[1,1';4',1"]terphenyl-4"-carbonsäure,
- 2"-Methoxymethoxy-2'-(5,5,8,8-tetramethyl-5,6,7,8-tetrahydronaphthalin-2-yl)-[1,1';4',1"]terphenyl-4"-carbonsäure,
- 2"-Methoxy-2'-(5,5,8,8-tetramethyl-5,6,7,8-tetrahydronaphthalien-2-yl)-[1,1';4',1"]terphenyl-4"-carbonsäure,
- 2"-Propyloxy-2'-(5,5,8,8-tetramethyl-5,6,7,8-tetrahydronaphthalin-2-yl)-[1,1';4',1"]terphenyl-4"-carbonsäure,
- 3"-Hydroxy-2'-(5,5,8,8-tetramethyl-5,6,7,8-tetrahydronaphthalin-2-yl)-[1,1';4',1"]terphenyl-4"-carbonsäure,
- 6-[2-(5,5,8,8-Tetramethyl-5,6,7,8-tetrahydronaphthalin-2-yl)-biphenyl-4-yl]nicotinsäure,
- 5-[2-(5,5,8,8-Tetramethyl-5,6,7,8-tetrahydronaphthalin-2-yl)-biphenyl-4-yl]pyridine-2-carbonsäure,
- 2'-(5,5,8,8-Tetramethyl-5,6,7,8-tetrahydronaphthalin-2-yl)-[1,1';4',1"]terphenyl-4"-hydroxamsäure,
- 2'-(5,5,8,8-Tetramethyl-5,6,7,8-tetrahydronaphthalin-2-yl)-[1,1';4',1"]terphenyl-4"-ol,
- [2'- (5,5,8,8-Tetramethyl-5,6,7,8-tetrahydronaphthalin-2-yl)-[1,1';4',1"]terphenyl-4"-yl]-methanol,
- 2'-(5,5,8,8-Tetramethyl-5,6,7,8-tetrahydronaphthalin-2-yl)-[1,1';4',1"]terphenyl-4"-carbaldehyd,
- 4'-Methoxycarbonylmethoxy-3'-(5,5,8,8-tetramethyl-5,6,7,8-tetrahydronaphthalin-2-yl)-biphenyl-4-carbonsäure,
- 4'-Carboxymethoxy-3'-(5,5,8,8-tetramethyl-5,6,7,8-tetrahydronaphthalin-2-yl)biphenyl-4-carbonsäure,
- 4'-(5-Ethoxycarbonylpentyloxy)-3'-(5,5,8,8-tetramethyl-5,6,7,8-tetrahydronaphthalin-2-yl)-biphenyl-4-carbonsäure,
- 4'-(5-Carboxypentyloxy)-3'-(5,5,8,8-tetramethyl-5,6,7,8-tetrahydronaphthalin-2-yl)-biphenyl-4-carbonsäure,
- 2'-(5,5,8,8-Tetramethyl-5,6,7,8-tetrahydronaphthalin-2-yl)-[1,1';4',1"]terphenyl-4"-carboxamid,
- N-Ethyl-2' -(5,5,8,8-tetramethyl-5,6,7,8-tetrahydronaphthalin-2-yl)-[1,1';4',1"]-terphenyl-4"-carboxamid,
- N,N-Diethyl-2'-(5,5,8,8-tetramethyl-5,6,7,8-tetrahydronaphthalin-2-yl)-[1,1';4',1"]terphenyl-4"-carboxamid,
- Morpholin-4-yl-[2'-(5,5,8,8-tetramethyl-5,6,7,8-tetrahydronaphthalin-2-yl)-[1,1';4',1"]terphenyl-4"-yl]methanon,
- (4-Hydroxyphenyl)-2'-(5,5,8,8-tetramethyl-5,6,7,8-tetrahydronaphthalin-2-yl)-[1,1';4',1"]terphenyl-4"-carboxamid,
- 3-(5,5,8,8-Tetramethyl-5,6,7,8-tetrahydronaphthalin-2-yl)-biphenyl-4-carboxymethyl-4'-carbonsäure,
- 3-(5,5,8,8-Tetramethyl-5,6,7,8-tetrahydronaphthalin-2-yl)-biphenyl-4,4'-dicarbonsäure,
- 3'-Methoxymethoxy-5'-(5,5,8,8-tetramethyl-5,6,7,8-tetrahydronaphthalin-2-yl)-biphenyl-4-carbonsäure,
- 3'-Methoxy-5'-(5,5,8,8-tetramethyl-5,6,7,8-tetrahydronaphthalin-2-yl)-biphenyl-4-carbonsäure,
- 3'-Propyloxy-5'-(5,5,8,8-tetramethyl-5,6,7,8-tetrahydronaphthalin-2-yl)-biphenyl-4-carbonsäure,
- 3'-Hydroxy-5'-(5,5,8,8-tetramethyl-5,6,7,8-tetrahydronaphthalin-2-yl)-biphenyl-4-carbonsäure,
- 4'-(5,5,8,8-Tetramethyl-5,6,7,8-tetrahydronaphthalin-2-yl)-[1,1';3',1"]terphenyl-4"-carbonsäure,
- 4'-(5-Carboxamidopentyloxy)-3'-(5,5,8,8-tetramethyl-5,6,7,8-tetrahydronaphthalin-2-yl)-biphenyl-4-carbonsäure,
- 3'-Methoxycarbonyl-5'-(5,5,8,8-tetramethyl-5,6,7,8-tetrahydronaphthalin-2-yl)-biphenyl-4-carbonsäure,
- 3'-Carboxyl-5'-(5,5,8,8-tetramethyl-5,6,7,8-tetrahydronaphthalin-2-yl)-biphenyl-4-carbonsäure,
- 2'-(4-Hydroxy-5,5,8,8-tetramethyl-5,6,7,8-tetrahydronaphthalin-2-yl)-[1,1';4',1"]terphenyl-4"-carbonsäure,
- 2'-(4-Methoxy-5,5,8,8-tetramethyl-5,6,7,8-tetrahydronaphthalin-2-yl)-[1,1';4',1"]terphenyl-4"-carbonsäure,
- 2'-(4-Propyloxy-5,5,8,8-tetramethyl-5,6,7,8-tetrahydronaphthalin-2-yl)-[1,1';4',1"]terphenyl-4"-carbonsäure,
- 2'-(4-Methoxymethoxy-5,5,8,8-tetramethyl-5,6,7,8-tetrahydronaphthalin-2-yl)-[1,1';4',1"]terphenyl-4"-carbonsäure,
- 2-[2-(5,5,8,8-tetramethyl-5,6,7,8-tetrahydronaphthalin-2-yl)-biphenyl-4-yl]-thiophen-4-carbonsäure.

20. Verbindungen gemäß einem der vorstehenden Ansprüche für die Verwendung als Medikament.

21. Verbindungen gemäß Anspruch 20 für eine Verwendung als Medikament, bestimmt zur Behandlung dermatologischer Beschwerden, dermatologischer Beschwerden mit entzündlicher und/oder immunallergischer Komponente vom rheumatischen Typ oder respiratorischer Komponente, von cardiovaskulären Beschwerden und ophtalmologischen Störungen.

22. Verwendung mindestens einer Verbindung gemäß einem der Ansprüche 1 bis 19 für die Herstellung eines Medikaments, bestimmt zur Behandlung von dermatologischen Beschwerden, von dermatologischen Beschwerden mit inflammatorischer und/oder immunallergischer Komponente vom rheumatischen Typ oder respiratorischer Beschwerden, von cardiovaskulären Beschwerden und ophtalmologischen Störungen.

23. Pharmazeutische Zubereitung, **dadurch gekennzeichnet, dass** sie in einem annehmbaren pharmazeutischen Träger mindestens eine Verbindung enthält, wie sie in einem der Ansprüche 1 bis 19 definiert ist.

24. Zubereitung gemäß Anspruch 23, **dadurch gekennzeichnet, dass** die Konzentration der mindestens einen Verbindung gemäß einem der Ansprüche 1 bis 19 zwischen 0,001 % und 5 Gew.-% liegt, bezogen auf das Gesamtgewicht der Zubereitung.

25. Kosmetische Zubereitung, **dadurch gekennzeichnet, dass** sie in einem kosmetisch annehmbaren Träger mindestens eine Verbindung enthält, wie sie in einem der Ansprüche 1 bis 19 definiert ist.

26. Zubereitung gemäß Anspruch 25, **dadurch gekennzeichnet, dass** die Konzentration der mindestens einen Verbindung gemäß einem der Ansprüche 1 bis 19 zwischen 0,001 und 3 Gew.-% liegt, bezogen auf das Gesamtgewicht der Zubereitung.

27. Verwendung einer kosmetischen Zubereitung, wie sie in einem der Ansprüche 25 oder 26 definiert ist, für die Körper- oder Haarpflege.

## Claims

1. Biphenyl compounds substituted with an aromatic or heteroaromatic radical, **characterized in that** they correspond to the general formula (I) below: 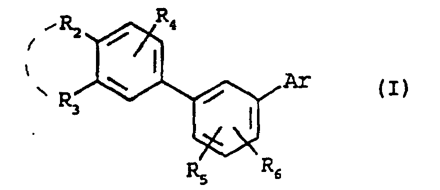 in which:
Ar represents an aromatic or heteroaromatic radical chosen from: 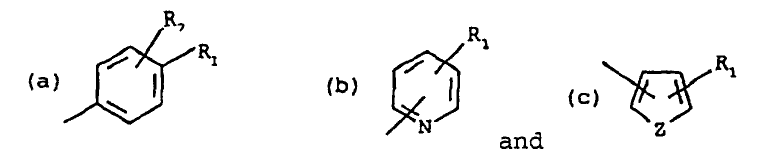
Z being O or S,
R₁ represents -CH₃, -CH₂-OH, -OR₈ or -COR₉,
R₂ and R₃, which may be identical or different, represent H, linear or branched C₁-C₁₅ alkyl, cycloalkyl, -ZR₁₀ or a polyether radical, at least one from among R₂ and R₃ representing a linear or branched C₁-C₁₅ alkyl radical, or
R₂ and R₃, taken together, form a 5- or 6- membered ring, optionally substituted with at least one methyl and/or optionally interrupted by an oxygen or sulphur atom or by an SO or SO₂ radical,
R₄ represents H, a halogen atom, linear or branched C₁-C₂₀ alkyl, -OR₁₀, -OCOR₁₁ or a polyether radical,
R₅ represents H, a halogen atom, linear or branched C₁-C₂₀ alkyl, -OCOR₁₁, -OR₁₂, mono- or polyhydroxyalkyl, -NO₂, 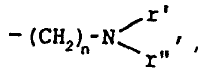 - (CH₂)ₙ-NHCOCH₃, -CH=CH-COR₁₃, -(CH₂)ₙCOR₁₃, n being 0 to 6, -O-(CH₂)ₘCOR₁₃, -O-(CH₂)ₘOH, m being 1 to 12, optionally substituted aryl, optionally substituted aralkyl, optionally substituted heteroaryl, a polyether radical or a -CH₂-polyether radical,
R₆ represents H, C₁-C₆ lower alkyl or -OR₁₀,
R₇ represents H, a halogen atom, linear or branched C₁-C₂₀ alkyl, -OR₁₀ or -OCOR₁₁ or a polyether radical,
R₈ represents H, C₁-C₆ lower alkyl or -COR₁₁,
R₉ represents H, C₁-C₆ lower alkyl, -OR₁₄ or 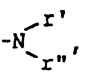
R₁₀ represents H or C₁-C₆ lower alkyl,
R₁₁ represents C₁-C₆ lower alkyl,
R₁₂ represents H, linear or branched C₁-C₂₀ alkyl, mono- or polyhydroxyalkyl, or optionally substituted aryl or aralkyl,
R₁₃ represents H, C₁-C₆ lower alkyl, -OR₁₀, aryl or 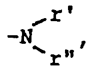
R₁₄ represents H, linear or branched C₁-C₂₀ alkyl, alkenyl, mono- or polyhydroxyalkyl, optionally substituted aryl or aralkyl, or a sugar residue,
r' and r", which may be identical or different, represent H, OH, C₁-C₆ lower alkyl, mono- or polyhydroxyalkyl, optionally substituted aryl, an amino acid residue or a peptide residue, or r' and r", taken together, form a heterocycle,
and the salts of the compounds of formula (I) when R₁ represents a carboxylic acid function, as well as the optical and geometrical isomers of the said compounds of formula (I).

2. Compounds according to Claim 1, **characterized in that** they are in the form of a salt of an alkali metal or alkaline-earth metal, or alternatively of zinc or of an organic amine.

3. Compounds according to either of Claims 1 and 2, **characterized in that** the C₁-C₆ lower alkyl radical is chosen from the group consisting of the methyl, ethyl, isopropyl, butyl, tert-butyl and hexyl radicals.

4. Compounds according to any one of the preceding claims, **characterized in that** the linear or branched alkyl radical, when it is C₁-C₁₅, is chosen from the group consisting of the methyl, ethyl, propyl, 2-ethylhexyl, octyl and dodecyl radicals, and, when it is C₁-C₂₀, is also chosen from the hexadecyl and octadecyl radicals.

5. Compounds according to any one of the preceding claims, **characterized in that** the monohydroxyalkyl radical is chosen from the group consisting of the hydroxymethyl, 2-hydroxyethyl, 2-hydroxypropyl and 3-hydroxypropyl radicals.

6. Compounds according to any one of the preceding claims, **characterized in that** the polyhydroxyalkyl radical is chosen from the group consisting of the 2,3-dihydroxypropyl, 2,3,4-trihydroxybutyl and 2,3,4,5-tetrahydroxypentyl radicals and the pentaerythritol residue.

7. Compounds according to any one of the preceding claims, **characterized in that** the polyether radical is chosen from the group consisting of the methoxymethoxy, methoxyethoxy and methoxyethoxymethoxy radicals.

8. Compounds according to any one of the preceding claims, **characterized in that** the -CH₂-polyether radical is chosen from the group consisting of the methoxymethoxymethyl, ethoxymethoxymethyl and methoxyethoxymethoxymethyl radicals.

9. Compounds according to any one of the preceding claims, **characterized in that** the aryl radical is a phenyl radical optionally substituted with at least one halogen, a hydroxyl, a nitro function, a polyether radical or an amino function optionally protected with an acetyl group or optionally substituted with at least one C₁-C₆ lower alkyl or C₁-C₆ alkoxy.

10. Compounds according to any one of the preceding claims, **characterized in that** the aralkyl radical is chosen from the group consisting of benzyl and phenethyl radicals optionally substituted with at least one halogen, a hydroxyl, a nitro function, a polyether radical or an amino function optionally protected with an acetyl group or optionally substituted with at least one C₁-C₆ lower alkyl or C₁-C₆ alkoxy.

11. Compounds according to any one of the preceding claims, **characterized in that** the heteroaryl radical is chosen from the group consisting of pyridyl, furyl and thienyl radicals, optionally substituted with at least one halogen, a C₁-C₆ lower alkyl, a hydroxyl, a C₁-C₃ alkoxy, a nitro function, a polyether radical or an amino function optionally protected with an acetyl group or optionally substituted with at least one C₁-C₆ lower alkyl or alkoxy.

12. Compounds according to any one of the preceding claims, **characterized in that** the alkenyl radical is chosen from the group consisting of radicals containing from 2 to 5 carbon atoms and containing one or two ethylenic unsaturation(s), in particular the allyl radical.

13. Compounds according to any one of the preceding claims, **characterized in that** the sugar residue is chosen from the group consisting of glucose, galactose, mannose and glucuronic acid residues.

14. Compounds according to any one of the preceding claims, **characterized in that** the amino acid residue is chosen from the group consisting of residues derived from lysine, from glycine or from aspartic acid.

15. Compounds according to any one of the preceding claims, **characterized in that** the peptide residue is chosen from the group consisting of dipeptide and tripeptide residues.

16. Compounds according to any one of the preceding claims, **characterized in that** when r' and r" form a heterocycle, this is chosen from the group consisting of piperidino, morpholino, pyrrolidino and piperazino radicals, optionally substituted in position 4 with a C₁-C₆ alkyl or a mono- or polyhydroxyalkyl.

17. Compounds according to any one of the preceding claims, **characterized in that** the halogen atom is chosen from the group consisting of fluorine, chlorine and bromine.

18. Compounds according to any one of the preceding claims, **characterized in that** they correspond to the general formulae (II) and (III) below: 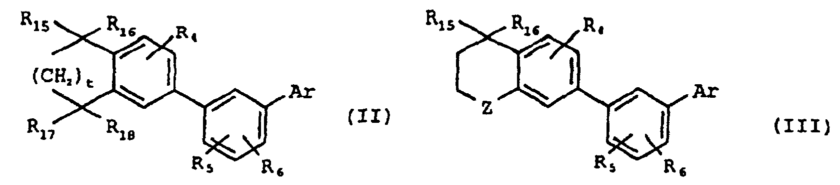 in which:
Ar represents a radical of formula (a) or (b) below: 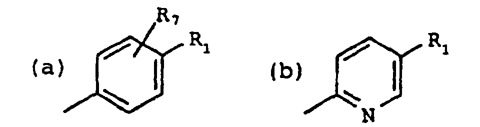
R₁, R₄, R₅, R₆, R₇ and Z having the same meanings as those given in Claim 1,
R₁₅, R₁₆, R₁₇ and R₁₈, which may be identical or different, represent H or -CH₃, and
t is 1 or 2.

19. Compounds according to any one of the preceding claims, **characterized in that** they are taken from the group consisting of:
- 4-[4-hydroxy-3-(5,6,7,8-tetrahydro-5,5,8,8-tetramethyl-2-naphthyl)-phenyl]benzoic acid, and its methyl ester,
- 4-[4-(5-hydroxypentyloxy)-3-(5,6,7,8-tetrahydro-5,5,8,8-tetramethyl-2-naphthyl)phenyl]benzoic acid, and its methyl ester,
- 4-[4-(6-hydroxyhexyloxy)-3-(5,6,7,8-tetrahydro-5,5,8,8-tetramethyl-2-naphthyl)phenyl]benzoic acid, and its methyl ester,
- 4-[4-(7-hydroxyheptyloxy)-3-(5,6,7,8-tetrahydro-5,5,8,8-tetramethyl-2-naphthyl)phenyl]benzoic acid,
- 4-[4-(8-hydroxyoctyloxy)-3-(5,6,7,8-tetrahydro-5,5,8,8-tetramethyl-2-naphthyl)phenyl]benzoic acid,
- 4-[4-(9-hydroxynonyloxy)-3-(5,6,7,8-tetrahydro-5,5,8,8-tetramethyl-2-naphthyl)phenyl]benzoic acid,
- 4-[4-methoxy-3-(5,6,7,8-tetrahydro-5,5,8,8-tetramethyl-2-naphthyl)phenyl]benzoic acid,
- 4- [4-methoxyethoxymethoxy-3-(5,6,7,8-tetrahydro-5,5,8,8-tetramethyl-2-naphthyl)phenyl]benzoic acid,
- 4-[4-benzyloxy-3-(5,6,7,8-tetrahydro-5,5,8,8-tetramethyl-2-naphthyl)phenyl]benzoic acid,
- 4'-(2,3-dihydroxypropoxy)-3'-(5,5,8,8-tetramethyl-5,6,7,8-tetrahydro-2-naphthyl)biphenyl-4-carboxylic acid (racemic),
- 4'-(2,2-dimethyl-[1,3]dioxolan-4-ylmethoxy)-3'-(5,5,8,8-tetramethyl-5,6,7,8-tetrahydro-2-naphthyl)biphenyl-4-carboxylic acid (racemic),
- 4'-(2-morpholin-4-ylethoxy)-3'-(5,5,8,8-tetramethyl-5,6,7,8-tetrahydro-2-naphthyl)biphenyl-4-carboxylic acid,
- methyl 2'-(5,5,8,8-tetramethyl-5,6,7,8-tetrahydro-2-naphthyl)-[1,1';4',1"]terphenyl-4"-carboxylate,
- 2'-(5,5,8,8-tetramethyl-5,6,7,8-tetrahydro-2-naphthyl)-[1,1';4',1"]terphenyl-4"-carboxylic acid,
- 4-methoxymethoxy-2'-(5,5,8,8-tetramethyl-5,6,7,8-tetrahydro-2-naphthyl)-[1,1';4',1"]terphenyl-4"-carboxylic acid,
- 4-hydroxy-2'-(5,5,8,8-tetramethyl-5,6,7,8-tetrahydro-2-naphthyl)-[1,1';4',1"]terphenyl-4"-carboxylic acid,
- 4-methoxy-2'-(5,5,8,8-tetramethyl-5,6,7,8-tetrahydro-2-naphthyl)-[1,1';4',1"]terphenyl-4"-carboxylic acid,
- 3-methoxymethoxy-2'-(5,5,8,8-tetramethyl-5,6,7,8-tetrahydro-2-naphthyl)-[1,1';4',1"]terphenyl-4"-carboxylic acid,
- 3-hydroxy-2'-(5,5,8,8-tetramethyl-5,6,7,8-tetrahydro-2-naphthyl)-[1,1';4',1"]terphenyl-4"-carboxylic acid,
- 3-methoxy-2'-(5,5,8,8-tetramethyl-5,6,7,8-tetrahydro-2-naphthyl)-[1,1';4',1"]terphenyl-4"-carboxylic acid,
- 2-methoxymethoxy-2'-(5,5,8,8-tetramethyl-5,6,7,8-tetrahydro-2-naphthyl)-[1,1';4',1"]terphenyl-4"-carboxylic acid,
- 2-hydroxy-2'-(5,5,8,8-tetramethyl-5,6,7,8-tetrahydro-2-naphthyl)-[1,1';4',1"]terphenyl-4"-carboxylic acid,
- 2-methoxy-2'-(5,5,8,8-tetramethyl-5,6,7,8-tetrahydro-2-naphthyl)-[1,1';4',1"]terphenyl-4"-carboxylic acid,
- 2'-methoxymethoxy-5'-(5,5,8,8-tetramethyl-5,6,7,8-tetrahydro-2-naphthyl)biphenyl-4-carboxylic acid,
- 2'-methoxy-5'-(5,5,8,8-tetramethyl-5,6,7,8-tetrahydro-2-naphthyl)biphenyl-4-carboxylic acid,
- 2'-propyloxy-5'-(5,5,8,8-tetramethyl-5,6,7,8-tetrahydro-2-naphthyl)biphenyl-4-carboxylic acid,
- 2'-hydroxy-5'-(5,5,8,8-tetramethyl-5,6,7,8-tetrahydro-2-naphthyl)biphenyl-4-carboxylic acid,
- 4'-(5,5,8,8-tetramethyl-5,6,7,8-tetrahydro-2-naphthyl)-[1,1';2',1"]terphenyl-4"-carboxylic acid,
- 2'-methoxymethoxy-3'-(5,5,8,8-tetramethyl-5,6,7,8-tetrahydro-2-naphthyl)biphenyl-4-carboxylic acid,
- 2'-hydroxy-3'-(5,5,8,8-tetramethyl-5,6,7,8-tetrahydro-2-naphthyl)biphenyl-4-carboxylic acid,
- 2'-methoxy-3'-(5,5,8,8-tetramethyl-5,6,7,8-tetrahydro-2-naphthyl)biphenyl-4-carboxylic acid,
- 3'-methoxymethoxymethyl-5'-(5,5,8,8-tetramethyl-5,6,7,8-tetrahydro-2-naphthyl)biphenyl-4-carboxylic acid,
- 3'-hydroxymethyl-5'-(5,5,8,8-tetramethyl-5,6,7,8-tetrahydro-2-naphthyl)biphenyl-4-carboxylic) acid,
- 2'-(4,4-dimethylthiochroman-7-yl)-[1,1';4',1"]terphenyl-4"-carboxylic acid,
- 2'-(4,4-dimethylthiochroman-6-yl)-[1,1';4',1"]terphenyl-4"-carboxylic acid,
- 2'-(3,5,5,8,8-pentamethyl-5,6,7,8-tetrahydro-2-naphthyl)-[1,1';4',1"]terphenyl-4"-carboxylic acid,
- 2'-(3-methoxymethoxy-5,5,8,8-tetramethyl-5,6,7,8-tetrahydro-2-naphthyl)-[1,1';4',1"]terphenyl-4"-carboxylic acid,
- 2'-(3-hydroxy-5,5,8,8-tetramethyl-5,6,7,8-tetrahydro-2-naphthyl)-[1,1';4',1"]terphenyl-4"-carboxylic acid,
- 2'-(3-methoxy-5,5,8,8-tetramethyl-5,6,7,8-tetrahydro-2-naphthyl)-[1,1';4',1"]terphenyl-4"-carboxylic acid,
- 2'-(3-propyloxy-5,5,8,8-tetramethyl-5,6,7,8-tetrahydro-2-naphthyl)-[1,1';4',1"]terphenyl-4"-carboxylic acid,
- 3"-methyl-2'-(5,5,8,8-tetramethyl-5,6,7,8-tetrahydro-2-naphthyl)-[1,1';4',1"]terphenyl-4"-carboxylic acid,
- 2"-hydroxy-2'-(5,5,8,8-tetramethyl-5,6,7,8-tetrahydro-2-naphthyl)-[1,1';4',1"]terphenyl-4"-carboxylic acid,
- 2"-methoxymethoxy-2'-(5,5,8,8-tetramethyl-5,6,7,8-tetrahydro-2-naphthyl)-[1,1';4',1"]terphenyl-4"-carboxylic acid,
- 2"-methoxy-2'-(5,5,8,8-tetramethyl-5,6,7,8-tetrahydro-2-naphthyl)-[1,1';4',1"]terphenyl-4"-carboxylic acid,
- 2"-propyloxy-2'-(5,5,8,8-tetramethyl-5,6,7,8-tetrahydro-2-naphthyl)-[1,1';4',1"]terphenyl-4"-carboxylic acid,
- 3"-hydroxy-2'-(5,5,8,8-tetramethyl-5,6,7,8-tetrahydro-2-naphthyl)-[1,1';4',1"]terphenyl-4"-carboxylic acid,
- 6-[2-(5,5,8,8-tetramethyl-5,6,7,8-tetrahydro-2-naphthyl)biphenyl-4-yl]nicotinic acid,
- 5-[2-(5,5,8,8-tetramethyl-5,6,7,8-tetrahydro-2-naphthyl)biphenyl-4-yl]-2-pyridinecarboxylic acid,
- 2'-(5,5,8,8-tetramethyl-5,6,7,8-tetrahydro-2-naphthyl)-[1,1';4',1"]terphenyl-4"-hydroxamic acid,
- 2'-(5,5,8,8-tetramethyl-5,6,7,8-tetrahydro-2-naphthyl)-[1,1';4',1"]terphenyl-4"-ol,
- [2'-(5,5,8,8-tetramethyl-5,6,7,8-tetrahydro-2-naphthyl)-[1,1';4',1"]terphenyl-4"-yl]methanol,
- 2'-(5,5,8,8-tetramethyl-5,6,7,8-tetrahydro-2-naphthyl)-[1,1';4',1"]terphenyl-4"-carbaldehyde,
- 4'-methoxycarbonylmethoxy-3'-(5,5,8,8-tetramethyl-5,6,7,8-tetrahydro-2-naphthyl)biphenyl-4-carboxylic acid,
- 4'-carboxymethoxy-3'-(5,5,8,8-tetramethyl-5,6,7,8-tetrahydro-2-naphthyl)biphenyl-4-carboxylic acid,
- 4'-(5-ethoxycarbonylpentyloxy)-3'-(5,5,8,8-tetramethyl-5,6,7,8-tetrahydro-2-naphthyl)biphenyl-4-carboxylic acid,
- 4'-(5-carboxypentyloxy)-3'-(5,5,8,8-tetramethyl-5,6,7,8-tetrahydro-2-naphthyl)biphenyl-4-carboxylic acid,
- 2'-(5,5,8,8-tetramethyl-5,6,7,8-tetrahydro-2-naphthyl)-[1,1';4',1"]terphenyl-4"-carboxamide,
- N-ethyl-2'-(5,5,8,8-tetramethyl-5,6,7,8-tetrahydro-2-naphthyl)-[1,1';4',1"]terphenyl-4"-carboxamide,
- N,N-diethyl-2'-(5,5,8,8-tetramethyl-5,6,7,8-tetrahydro-2-naphthyl)-[1,1';4',1"]terphenyl-4"-carboxamide,
- morpholin-4-yl-(2'-(5,5,8,8-tetramethyl-5,6,7,8-tetrahydro-2-naphthyl)-[1,1';4',1"]terphenyl-4"-yl]methanone,
- (4-hydroxyphenyl)-2'-(5,5,8,8-tetramethyl-5,6,7,8-tetrahydro-2-naphthyl)-[1,1';4',1"]terphenyl-4"-carboxamide,
- 3-(5,5,8,8-tetramethyl-5,6,7,8-tetrahydro-2-naphthyl)biphenyl-4-carboxymethyl-4'-carboxylic acid,
- 3-(5,5,8,8-tetramethyl-5,6,7,8-tetrahydro-2-naphthyl)biphenyl-4,4'-dicarboxylic acid,
- 3'-methoxymethoxy-5'-(5,5,8,8-tetramethyl-5,6,7,8-tetrahydro-2-naphthyl)biphenyl-4-carboxylic acid,
- 3'-methoxy-5'-(5,5,8,8-tetramethyl-5,6,7,8-tetrahydro-2-naphthyl)biphenyl-4-carboxylic acid,
- 3'-propyloxy-5'-(5,5,8,8-tetramethyl-5,6,7,8-tetrahydro-2-naphthyl)biphenyl-4-carboxylic acid,
- 3'-hydroxy-5'-(5,5,8,8-tetramethyl-5,6,7,8-tetrahydro-2-naphthyl)biphenyl-4-carboxylic acid,
- 4'-(5,5,8,8-tetramethyl-5,6,7,8-tetrahydro-2-naphthyl)-[1,1';3',1"] terphenyl-4"-carboxylic acid,
- 4'-(5-carboxamidopentyloxy)-3'-(5,5,8,8-tetramethyl-5,6,7,8-tetrahydro-2-naphthyl)biphenyl-4-carboxylic acid,
- 3'-methoxycarbonyl-5'-(5,5,8,8-tetramethyl-5,6,7,8-tetrahydro-2-naphthyl)biphenyl-4-carboxylic acid,
- 3'-carboxyl-5'-(5,5,8,8-tetramethyl-5,6,7,8-tetrahydro-2-naphthyl)biphenyl-4-carboxylic acid,
- 2'-(4-hydroxy-5,5,8,8-tetramethyl-5,6,7,8-tetrahydro-2-naphthyl)-[1,1';4',1"]terphenyl-4"-carboxylic acid,
- 2'-(4-methoxy-5,5,8,8-tetramethyl-5,6,7,8-tetrahydro-2-naphthyl)-[1,1';4',1"]terphenyl-4"-carboxylic acid,
- 2'-(4-propyloxy-5,5,8,8-tetramethyl-5,6,7,8-tetrahydro-2-naphthyl)-[1,1';4',1"]terphenyl-4"-carboxylic acid,
- 2'-(4-methoxymethoxy-5,5,8,8-tetramethyl-5,6,7,8-tetrahydro-2-naphthyl)-[1,1';4',1"]terphenyl-4"-carboxylic acid,
- 2-[2-(5,5,8,8-tetramethyl-5,6,7,8-tetrahydro-2-naphthyl)biphenyl-4-yl]-4-thiophenecarboxylic acid.

20. Compounds according to any one of the preceding claims, for use as a medicinal product.

21. Compounds according to Claim 20, for use as a medicinal product intended for the treatment of dermatological complaints, dermatological complaints with an inflammatory and/or immunoallergic component of the rheumatic or respiratory type, cardiovascular complaints and opthalmological disorders.

22. Use of at least one of the compounds as defined according to any one of Claims 1 to 19, for the preparation of a medicinal product intended for the treatment of dermatological complaints, dermatological complaints with an inflammatory and/or immunoallergic component of the rheumatic or respiratory type, cardiovascular complaints and opthalmological disorders.

23. Pharmaceutical composition, **characterized in that** it comprises, in a pharmaceutically acceptable support, at least one compound as defined according to any one of Claims 1 to 19.

24. Composition according to Claim 23, **characterized in that** the concentration of at least one compound according to one of Claims 1 to 19 is between 0.001% and 5% by weight relative to the total weight of the composition.

25. Cosmetic composition, **characterized in that** it contains, in a cosmetically acceptable support, at least one compound as defined according to any one of Claims 1 to 19.

26. Composition according to Claim 25, **characterized in that** the concentration of at least one compound according to any one of Claims 1 to 19 is between 0.001 and 3% by weight relative to the total weight of the composition.

27. Use of a cosmetic composition as defined according to either of Claims 25 and 26, for body or hair hygiene.
